(19) Europäisches Patentamt
European Patent Office
Office européen des brevets

(11) Publication number: **0 599 274 A1**

(12) # EUROPEAN PATENT APPLICATION

(21) Application number: **93118837.9**

(22) Date of filing: **23.11.93**

(51) Int. Cl.$^5$: **C12N 15/12**, C07K 13/00, C12P 21/08, C12N 5/10, G01N 33/68, G01N 33/577, A61K 39/395

(30) Priority: **24.11.92 US 981165**

(43) Date of publication of application: **01.06.94 Bulletin 94/22**

(84) Designated Contracting States: **AT BE CH DE DK ES FR GB GR IE IT LI LU MC NL PT SE**

(71) Applicant: **Bristol-Myers Squibb Company**
**345 Park Avenue**
**New York, N.Y. 10154(US)**

(72) Inventor: **Plowman, Gregory D.**
**1000 Union Street No. C.**
**Seattle, WA 98101(US)**
Inventor: **Culouscou, Jean-Michel**
**3034 W. Viewmont Way W.**
**Seattle, WA 98199(US)**
Inventor: **Shoyab, Mohammed**
**2405 Westmont Way W.**
**Seattle, WA 98199(US)**

(74) Representative: **Kinzebach, Werner, Dr. et al**
**Patentanwälte**
**Reitstötter, Kinzebach und Partner**
**Postfach 86 06 49**
**D-81633 München (DE)**

(54) **HER4, a human receptor tyrosine kinase of the epidermal growth factor receptor family.**

(57) The molecular cloning, expression, and biological characteristics of a novel receptor tyrosine kinase related to the epidermal growth factor receptor, termed HER4/p180$^{erbB4,}$ are described. A HER4 ligand capable of inducing cellular differentiation of breast cancer cells is also disclosed. In view of the expression of HER4 in several human cancers and in certain tissues of neuronal and muscular origin, various diagnostic and therapeutic uses of HER4-derived and HER4-related biological compositions are provided.

EP 0 599 274 A1

FIGURE 5

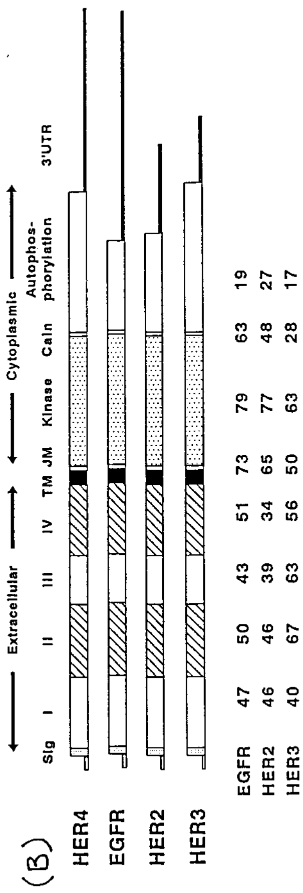
(B)
HER4
EGFR
HER2
HER3
Extracellular
Cytoplasmic
Sig
I
II
III
IV
TM
JM
Kinase
Caln
Autophos-
phorylation
3'UTR
EGFR 47 50 43 51 73 79 63 19
HER2 46 46 39 34 65 77 48 27
HER3 40 67 63 56 50 63 28 17

## 1. INTRODUCTION

The present invention is generally directed to a novel receptor tyrosine kinase related to the epidermal growth factor receptor, termed HER4/p180$^{erbB4}$ ("HER4"),and to novel diagnostic and therapeutic compositions comprising HER4-derived or HER4-related biological components. The invention is based in part upon applicants discovery of human HER4, its complete nucleotide coding sequence, and functional properties of the HER4 receptor protein. More specifically, the invention is directed to HER4 biologics comprising, for example, polynucleotide molecules encoding HER4, HER4 polypeptides, anti-HER4 antibodies which recognize epitopes of HER4 polypeptides, ligands which interact with HER4, and diagnostic and therapeutic compositions and methods based fundamentally upon such molecules. In view of the expression of HER4 in several human cancers and in certain tissues of neuronal and muscular origin, the present invention provides a framework upon which effective biological therapies may be designed. The invention is hereinafter described in detail, in part by way of experimental examples specifically illustrating various aspects of the invention and particular embodiments thereof.

## 2. BACKGROUND OF THE INVENTION

Cells of virtually all tissue types express transmembrane receptor molecules with intrinsic tyrosine kinase activity through which various growth and differentiation factors mediate a range of biological effects (reviewed in Aaronson, 1991, Science 254: 1146-52). Included in this group of receptor tyrosine kinases (RTKs) are the receptors for polypeptide growth factors such as epidermal growth factor (EGF), insulin, plateletderived growth factor (PDGF), neurotrophins (i.e., NGF), and fibroblast growth factor (FGF). Recently, the ligands for several previously-characterized receptors have been identified, including ligands for c-kit (steel factor), met (hepatocyte growth factor), trk (nerve growth factor) (see, respectively, Zsebo et al., 1990, Cell 63: 195-201; Bottardo et al., 1991, Science 251: 802-04; Kaplan et al., 1991, Nature 350: 158-160). In addition, the soluble factor NDF, or heregulin-alpha (HRG-$\alpha$), has been identified as the ligand for HER2, a receptor which is highly related to HER4 (Wen et al., 1992, Cell 69:559-72; Holmes et al., 1992 Science 256:1205-10). However, at present, the ligands for a number of isolated and/or characterized receptor tyrosine kinases have still not been identified, including those for the eph, eck, elk, ret, and HER3 receptors.

Biological relationships between various human malignancies and genetic aberrations in growth factor-receptor tyrosine kinase signal pathways are known to exist. Among the most notable such relationships involve the EGF receptor (EGFR) family of receptor tyrosine kinases (see Aaronson, *supra*). Three human EGFR-family members have been identified and are known to those skilled in the art: EGFR, HER2/p185$^{erbB2}$, and HER3/p160$^{erbB3}$ (see, respectively, Ullrich et al, 1984, Nature 309: 418-25; Coussens et al., 1985, Science 230: 1132-39; and Plowman et al., 1990, Proc. Natl. Acad. Sci. U.S.A. 87: 4905-09). EGRF-related molecules from other species have also been identified.

The complete nucleotide coding sequence of other EGFR-family members has also been determined from other organisms including: the drosophila EGFR ("DER": Livneh, E. et al., 1985, Cell 40: 599-607), nematode EGFR ("let-23": Aroian, R.V. et al., 1990, Nature 348: 693-698), chicken EGFR ("CER": Lax, I. et al., 1988, Mol. Cell. Biol. 8: 1970-1978), rat EGFR (Petch, L.A. et al., 1990, Mol. Cell. Biol. 10: 2973-2982), rat HER2/neu (Bargmann, C.I. et al., 1986, Nature, 319: 226-230) and a novel member isolated from the fish and termed *Xiphophorus* melanoma related kinase ("Xmrk": Wittbrodt, J. et al., 1989, Nature 342: 415-421). In addition, PCR technology has led to the isolation of other short DNA fragments that may encode novel receptors or may represent species-specific homologs of known receptors. One recent example is the isolation tyro-2 (Lai, C. and Lemke, G., 1991, Neuron 6: 691-704) a fragment encoding 54 amino acids that is most related to the EGFR family.

Overexpression of EGFR-family receptors is frequently observed in a variety of aggressive human epithelial carcinomas. In particular, increased expression of EGFR is associated with more aggressive carcinomas of the breast, bladder, lung and stomach (see, for example, Neal et al., 1985, Lancet 1: 366-68; Sainsbury et al., 1987, Lancet 1: 1398-1402; Yasui et al., 1988, Int. J. Cancer 41: 211-17; Veale et al., 1987, Cancer 55: 513-16). In addition, amplification and overexpression of HER2 has been associated with a wide variety of human malignancies, particularly breast and ovarian carcinomas, for which a strong correlation between HER2 overexpression and poor clinical prognosis and/or increased relapse probability have been established (see, for example, Slamon et al., 1987, Science 235: 177-82, and 1989, Science 244: 707-12). Overexpression of HER2 has also been correlated with other human carcinomas, including carcinoma of the stomach, endometrium, salivary gland, bladder, and lung (Yokota et al., 1986, Lancet 1: 765-67; Fukushigi et al., 1986, Mol. Cell. Biol. 6: 955-58; Yonemura et al., 1991, Cancer Res. 51: 1034; Weiner et al., 1990, Cancer Res. 50: 421-25; Geurin et al., 1988, Oncogene Res. 3:21-31; Semba et al., 1985, Proc. Natl. Acad.

Sci. U.S.A. 82: 6497-6501; Zhau et al., 1990, Mol. Carcinog. 3: 354-57; McCann et al., 1990, Cancer 65: 88-92). Most recently, a potential link between HER2 overexpression and gastric carcinoma has been reported (Jaehne et al., 1992, J. Cancer Res. Clin. Oncol. 118: 474-79). Finally, amplified expression of the recently described HER3 receptor has been observed in a wide variety of human adenocarcinomas (Poller et al., 1992, J. Path, in press; Krause et al, 1989, Proc. Natl. Acad. Sci. U.S.A. 86: 9193-97; European Patent Application No. 91301737, published 9.4.91, EP 444 961).

Several structurally related soluble polypeptides capable of specifically binding to EGFR have been identified and characterized, including EGF, transforming growth factor-alpha (TGF-α), amphiregulin (AR), heparin-binding EGF (HB-EGF), and vaccinia virus growth factor (VGF) (see, respectively, Savage et al., 1972, J. Biol. Chem. 247: 7612-21; Marquardt et al., 1984, Science 223: 1079-82; Shoyab et al., 1989, Science 243: 1074-76; Higashiyama et al., 1991, Science 251: 936-39; Twardzik et al., 1985, Proc. Natl. Acad. Sci. U.S.A. 82: 5300-04). Despite the close structural relationships among receptors of the EGFR-family, none of these ligands has been conclusively shown to interact with HER2 or HER3.

Recently, several groups have reported the identification of specific ligands for HER2. Some of these ligands, such as gp30 (Lupu et al., 1990, Science 249: 1552-55; Bacus et al., 1992, Cell Growth and Differentiation 3: 401-11) interact with both EGFR and HER2, while others are reported to bind specifically to HER2 (Wen et al., 1992, Cell 69: 559-72; Peles et al., 1992, Cell 69: 205-16; Holmes et al., 1992, Science 256: 1205-10; Lupu et al., 1992, Proc. Natl. Acad. Sci. U.S.A. 89: 2287-91; Huang et al., 1992, J. Biol. Chem. 276: 11508-121). The best characterized of these ligands are neu differentiation factor (NDF) purified and cloned from ras-transformed Rat1-EJ cells (Wen et al., Peles et al., *supra*), and the heregulins (HRF-α, -β1, -β2, -β3), purified and cloned from human MDA-MB-231 cells (Holmes et al., *supra*). NDF and HRG-α share 93% sequence identity and appear to be the rat and human homologs of the same protein. Both of these proteins are similar size (44-45 kDa), increase tyrosine phosphorylation of HER2 in MDA-MB-453 cells and not the EGF-receptor, and have been reported to bind to HER2 in cross-linking studies on human breast cancer cells. In addition, NDF has been shown to induce differentiation of human mammary tumor cells to milk-producing, growth-arrested cells, whereas the heregulin family have been reported to stimulate proliferation of cultured human breast cancers cell monolayers.

The means by which receptor polypeptides transduce regulatory signals in response to ligand binding is not fully understood, and continues to be the subject of intensive investigation. However, important components of the process have been uncovered, including the understanding that phosphorylation of and by cell surface receptors hold fundamental roles in signal transduction. In addition to the involvement of phosphorylation in the signal process, the intracellular phenomena of receptor dimerization and receptor crosstalk function as primary components of the circuit through which ligand binding triggers a resulting cellular response. Ligand binding to transmembrane receptor tyrosine kinases induces receptor dimerization, leading to activation of kinase function through the interaction of adjacent cytoplasmic domains. Receptor crosstalk refers to intracellular communication between two or more proximate receptor molecules mediated by, for example, activation of one receptor through a mechanism involving the kinase activity of the other. One particularly relevant example of such a phenomenon is the binding of EGF to the EGFR, resulting in activation of the EGFR kinase domain and cross-phosphorylation of HER2 (Kokai et al., 1989, Cell 58: 287-92; Stern et al., 1988, EMBO J. 7: 995-1001; King et al., 1989, Oncogene 4: 13-18).

## 3. SUMMARY OF THE INVENTION

HER4 is the fourth member of the EGFR-family of receptor tyrosine kinases and is likely to be involved not only in regulating normal cellular function but also in the loss of normal growth control associated with certain human cancers. In this connection, HER4 appears to be closely connected with certain carcinomas of epithelial origin, such as adenocarcinoma of the breast. As such, its discovery, and the elucidation of the HER4 coding sequence, open a number of novel approaches to the diagnosis and treatment of human cancers in which the aberrant expression and/or function of this cell surface receptor is involved.

The complete nucleotide sequence encoding the prototype HER4 polypeptide of the invention is disclosed herein, and provides the basis for several general aspects of the invention hereinafter described. Thus, the invention includes embodiments directly involving the production and use of *HER4* polynucleotide molecules. In addition, the invention provides HER4 polypeptides, such as the prototype HER4 polypeptide disclosed and characterized in the sections which follow. Polypeptides sharing nearly equivalent structural characteristics with the prototype HER4 molecule are also included within the scope of this invention. Furthermore, the invention includes polypeptides which interact with HER4 expressed on the surface of certain cells thereby affecting their growth and/or differentiation. The invention is also directed to anti-HER4 antibodies, which have a variety of uses including but not limited to their use as components of novel biological approaches to human cancer diagnosis and therapy provided by the invention.

The invention also relates to the discovery of an apparent functional relationship between HER4 and HER2, and the therapeutic aspects of the invention include those which are based on applicants' preliminary understanding of this relationship. Applicants' data strongly suggests that HER4 interacts with HER2 either by heterodimer formation or receptor crosstalk, and that such interaction appears to be one mechanism by which the HER4 receptor mediates effects on cell behavior. The reciprocal consequence is that HER2 activation is in some circumstances mediated through HER4.

## 4. BRIEF DESCRIPTIONS OF THE FIGURES

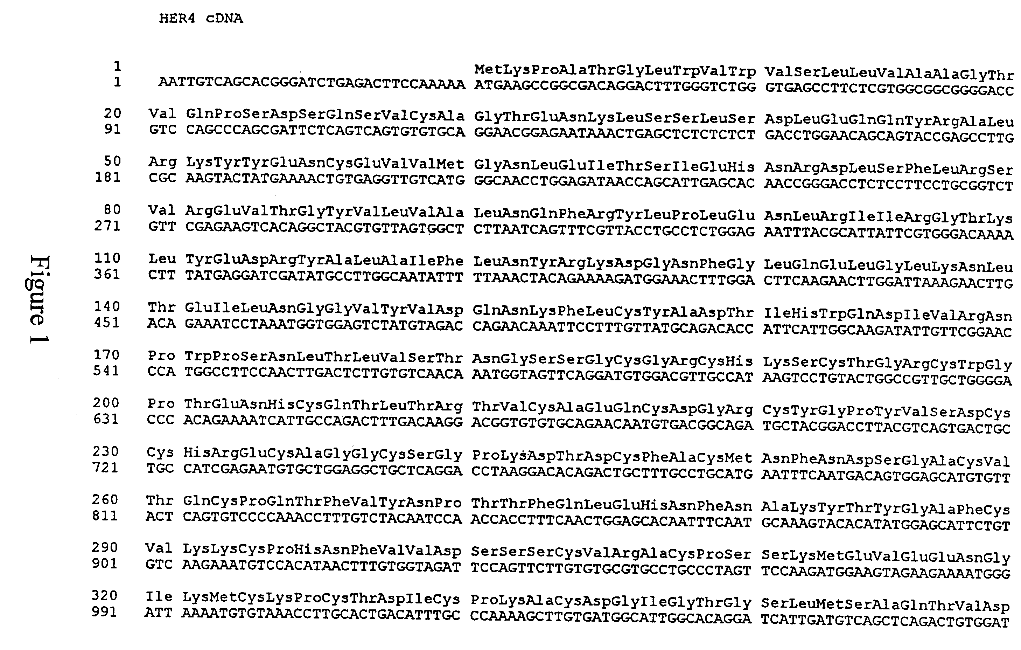

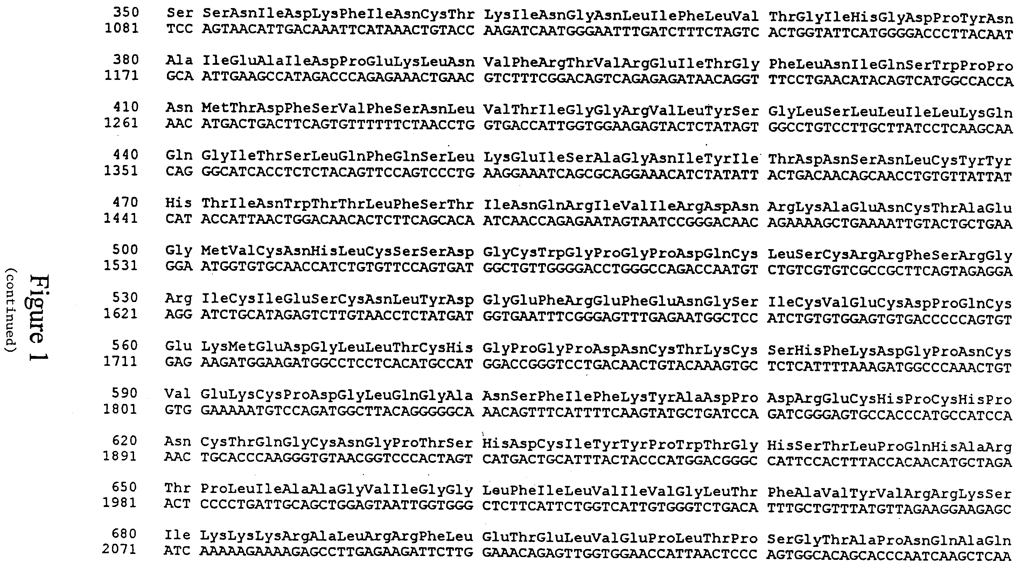

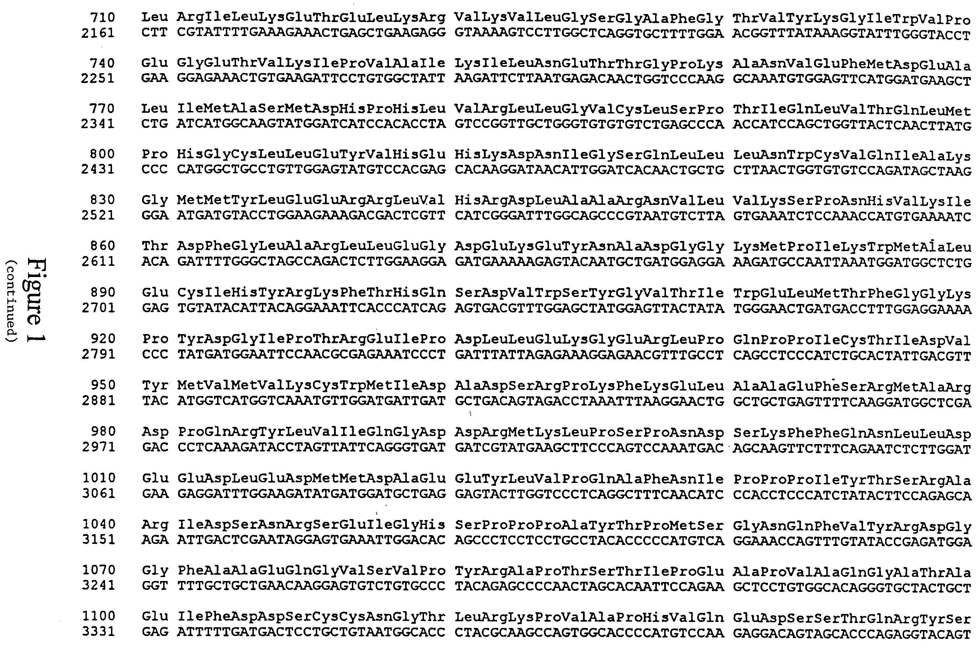

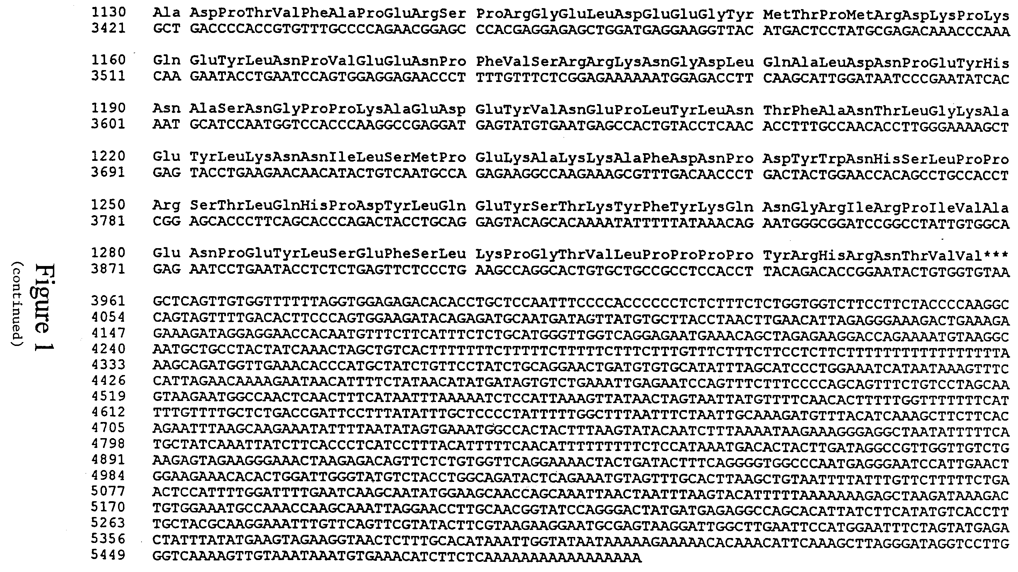

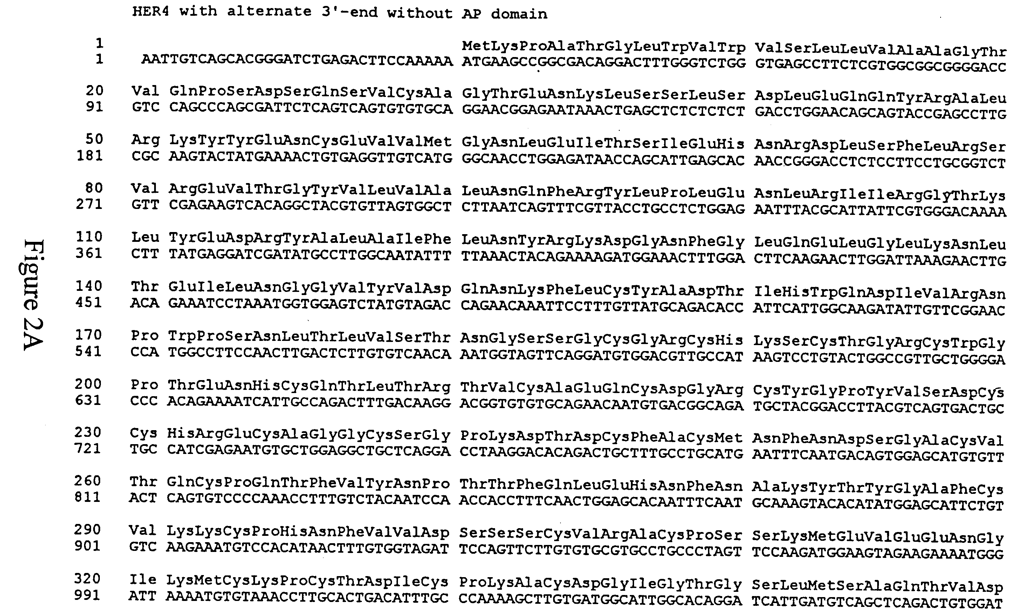

FIG. 1. Nucleotide sequence [SEQ ID NO: 1] and deduced amino acid sequence [SEQ ID NO: 2] of HER4 (1308 amino acid residues). Nucleotides are numbered on the left, and amino acids are numbered above the sequence.

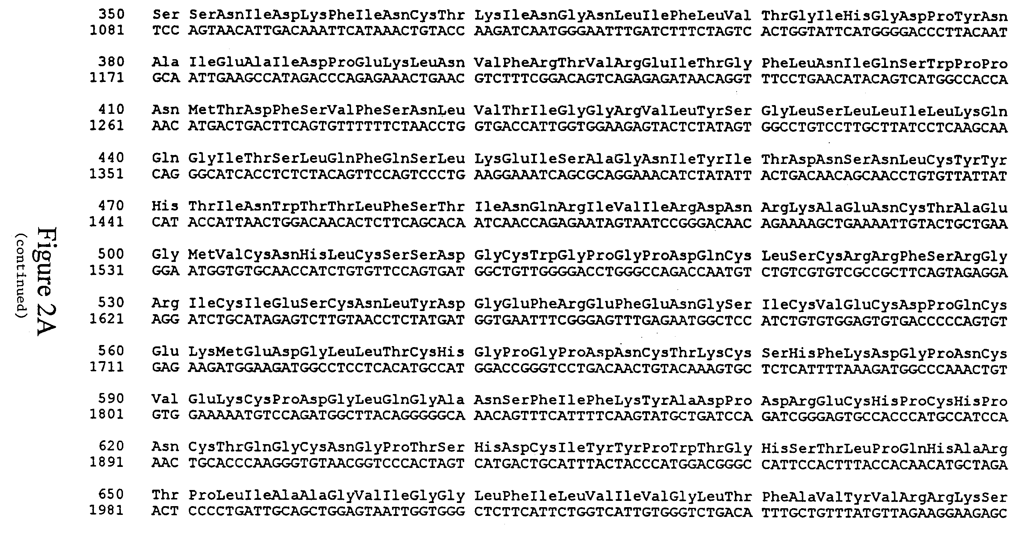

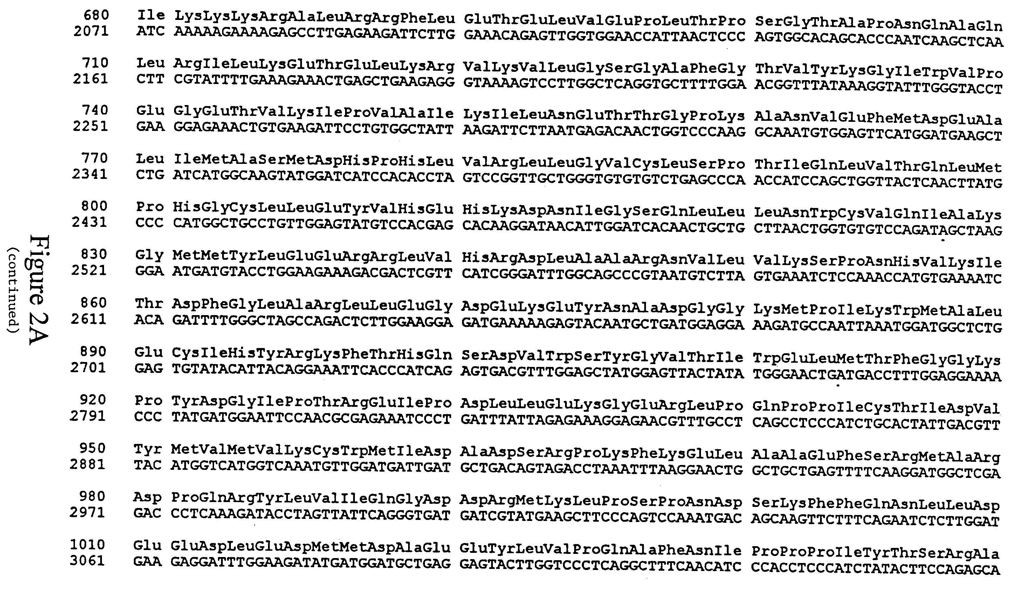

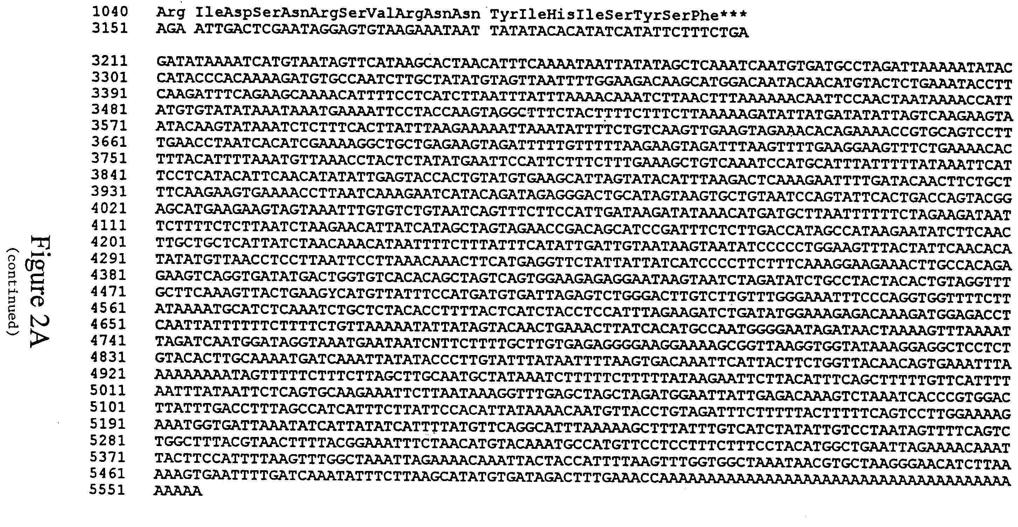

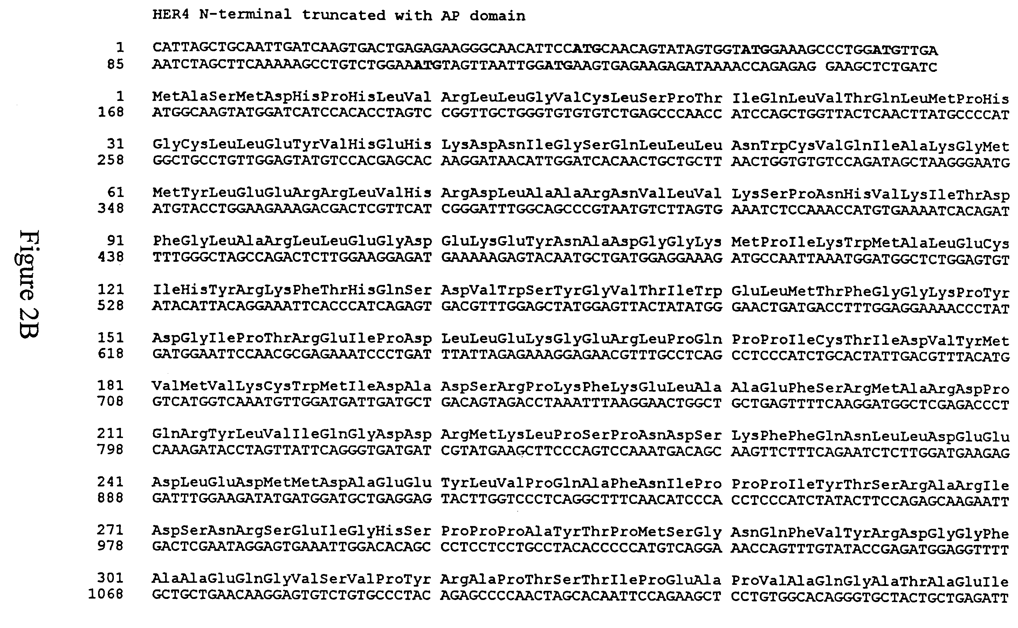

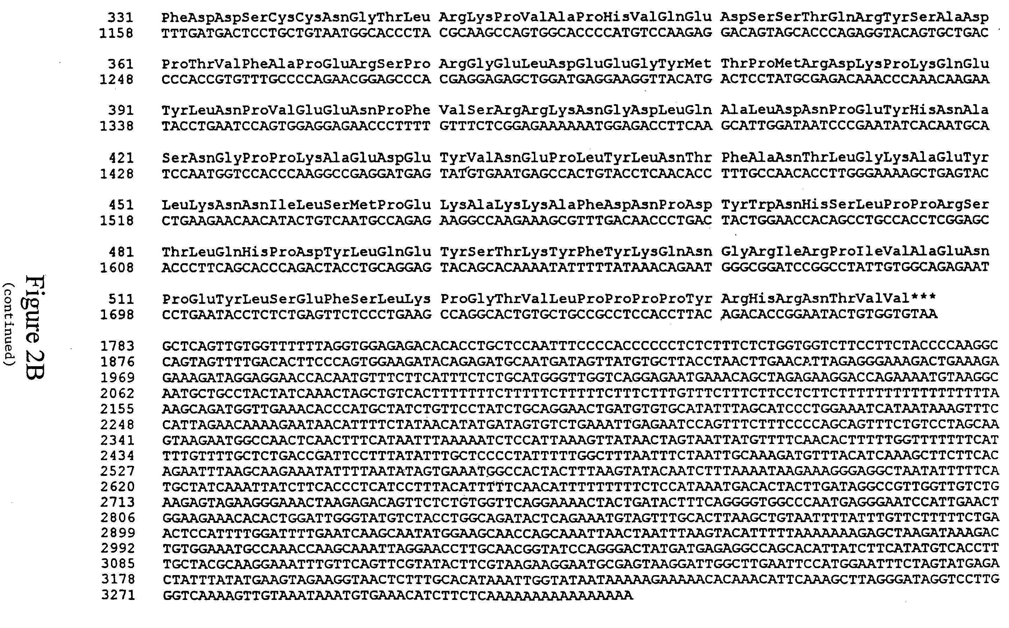

FIG: 2. Nucleotide sequence (FIG. 2(A) [SEQ ID NO: 3]; FIG: 2(B) [SEQ ID NO: 5] and deduced amino acid sequence (FIG. 2(A) [SEQ ID NO: 4]; FIG. 2(B) [SEQ ID NO: 6]) of cDNAs encoding HER4 variants. (A) HER4 with alternate 3' end and without autophosphorylation domain. This sequence is identical with that of HER4 shown in FIG. 1 up to nucleotide 3168, where the sequence diverges and the open reading frame stops after 13 amino acids, followed by an extended, unique 3'-untranslated region. (B) HER4 with N-terminal truncation. This sequence contains the 3'-portion of the HER4 sequence where nucleotide position 156 of the truncated sequence aligns with position 2335 of the complete HER4 sequence shown in FIG. 1 (just downstream from the region encoding the ATP-binding site of the HER4 kinase). The first 155 nucleotides of the truncated sequence are unique from HER4 and may represent the 5'-untranslated region of a transcript derived from a cryptic promoter within an intron of the HER4 gene. (Section 6.2.2., *infra*).

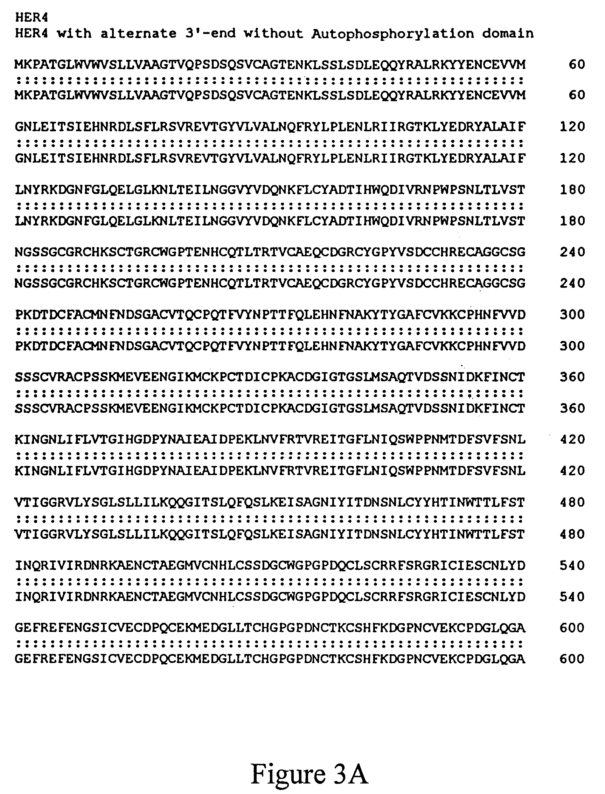

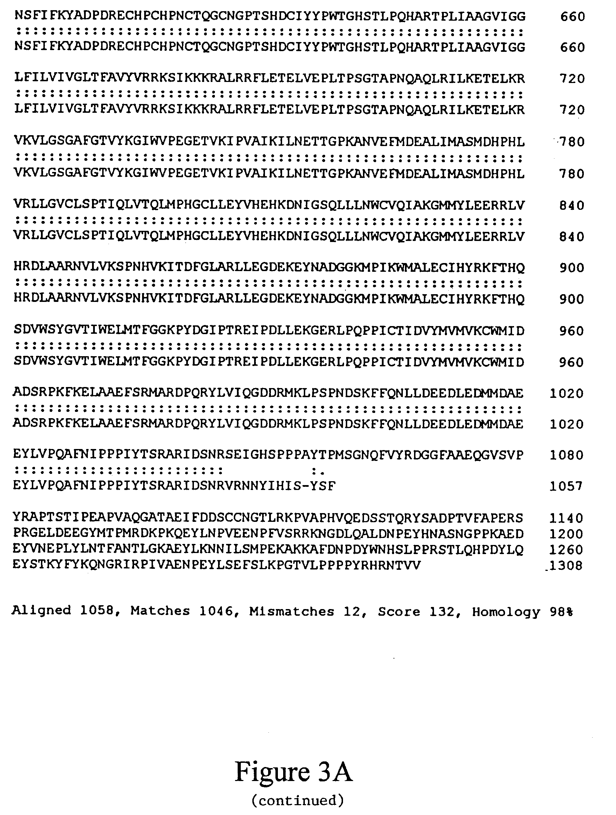

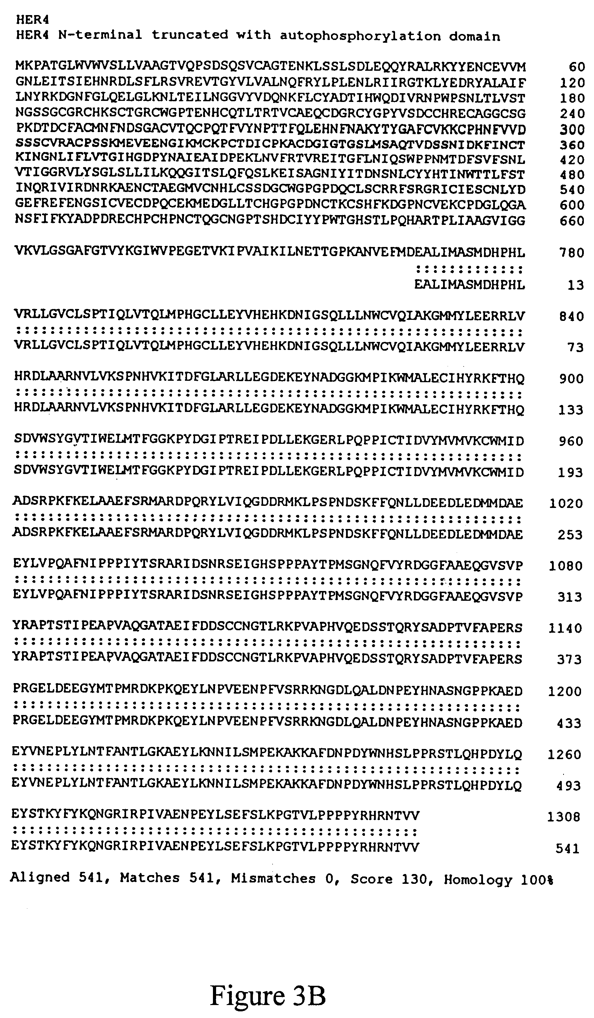

FIG. 3. The deduced amino acid sequence of two variant forms of human HER4 aligned with the full length HER4 receptor as represented in FIG. 1. Sequences are displayed using the single-letter code and are numbered on the right with the complete HER4 sequence on top and the variant sequences below. Identical residues are indicated by a colon between the aligned residues. (A) HER4 with alternate 3'-end, lacking an autophosphorylation domain [SEQ ID NO: 4]. This sequence is identical with that of HER4 [SEQ ID NO: 2] shown in FIG. 1 up to amino acid 1045, where the sequence diverges and continues for 13 amino acids before reaching a stop codon. (B) HER4 with N-terminal truncation [SEQ ID NO: 6]. This sequence is identical to the 3'-portion of the HER4 [SEQ ID No. 2] shown in FIG. 1 beginning at amino acid 768. (Section 6.2.2., infra).

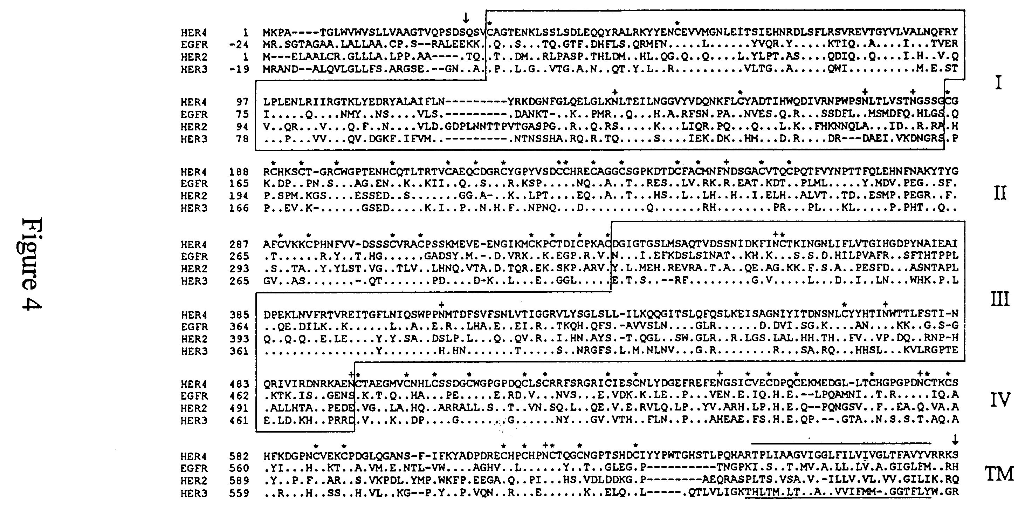

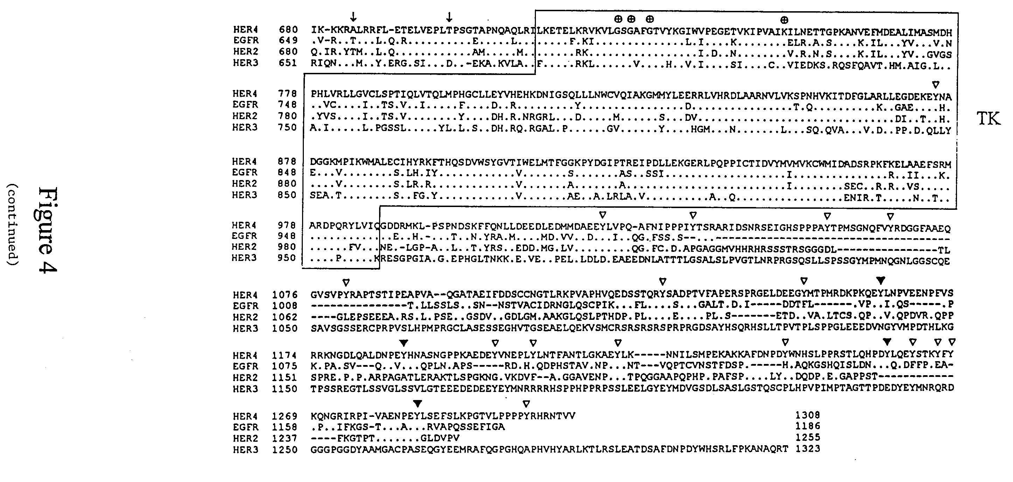

FIG. 4. Deduced amino acid sequence of human HER4 [SEQ ID NO: 2] and alignment with other human EGFR-family members (EGFR [SEQ ID NO: 7]; HER2 [SEQ ID NO: 8]; HER3 [SEQ ID NO: 9]) Sequences are displayed using the single-letter code and are numbered on the left. Identical residues are denoted with dots, gaps are introduced for optimal alignment, cysteine residues are marked with an asterisk, and N-linked glycosylation sites are denoted with a plus (+). Potential protein kinase C phosphorylation sites are indicated by arrows (HER4 amino acid positions 679, 685, and 699). The predicted ATP-binding site is shown with 4 circled crosses, C-terminal tyrosines are denoted with open triangles, and tyrosines in HER4 that are conserved with the major autophosphorylation sites in the EGFR are indicated with black triangles. The predicted extracellular domain extends from the boundary of the signal sequence marked by an arrow at position 25, to the hydrophobic transmembrane domain which is overlined from amino acid positions 650 through 675. Various subdomains are labeled on the right: I, II, III, and IV = extracellular subdomains ( domains II and IV are cysteine-rich); TM = transmembrane domain; TK = tyrosine kinase domain. Domains I, III, TK are boxed.

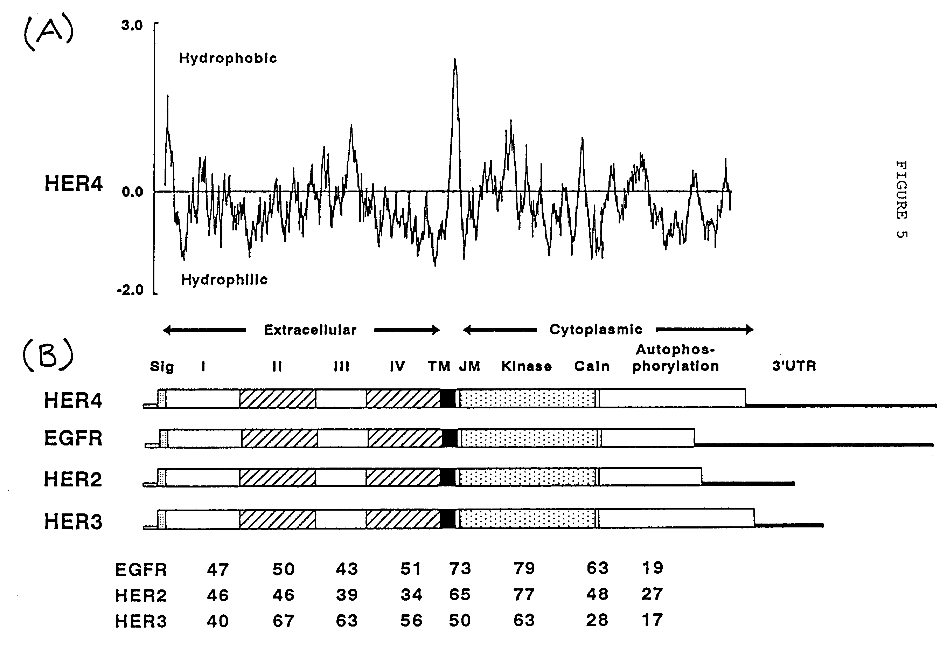

FIG. 5. (A) Hydropathy profile of HER4, aligned with (B) Comparison of protein domains for HER4 (1308 amino acids), EGFR (1210 amino acids), HER2 (1255 amino acids), and HER3 (1342 amino acids). The signal peptide is represented by a stippled box, the cysteine-rich extracellular subdomains are hatched, the transmembrane domain is filled, and the cytoplasmic tyrosine kinase domain is stippled. The percent amino acid sequence identities between HER4 and other EGFR-family members are indicated. Sig, signal peptide; I, II, III, and IV, extracellular domains; TM, transmembrane domain; JM, juxtamembrane domain; CaIn, calcium influx and internalization domain; 3'UTR, 3' untranslated region.

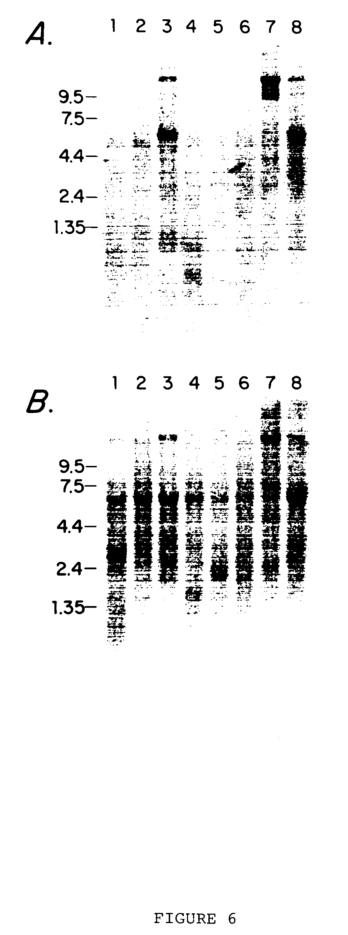

FIG. 6. Northern blot analysis of mRNA from human tissues hybridized to HER4 probes from (A) the 3'-autophosphorylation domain, and (B) the 5'-extracellular domain (see Section 6.2.3., *infra*). RNA size markers (in kilobases) are shown on the left. Lanes 1 through 8 represent 2 µg of poly(A)+ mRNA from pancreas, kidney, skeletal muscle, liver, lung, placenta, brain, and heart, respectively.

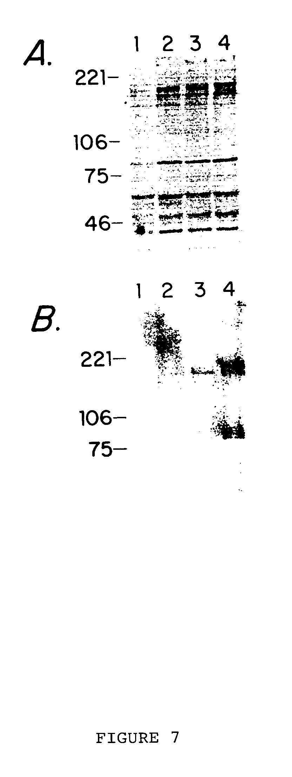

FIG. 7. Immunoblot analysis of recombinant HER4 stably expressed in CHO-KI cells, according to procedure outlined in Section 7.1.3, *infra*. Membrane preparations from CHO-KI cells expressing recombinant HER4 were separated on 7% SDS-polyacrylamide gels and transferred to nitrocellulose. Blots were hybridized with (A) a monoclonal antibody to the C-terminus of HER2 (Ab3, Oncogene Science, Uniondale, NY) that cross-reacts with HER4 or (B) a sheep antipeptide polyclonal antibody to a common epitope of HER2 and HER4. Lane 1, parental CHO-KI cells; lanes 2 - 4, CHO-KI/HER4 cell clones 6, 21, and 3, respectively. Note the 180 kDa HER4 protein and the 130 kDa cross-reactive species. The size in kilodaltons of prestained high molecular weight markers (BioRad, Richmond, CA) is shown on the left.

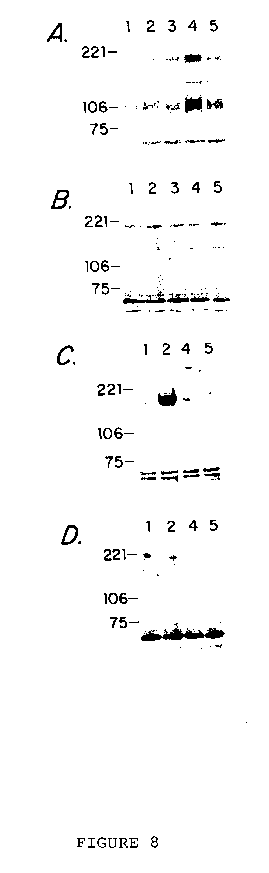

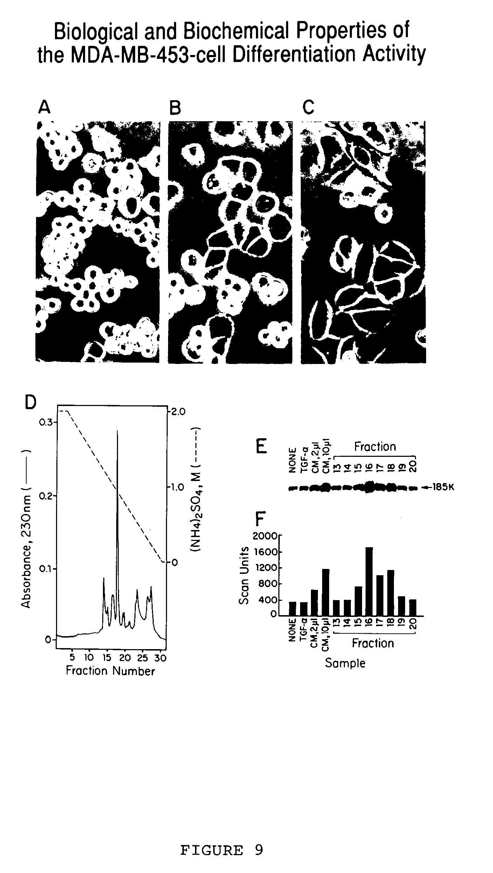

FIG. 8. Specific activation of HER4 tyrosine kinase by a breast cancer differentiation factor (see Section 8., *infra*). Four recombinant cell lines, each of which was engineered to overexpress a single member of EGFR-family of tyrosine kinase receptors (EGFR, HER2, HER3, and HER4), were prepared according to the methods described in Sections 7.1.2 and 8.1., *infra*. Cells from each of the four recombinant cell lines were stimulated with various ligand preparations and assayed for receptor tyrosine phosphorylation using the assay described in Section 8.2., *infra*. (A) CHO/HER4 #3 cells, (B) CHO/HER2 cells, (C) NRHER5 cells, and (D) 293/HER3 cells. Cells stimulated with : lane 1, buffer control; lane 2, 100 ng/ml EGF; lane 3, 200 ng/ml amphiregulin; lane 4, 10 µl phenyl column fraction 17 (Section 9, *infra*); lane 5, 10 µl phenyl column fraction 14 (Section 9., *infra*, and see description of FIG. 9 below). The size (in kilodaltons) of the prestained molecular weight markers are labeled on the left of each panel. The phosphorylated receptor in each series migrates just below the 221 kDa marker. Bands at the bottom of the gels are extraneous and are due to the reaction of secondary antibodies with the antibodies used in the immunoprecipitation.

FIG. 9. Biological and biochemical properties of the MDA-MB-453-cell differentiation activity purified from the conditioned media of HepG2 cells (Section 9., *infra*). (A, B, and C) Induction of morphologic differentiation. Conditioned media from HepG2 cells was subjected to ammonium sulfate fractionation, followed by dialysis against PBS. Dilutions of this material were added to MDA-MB-453 monolayer at the indicated protein concentrations. (A) control; (B) 80 ng per well; (C) 2.0 µg per well. (D) Phenyl-5PW column elution profile monitored at 230 nm absorbance. (E) Stimulation of MDA-MB-453 tyrosine autophosphorylation with the following ligand preparations: None (control with no factor added); TGF-α (50 ng/ml); CM (16-fold concentrated HepG2 conditioned medium tested at 2 µl and 10 µl per well); fraction (phenyl column fractions 13 to 20, 10 µl per well). (F) Densitometry analysis of the phosphorylation signals shown in (E).

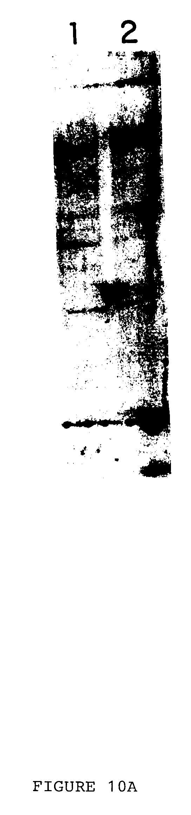

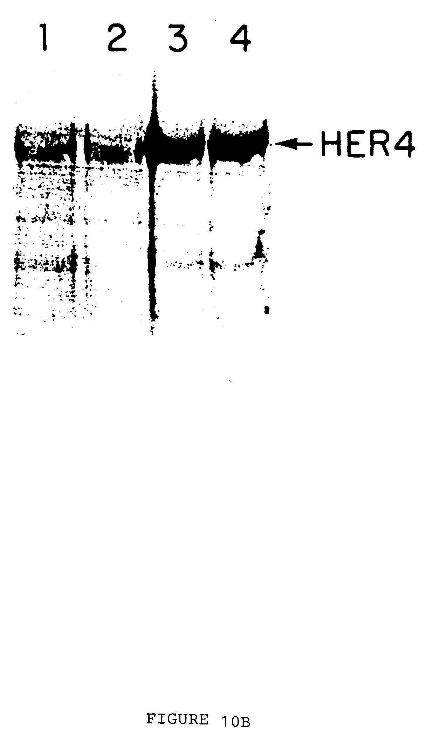

FIG. 10. NDF-induced tyrosine phosphorylation of (A) MDA-MB-453 cells (lane 1, mock transfected COS cell supernatant; lane 2, NDF transfected COS cell supernatant); and (B) CHO/HER4 21-2 cells (lanes 1 and 2, mock transfected COS cell supernatant; lanes 3 and 4, NDF transfected COS cell supernatant). See Section 10., *infra*. Tyrosine phosphorylation was determined by the tyrosine kinase stimulation assay described in Section 8.2., *infra*.

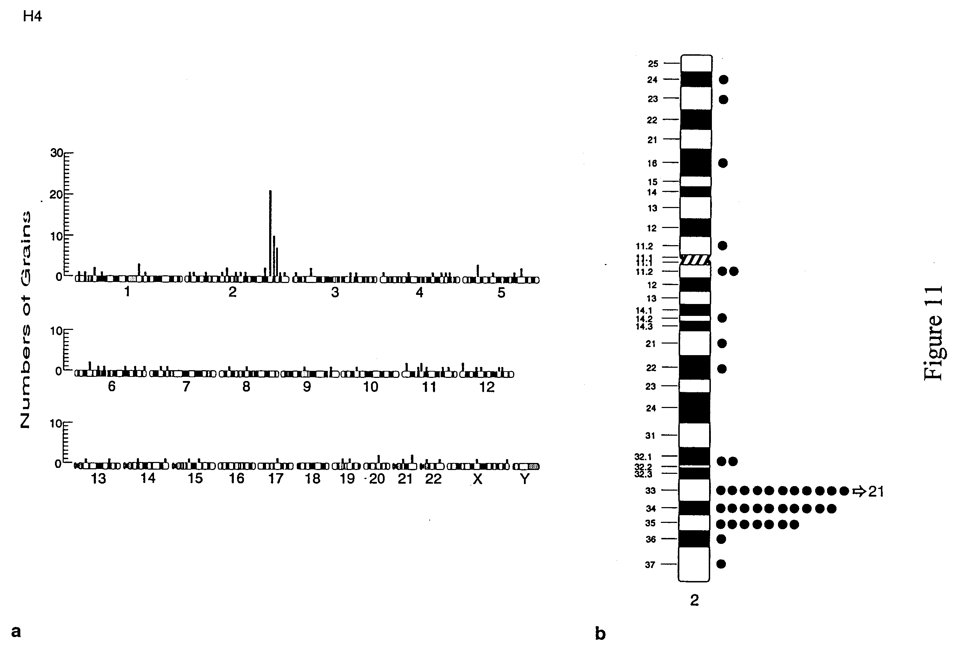

FIG. 11. Regional location of the HER4 gene to human chromosome 2 band q33. (A) Distribution of 124 sites of hybridization on human chromosomes. (B) Distribution of autoradiographic grains on diagram of chromosome 2.

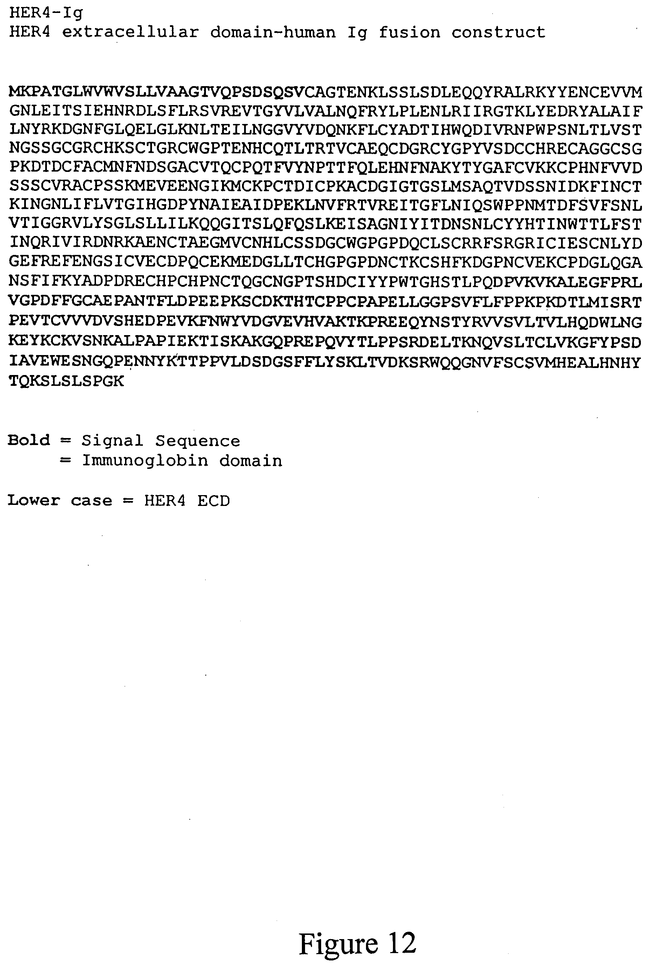

FIG. 12. Amino acid sequence of HER4-Ig fusion protein [SEQ ID NO: 10] (Section 5.4., *infra*).

## 5. DETAILED DESCRIPTION OF THE INVENTION

The present invention is directed to HER4/p180$^{erbB4}$ ("HER4"), a closely related yet distinct member of the Human EGF Receptor (HER)/*neu* subfamily of receptor tyrosine kinases, as well as HER4-encoding polynucleotides (e.g., cDNAs, genomic DNAs, RNAs, anti-sense RNAs, etc.), the production of mature and precursor forms of HER4 from a *HER4* polynucleotide coding sequence, recombinant HER4 expression vectors, HER4 analogues and derivatives, anti-HER4 antibodies, HER4 ligands, and diagnostic and therapeutic uses of HER4 polynucleotides, polypeptides, ligands, and antibodies in the field of human oncology and neurobiology.

The invention also reveals an apparent functional relationship between the HER4 and HER2 receptors involving HER4-mediated phosphorylation of HER2, potentially via intracellular receptor crosstalk or receptor dimerization. In this connection, the invention also provides a HER4 ligand capable of inducing cellular differentiation in breast carcinoma cells that appears to involve HER4-mediated phosphorylation of HER2. Furthermore, applicants' data provide evidence that NDF/HRG-α mediate biological effects on certain cells not solely through HER2, as has been reported in the literature, but instead by means of a direct interaction with HER4, or through an interaction with a HER2/ HER4 complex. In cell lines expressing both HER2 and HER4, binding of NDF to HER4 may stimulate HER2 either by heterodimer formation of these two related receptors or by intracellular receptor crosstalk.

Unless otherwise indicated, the practice of the present invention utilizes standard techniques of molecular biology and molecular cloning, microbiology, immunology, and recombinant DNA known in the art. Such techniques are described and explained throughout the literature, and can be found in a number of more comprehensive publications such as, for example, Maniatis et al, Molecular Cloning; A Laboratory Manual (Second Edition, 1989).

## 5.1. HER4 POLYNUCLEOTIDES

One aspect of the present invention is directed to HER4 polynucleotides, including recombinant polynucleotides encoding the prototype HER4 polypeptide shown in FIG. 1, polynucleotides which are related or are complementary thereto, and recombinant vectors and cell lines incorporating such recombinant polynucleotides. The term "recombinant polynucleotide" as used herein refers to a polynucleotide of genomic, cDNA, synthetic or semisynthetic origin which, by virtue of its origin or manipulation, is not associated with any portion of the polynucleotide with which it is associated in nature, and may be linked to a polynucleotide other than that to which it is linked in nature, and includes single or double stranded polymers of ribonucleotides, deoxyribonucleotides, nucleotide analogs, or combinations thereof. The term also includes various modifications known in the art, including but not limited to radioactive and chemical labels, methylation, caps, internucleotide modifications such as those with charged linkages (e.g., phosphorothothioates, phosphorodithothioates, etc.) and uncharged linkages (e.g., methyl phosphonates, phosphotriesters, phosphoamidites, carbamites, etc.), as well as those containing pendant moeties, intercalcators, chelators, alkylators, etc. Related polynucleotides are those having a contiguous stretch of about 200 or more nucleotides and sharing at least about 80% homology to a corresponding sequence of nucleotides within the nucleotide sequence disclosed in FIG. 1. Several particular embodiments of such HER4 polynucleotides and vectors are provided in example Sections 6 and 7, *infra*.

HER4 polynucleotides may be obtained using a variety of general techniques known in the art, including molecular cloning and chemical synthetic methods. One method by which the molecular cloning of cDNAs encoding the prototype HER4 polypeptide of the invention (FIG. 1), as well as several HER4 polypeptide variants, is described by way of example in Section 6., *infra*. Conserved regions of the sequences of EGFR, HER2, HER3, and Xmrk are used for selection of the degenerate oligonucleotide primers which are then used to isolate HER4. Since many of these sequences have extended regions of amino acid identity, it is difficult to determine if a short PCR fragment represents a unique molecule or merely the species-specific counterpart of EGFR, HER2, or HER3. Often the species differences for one protein are as great as the differences within species for two distinct proteins. For example, fish Xmrk has regions of 47/55 (85%) amino acid identity to human EGFR, suggesting it might be the fish EGFR, however isolation of another clone that has an amino acid sequence identical to Xmrk in this region (57/57) shows a much higher homology to human EGFR in its flanking sequence (92% amino acid homology) thereby suggesting that it, and not Xmrk, is the fish EGFR (Wittbrodt, J. et al., 1989, Nature 342: 415-421). As described in Section 6., *infra*, it was necessary to confirm that a murine HER4/erbB4 PCR fragment was indeed a unique gene, and not the murine homolog of EGFR, HER2, or HER3, by isolating genomic fragments corresponding to murine EGFR, erbB2 and erbB3. Sequence analysis of these clones confirmed that this fragment was a novel member of the EGFR family. Notably a region of the murine clone had a stretch of 60/64 amino acid identity to human HER2, but comparison with the amino acid and DNA sequences of the other EGFR homologs from the same species (mouse) firmly established it encoded a novel transcript.

HER4 polynucleotides may be obtained from a variety of cell sources which produce HER4-like activities and/or which express HER4-encoding mRNA. In this connection, applicants have identified a number of suitable human cell sources for HER4 polynucleotides, including but not limited to brain, cerebellum, pituitary, heart, skeletal muscle, and a variety of breast carcinoma cell lines (see Section 6., *infra*).

For example, polynucleotides encoding HER4 polypeptides may be obtained by cDNA cloning from RNA isolated and purified from such cell sources or by genomic cloning. Either cDNA or genomic libraries of clones may be prepared using techniques well known in the art and may be screened for particular HER4-encoding DNAs with nucleotide probes which are substantially complementary to any portion of the HER4 gene. Various PCR cloning techniques may also be used to obtain the HER4 polynucleotides of the invention. A number of PCR cloning protocols suitable for the isolation of HER4 polynucleotides have been reported in the literature (see, for example, PCR protocols: A Guide to Methods and Applications, Eds. Inis et al., Academic Press, 1990).

For the construction of expression vectors, polynucleotides containing the entire coding region of the desired HER4 may be isolated as full length clones or prepared by splicing two or more polynucleotides together. Alternatively, HER4-encoding DNAs may be synthesized in whole or in part by chemical synthesis using techniques standard in the art. Due to the inherent degeneracy of nucleotide coding sequences, any polynucleotide encoding the desired HER4 polypeptide may be used for recombinant expression. Thus, for example, the nucleotide sequence encoding the prototype HER4 of the invention provided in FIG. 1 may be altered by substituting nucleotides such that the same HER4 product is obtained.

The invention also provides a number of useful applications of the the HER4 polynucleotides of the invention, including but not limited to their use in the preparation of HER4 expression vectors, primers and probes to detect and/or clone HER4, and diagnostic reagents. Diagnostics based upon HER4 polynucleotides include various hybridization and PCR assays known in the art, utilizing HER4 polynucleotides as primers or probes, as appropriate. One particular aspect of the invention relates to a PCR kit comprising a pair of primers capable of priming cDNA synthesis in a PCR reaction, wherein each of the primers is a HER4 polynucleotide of the invention. Such a kit may be useful in the diagnosis of certain human cancers which are characterized by aberrant HER4 expression. For example, certain human carcinomas may overexpress HER4 relative to their normal cell counterparts, such as human carcinomas of the breast. Thus, detection of HER4 overexpression mRNA in breast tissue may be an indication of neoplasia. In another, related embodiment, human carcinomas characterized by overexpression of HER2 and expression or overexpression of HER4 may be diagnosed by a polynucleotide-based assay kit capable of detecting both HER2 and HER4 mRNAs, such a kit comprising, for example, a set of PCR primer pairs derived from divergent sequences in the HER2 and HER4 genes, respectively.

## 5.2. HER4 POLYPEPTIDES

Another aspect of the invention is directed to HER4 polypeptides, including the prototype HER4 polypeptide provided herein, as well as polypeptides derived from or having substantial homology to the amino acid sequence of the prototype HER4 molecule. The term "polypeptide" in this context refers to a polypeptide prepared by synthetic or recombinant means, or which is isolated from natural sources. The term "substantially homologous" in this context refers to polypeptides of about 80 or more amino acids sharing greater than about 90% amino acid homology to a corresponding contiguous amino acid sequence in the prototype HER4 primary structure (FIG. 1). The term "prototype HER4" refers to a polypeptide having the amino acid sequence of precursor or mature HER4 as provided in FIG. 1, which is encoded by the consensus cDNA nucleotide sequence also provided therein, or by any polynucleotide sequence which encodes the same amino acid sequence.

HER4 polypeptides of the invention may contain deletions, additions or substitutions of amino acid residues relative to the sequence of the prototype HER4 depicted in FIG. 1 which result in silent changes thus producing a bioactive product. Such amino acid substitutions may be made on the basis of similarity in polarity, charge, solubility, hydrophobicity, hydrophilicity and/or the amphipathic nature of the resides involved. For example, negatively charged amino acids include aspartic acid and glutamic acid; positively charged amino acids include lysine and arginine; amino acids with uncharged polar head groups or nonpolar head groups having similar hydrophilicity values include the following: leucine, isoleucine, valine; glycine, alanine; asparagine, glutamine; serine, threonine; phenylalanine, tyrosine.

The HER4 polypeptide depicted in FIG. 1 has all of the fundamental structural features characterizing the EGFR-family of receptor tyrosine kinases (Hanks et al., 1988, Science 241: 42-52). The precursor contains a single hydrophobic stretch of 26 amino acids characteristic of a transmembrane region that bisects the protein into a 625 amino acid extracellular ligand binding domain, and a 633 amino acid C-terminal cytoplasmic domain. The ligand binding domain can be further divided into 4 subdomains (I - IV), including two cysteine-rich regions (II, residues 186-334; and IV, residues 496-633), and two flanking domains (I, residues 29-185; and III, residues 335-495) that may define specificity for ligand binding (Lax et al., 1988, Mol. Cell. Biol. 8:1970-78). The extracellular domain of HER4 is most similar to HER3, where domains II-IV of HER4 share 56-67% identity to the respective domains of HER3. In contrast, the same regions of EGFR and HER2 exhibit 43-51% and 34-46% homology to HER4, respectively (FIG. 4). The 4 extracellular subdomains of EGFR and HER2 share 39-50% identity. HER4 also conserves all 50 cysteines present in the extracellular portion of EGFR, HER2, and HER3, except that the HER2 protein lacks the fourth cysteine in domain IV. There are 11 potential N-linked glycosylation sites in HER4, conserving 4 of 12 potential sites in EGFR, 3 of 8 sites in HER2, and 4 of 10 sites in HER3.

Following the transmembrane domain of HER4 is a cytoplasmic juxtamembrane region of 37 amino acids. This region shares the highest degree of homology with EGFR (73% amino acid identity) and contains two consensus protein kinase C phosphorylation sites at amino acid residue numbers 679 (Serine) and 699 (Threonine) in the FIG. 1 sequence, the latter of which is present in EGFR and HER2. Notably, HER4 lacks a site analogous to Thr654 of EGFR. Phosphorylation of this residue in the EGFR appears to block ligand-induced internalization and plays an important role in its transmembrane signaling (Livneh et al., 1988, Mol. Cell. Biol. 8: 2302-08). HER4 also contains Thr692 analogous to Thr694 of HER2. This threonine is absent in EGFR and HER3 and has been proposed to impart cell-type specificity to the mitogenic and transforming activity of the HER2 kinase (DiFiore et al. 1992, EMBO J. 11: 3927-33). The juxtamembrane region of HER4 also contains a MAP kinase consensus phosphorylation site at amino acid number 699 (Threonine), in a position homologous to Thr699 of EGFR which is phosphorylated by MAP kinase in response to EGF stimulation (Takishima et al., 1991, Proc. Natl. Acad. Sci. U.S.A. 88: 2520-25).

The remaining cytoplasmic portion of HER4 consists of a 276 amino acid tyrosine kinase domain, an acidic helical structure of 38 amino acids that is homologous to a domain required for ligand-induced internalization of the EGFR (Chen et al., 1989, Cell 59:33-43), and a 282 amino acid region containing 18 tyrosine residues characteristic of the autophosphorylation domains of other EGFR-related proteins (FIG. 4). The 276 amino acid tyrosine kinase domain conserves all the diagnostic structural motifs of a tyrosine kinase, and is most related to the catalytic domains of EGFR (79% identity) and HER2 (77% identity), and to a lesser degree, HER3 (63% identity). In this same region, EGFR and HER2 share 83% identity. Examples of the various conserved structural motifs include the following: the ATP-binding motif (GXGXXG) [SEQ ID NO: 11] with a distal lysine residue that is predicted to be involved in the phosphotransfer reaction (Hanks et al., 198, Science 241: 42-52; Hunter and Cooper, in The Enzymes Vol. 17 (eds. Boyer and Krebs) pp. 191-246 (Academic Press 1986)); tyrosine-kinase specific signature sequences (DLAARN [SEQ ID NO: 12] and PIKWMA [SEQ ID NO: 13]) and Tyr875 (FIG. 4), a residue that frequently serves as an autophosphorylation site in many tyrosine kinases (Hunter and Cooper, *supra*); and approximately 15 residues that are either highly or completely conserved among all known protein kinases (Plowman et al., 1990, Proc. Natl. Acad. Sci. U.S.A. 87: 4905-09; Hanks et al., *supra*). The C-terminal 282 amino acids of HER4 has limited homology with HER2 (27%) and EGFR (19%). However, the C-terminal domain of each EGFR-family receptor is proline-rich and conserves stretches of 2-7 amino acids that are generally centered around a tyrosine residue. These residues include the major tyrosine autophosphorylation sites of EGFR at Tyr1068, Tyr1086, Tyr1148, and Tyr1173 (FIG. 4, filled triangles; Margolis et al., 1989, J. Biol. Chem. 264: 10667-71).

## 5.3. RECOMBINANT SYNTHESIS OF HER4 POLYPEPTIDES

The HER4 polypeptides of the invention may be produced by the cloning and expression of DNA encoding the desired HER4 polypeptide. Such DNA may be ligated into a number of expression vectors well known in the art and suitable for use in a number of acceptable host organisms, in fused or mature form, and may contain a signal sequence to permit secretion. Both prokaryotic and eukaryotic host expression systems may be employed in the production of recombinant HER4 polypeptides. For example, the prototype HER4 precursor coding sequence or its functional equivalent may be used in a host cell capable of processing the precursor correctly. Alternatively, the coding sequence for mature HER4 may be used to directly express the mature HER4 molecule. Functional equivalents of the HER4 precursor coding sequence include any DNA sequence which, when expressed inside the appropriate host cell, is capable of directing the synthesis, processing and/or export of HER4.

Production of a HER4 polypeptide using recombinant DNA technology may be divided into a four-step process for the purposes of description: (1) isolation or generation of DNA encoding the desired HER4 polypeptide; (2) construction of an expression vector capable of directing the synthesis of the desired HER4 polypeptide; (3) transfection or transformation of appropriate host cells capable of replicating and expressing the HER4 coding sequence and/or processing the initial product to produce the desired HER4 polypeptide; and (4) identification and purification of the desired HER4 product.

### 5.3.1. ISOLATION OR GENERATION OF HER4 ENCODING DNA

HER4-encoding DNA, or functional equivalents thereof, may be used to construct recombinant expression vectors which will direct the expression of the desired HER4 polypeptide product. In a specific embodiment, DNA encoding the prototype HER4 polypeptide (FIG. 1), or fragments or functional equivalents thereof, may be used to generate the recombinant molecules which will direct the expression of the recombinant HER4 product in appropriate host cells. HER4-encoding nucleotide sequences may be obtained from a variety of cell sources which produce HER4-like activities and/or which express HER4-encoding mRNA. For example, HER4-encoding cDNAs may be obtained from the breast adenocarcinoma cell line MDA-MB-453 (ATCC HTB131) as described in Section 6., *infra*. In addition, a number of human cell sources are suitable for obtaining HER4 cDNAs, including but not limited to various epidermoid and breast carcinoma cells, and normal heart, kidney, and brain cells (see Section 6.2.3., *infra*).

The HER4 coding sequence may be obtained by molecular cloning from RNA isolated and purified from such cell sources or by genomic cloning. Either cDNA or genomic libraries of clones may be prepared using techniques well known in the art and may be screened for particular HER4-encoding DNAs with nucleotide probes which are substantially complementary to any portion of the HER4 gene. Alternatively, cDNA or genomic DNA may be used as templates for PCR cloning with suitable oligonucleotide primers. Full length clones, i.e., those containing the entire coding region of the desired HER4 may be selected for constructing expression vectors, or overlapping cDNAs can be ligated together to form a complete coding sequence. Alternatively, HER4-encoding DNAs may be synthesized in whole or in part by chemical synthesis using techniques standard in the art.

### 5.3.2. CONSTRUCTION OF HER4 EXPRESSION VECTORS

Various expression vector/host systems may be utilized equally well by those skilled in the art for the recombinant expression of HER4 polypeptides. Such systems include but are not limited to microorganisms such as bacteria transformed with recombinant bacteriophage DNA, plasmid DNA or cosmid DNA expression vectors containing the desired HER4 coding sequence; yeast transformed with recombinant yeast expression vectors containing the desired HER4 coding sequence; insect cell systems infected with recombinant virus expression vectors (e.g., baculovirus) containing the desired HER4 coding sequence; plant cell systems infected with recombinant virus expression vectors (e.g., cauliflower mosaic virus CaMV; tobacco mosaic virus, TMV) or transformed with recombinant plasmid expression vectors (e.g., Ti plasmid) containing the desired HER4 coding sequence; or animal cell systems infected with recombinant virus expression vectors (e.g., adenovirus, vaccinia virus) including cell lines engineered to contain multiple copies of the HER4 DNA either stably amplified (e.g., CHO/dhfr, CHO/glutamine synthetase) or unstably amplified in double-minute chromosomes (e.g., murine cell lines).

The expression elements of these vectors vary in their strength and specificities. Depending on the host/vector system utilized, any one of a number of suitable transcription and translation elements may be used. For instance, when cloning in mammalian cell systems, promoters isolated from the genome of mammalian cells, (e.g., mouse metallothionein promoter) or from viruses that grow in these cells, (e.g., vaccinia virus 7.5K promoter or Moloney murine sarcoma virus long terminal repeat) may be used. Promoters produced by recombinant DNA or synthetic techniques may also be used to provide for transcription of the inserted sequences.

Specific initiation signals are also required for sufficient translation of inserted protein coding sequences. These signals include the ATG initiation codon and adjacent sequences. In cases where the entire HER4 gene including its own initiation codon and adjacent sequences are inserted into the appropriate expression vectors, no additional translational control signals may be needed. However, in cases where only a portion of the coding sequence is inserted, exogenous translational control signals, including the ATG initiation codon must be provided. Furthermore, the initiation codon must be in phase with the reading frame of the HER4 coding sequences to ensure translation of the entire insert. These exogenous translational control signals and initiation codons can be of a variety of origins, both natural and synthetic. The efficiency of expression may be enhanced by the inclusion of transcription attenuation sequences, enhancer elements, etc.

For example, in cases where an adenovirus is used as an expression vector, the desired HER4 coding sequence may be ligated to an adenovirus transcription/translation control complex, e.g., the late promoter and tripartite leader sequence. This chimeric gene may then be inserted in the adenovirus genome by *in vitro* or *in vivo* recombination. Insertion in a non-essential region of the viral genome (e.g., region E3 or E4) will result in a recombinant virus that is viable and capable of expressing HER4 in infected hosts. Similarly, the vaccinia 7.5K promoter may be used. An alternative expression system which could be used to express HER4 is an insect system. In one such system, Autographa californica nuclear polyhidrosis virus (AcNPV) is used as a vector to express foreign genes. The virus grows in Spodoptera frugiperda cells. The HER4 coding sequence may be cloned into non-essential regions (for example the polyhedrin gene) of the virus and placed under control of an AcNPV promoter (for example the polyhedrin promoter). Successful insertion of the HER4 coding sequence will result in inactivation of the polyhedrin gene and production of non-occluded recombinant virus (i.e., virus lacking the proteinaceous coat encoded by the polyhedrin gene). These recombinant viruses are then used to infect Spodoptera frugiperda cells in which the inserted gene is expressed. Yet another approach uses retroviral vectors prepared in amphotropic packaging cell lines, which permit high efficiency expression in numerous cells types. This method allows one to assess cell-type specific processing, regulation or function of the inserted protein coding sequence.

In addition, a host cell strain may be chosen which modulates the expression of the inserted sequences, or modifies and processes the gene product in the specific fashion desired. Expression from certain promotes can be elevated in the presence of certain inducers. (e.g., zinc and cadmium ions for metallothionein promoters). Therefore, expression of the recombinant HER4 polypeptide may be controlled. This is important if the protein product of the cloned foreign gene is lethal to host cells. Furthermore, modifications (e.g., phosphorylation) and processing (e.g., cleavage) of protein products are important for the function of the protein. Different host cells have characteristic and specific mechanisms for the post-translational processing and modification of protein. Appropriate cell lines or host systems can be chosen to ensure the correct modification and processing of the foreign protein expressed.

### 5.3.3. TRANSFORMANTS EXPRESSING HER4 GENE PRODUCTS

The host cells which contain the recombinant coding sequence and which express the desired HER4 polypeptide product may be identified by at least four general approaches (a) DNA-DNA, DNA-RNA or RNA-antisense RNA hybridization; (b) the presence or absence of "marker" gene functions; (c) assessing the level of transcription as measured by the expression of HER4 mRNA transcripts in the host cell; and (d) detection of the HER4 product as measured by immunoassay and, ultimately, by its biological activities.

In the first approach, for example, the presence of HER4 coding sequences inserted into expression vectors can be detected by DNA-DNA hybridization using hybridization probes and/or primers for PCR reactions comprising polynucleotides that are homologous to the HER4 coding sequence.

In the second approach, the recombinant expression vector/host system can be identified and selected based upon the presence or absence of certain "marker" gene functions (e.g., thymidine kinase activity, resistance to antibiotics, resistance to methotrexate (MTX), resistance to methionine sulfoximine (MSX), transformation phenotype, occlusion body formation in baculovirus, (etc.). For example, if the HER4 coding sequence is inserted within a marker gene sequence of the vector, recombinants containing that coding sequence can be identified by the absence of the marker gene function. Alternatively, a marker gene can be placed in tandem with the HER4 sequence under the control of the same or different promoter used to control the expression of the HER4 coding sequence. Expression of the marker in response to induction or selection indicates expression of the HER4 coding sequence. In a particular embodiment described by way of example herein, a HER4 expression vector incorporating glutamine synthetase as a selectable marker is constructed, used to transfect CHO cells, and amplified expression of HER4 in CHO cells is obtained by selection with increasing concentration of MSX.

In the third approach, transcriptional activity for the HER4 coding region can be assessed by hybridization assays. For example, polyadenylated RNA can be isolated and analyzed by Northern blot using a probe homologous to the HER4 coding sequence or particular portions thereof. Alternatively, total nucleic acids of the host cell may be extracted and assayed for hybridization to such probes.

In the fourth approach, the expression of HER4 can be assessed immunologically, for example by Western blots, immunoassays such as radioimmunoprecipitation, enzyme-linked immunoassays and the like. Alternatively, expression of HER4 may be assessed by detecting a biologically active product. Where the host cell secretes the gene product the cell free media obtained from the cultured transfectant host cell may be assayed for HER4 activity. Where the gene product is not secreted, cell lysates may be assayed for such activity. In either case, assays which measure ligand binding to HER4, HER4 phosphorylation, or other bioactivities of HER4 may be used.

### 5.4. ANTI-HER4 ANTIBODIES

The invention is also directed to polyclonal and monoclonal antibodies which recognize epitopes of HER4 polypeptides. Anti-HER4 antibodies are expected to have a variety of useful applications in the field of oncology, several of which are described generally below. More detailed and specific descriptions of various uses for anti-HER4 antibodies are provided in the sections and subsections which follow. Briefly, anti-HER4 antibodies may be used for the detection and quantification of HER4 polypeptide expression in cultured cells, tissue samples, and *in vivo*. Such immunological detection of HER4 may be used, for example, to identify, monitor, and assist in the prognosis of neoplasms characterized by aberrant or attenuated HER4 expression and/or function. Additionally, monoclonal antibodies recognizing epitopes from different parts of the HER4 structure may be used to detect and/or distinguish between native HER4 and various subcomponent and/or mutant forms of the molecule. Anti-HER4 antibody preparations are also envisioned as useful biomodulatory agents capable of effectively treating particular human cancers. In addition to the various diagnostic and therapeutic utilities of anti-HER4 antibodies, a number of industrial and research applications will be obvious to those skilled in the art, including, for example, the use of anti-HER4 antibodies as affinity reagents for the purification of HER4 polypeptides, and as immunological probes for elucidating the biosynthesis, metabolism and biological functions of HER4.

Anti-HER4 antibodies may be useful for influencing cell functions and behaviors which are directly or indirectly mediated by HER4. As an example, modulation of HER4 biological activity with anti-HER4 antibodies may influence HER2 activation and, as a consequence, modulate intracellular signals generated by HER2. In this regard, anti-HER4 antibodies may be useful to effectively block ligand-induced, HER4-mediated activation of HER2, thereby affecting HER2 biological activity. Conversely, anti-HER4 antibodies capable of acting as HER4 ligands may be used to trigger HER4 biological activity and/or initiate a ligand-induced, HER4-mediated effect on HER2 biological activity, resulting in a cellular response such as differentiation, growth inhibition, etc.

Additionally, anti-HER4 antibodies conjugated to cytotoxic compounds may be used to selectively target such compounds to tumor cells expressing HER4, resulting in tumor cell death and reduction or eradication of the tumor. In a particular embodiment, toxin-conjugated antibodies having the capacity to bind to HER4 and internalize into such cells are administered systemically for targeted cytotoxic effect. The preparation and use of radionuclide and toxin conjugated anti-HER4 antibodies are further described in Section 5.5., *infra*.

Overexpression of HER2 is associated with several human cancers. Applicants' data indicate that HER4 is expressed in certain human carcinomas in which HER2 overexpression is present. Therefore, anti-HER4 antibodies may have growth and differentiation regulatory effects on cells which overexpress HER2 in combination with HER4 expression, including but not limited to breast adenocarcinoma cells. Accordingly, this invention includes antibodies capable of binding to the HER4 receptor and modulating HER2 or HER2-HER4 functionality, thereby affecting a response in the target cell. For the treatment of cancers involving HER4-mediated regulation of HER2 biological activity, agents capable of selectively and specifically affecting the intracellular molecular interaction between these two receptors may be conjugated to internalizing anti-HER4 antibodies. The specificity of such agents may result in biological effects only in cells which co-express HERS and HER4, such as breast cancer cells.

Various procedures known in the art may be used for the production of polyclonal antibodies to epitopes of HER4. For the production of polyclonal antibodies, a number of host animals are acceptable for the generation of anti-HER4 antibodies by immunization with one or more injections of a HER4 polypeptide preparation, including but not limited to rabbits, mice, rats, etc. Various adjuvants may be used to increase the immunological response in the host animal, depending on the host species, including but not limited to Freund's (complete and incomplete), mineral gels such as aluminum hydroxide, surface active substances such as lysolecithin, pluronic polyols, polyanions, oil emulsions, keyhole lympet hemocyanins, dinitrophenol, and potentially useful human adjuvants such as BCG (bacille Calmette-Guerin) and Corynebacterium parvum.

A monoclonal antibody to an epitope of HER4 may be prepared by using any technique which provides for the production of antibody molecules by continuous cell lines in culture. These include but are not limited to the hybridoma technique originally described by Kohler and Milstein (1975, Nature 256, 495-497), and the more recent human B-cell hybridoma technique (Kosbor et al., 1983, Immunology Today 4:72) and EBV-hybridoma technique (Cole et al., 1985, Monoclonal Antibodies and Cancer Therapy, Alan R. Liss, Inc., pp. 77-96). In additions techniques developed for the production of "chimeric antibodies" by splicing the genes from a mouse antibody molecule of appropriate antigen specificity together with genes from a human antibody molecule of appropriate biological activity may be used (Morrison et al., 1984, Proc. Natl. Acad. Sci., 81:6851-6855; Neuberger et al., 1984, Nature, 312:604-608; Takeda et al., 1985, Nature, 314:452-454). Alternatively, techniques described for the production of single chain antibodies (U.S. Patent 4,946,778) can be adapted to produce HER4-specific single chain antibodies. Recombinant human or humanized versions of anti-HER4 monoclonal antibodies are a preferred embodiment for human therapeutic applications. Humanized antibodies may be prepared according to procedures in the literature (e.g., Jones et al., 1986, Nature 321: 522-25; Reichman et al., 1988, Nature 332: 323-27; Verhoeyen et al., 1988, Science 239: 1534-36). The recently described "gene conversion mutagenesis" strategy for the production of humanized anti-HER2 monoclonal antibody may also be employed in the production of humanized anti-HER4 antibodies (Carter et al., 1992, Proc. Natl. Acad. Sci. U.S.A. 89: 4285-89). Alternatively, techniques for generating a recombinant phage library of random combinations of heavy and light regions may be used to prepare recombinant anti-HER4 antibodies (e.g., Huse et al., 1989, Science 246: 1275-81).

As an example, anti-HER4 monoclonal antibodies may be generated by immunization of mice with cells selectively overexpressing HER4 (e.g., CHO/HER4 21-2 cells as deposited with the ATCC) or with partially purified recombinant HER4 polypeptides. In one embodiment, the full length HER4 polypeptide (FIG. 1) may be expressed in Baculovirus systems, and membrane fractions of the recombinant cells used to immunize mice. Hybridomas are then screened on CHO/HER4 cells (e.g., CHO HER4 21-2 cells as deposited with the ATCC) to identify monoclonal antibodies reactive with the extracellular domain of HER4. Such monoclonal antibodies may be evaluated for their ability to block NDF, or HepG2-differentiating factor, binding to HER4; for their ability to bind and stay resident on the cell surface, or to internalize into cells expressing HER4; and for their ability to directly upregulate or downregulate HER4 tyrosine autophosphorylation and/or to directly induce a HER4-mediated signal resulting in modulation of cell growth or differentation. In this connection, monoclonal antibodies N28 and N29, directed to HER2, specifically bind HER2 with high affinity. However, monoclonal N29 binding results in receptor internalization and downregulation, morphologic differentiation, and inhibition of HER2 expressing tumor cells in athymic mice. In contrast, monoclonal N28 binding to HER2 expressing cells results in stimulation of autophosphorylation, and an acceleration of tumor cell growth both *in vitro* and *in vivo* (Bacus et al., 1992, Cancer Res. 52: 2580-89; Stancovski et al., 1991, Proc. Natl. Acad. Sci. U.S.A. 88: 8691-95). In yet another embodiment, a soluble recombinant HER4-Immunoglobulin (HER4-Ig) fusion protein is expressed and purified on a Protein A affinity column. The amino acid sequence of one such HER4-Ig fusion protein is provided in FIG. 12. The soluble HER4-Ig fusion protein may then be used to screen phage libraries designed so that all available combinations of a variable domain of the antibody binding site are presented on the surfaces of the phages in the library. Recombinant anti-HER4 antibodies may be propagated from phage which specifically recognize the HER4-Ig fusion protein.

Antibody fragments which contain the idiotype of the molecule may be generated by known techniques. For example, such fragments include but are not limited to: the $F(ab')_2$ fragment which can be produced by pepsin digestion of the intact antibody molecule; the Fab' fragments which can be generated by reducing the disulfide bridges of the $F(ab')_2$ fragment, and the two Fab fragments which can be generated by treating the antibody molecule with papain and a reducing agent. Alternatively, Fab expression libraries may be constructed (Huse et al., 1989, Science, 246:1275-1281) to allow rapid and easy identification of monoclonal Fab fragments with the desired specificity to HER4 protein.

### 5.5. DIAGNOSTIC METHODS

The invention also relates to the detection of human neoplastic conditions, particularly carcinomas of epithelial origin, and more particularly human breast carcinomas. In one embodiment, oligomers corresponding to portions of the consensus HER4 cDNA sequence provided in FIG. 1 are used for the quantitative detection of HER4 mRNA levels in a human biological sample, such as blood, serum, or tissue biopsy samples, using a suitable hybridization or PCR format assay, in order to detect cells or tissues expressing abnormally high levels of HER4 as an indication of neoplasia. In a related embodiment, detection of HER4 mRNA may be combined with the detection HER2 mRNA overexpression, using appropriate HER2 sequences, to identify neoplasias in which a functional relationship between HER2 and HER4 may exist.

In another embodiment, labeled anti-HER4 antibodies or antibody derivatives are used to detect the presence of HER4 in biological samples, using a variety of immunoassay formats well known in the art, and may be used for in situ diagnostic radioimmunoimaging. Current diagnostic and staging techniques do not routinely provide a comprehensive scan of the body for metastatic tumors. Accordingly, anti-HER4 antibodies labeled with, for example, fluorescent, chemiluminescent, and radioactive molecules may overcome this limitation. In a preferred embodiment, a gamma-emitting diagnostic radionuclide is attached to a monoclonal antibody which is specific for an epitope of HER4, but not significantly cross-reactive with other EGFR-family members. The labeled antibody is then injected into a patient systemically, and total body imaging for the distribution and density of HER4 molecules is performed using gamma cameras, followed by localized imaging using computerized tomography or magnetic resonance imaging to confirm and/or evaluate the condition, if necessary. Preferred diagnostic radionuclides include but are not limited to technetium-99m, indium-111, iodine-123, and iodine-131.

Recombinant antibody-metallothionein chimeras (Ab-MTs) may be generated as recently described (Das et al., 1992, Proc. Natl. Acad. Sci. U.S.A. 89: 9749-53). Such Ab-MTs can be loaded with technitium-99m by virtue of the metallothionein chelating function, and may offer advantages over chemically conjugated chelators. In particular, the highly conserved metallothionein structure may result in minimal immunogenicity.

## 5.6. TARGETED CANCER THERAPY

The invention is also directed to methods for the treatment of human cancers involving abnormal expression and/or function of HER4 and cancers in which HER2 overexpression is combined with the proximate expression of HER4, including but not limited to human breast carcinomas and other neoplasms overexpressing HER4 or overexpressing HER2 in combination with expression of HER4. The cancer therapy methods of the invention are generally based on treatments with unconjugated, toxin- or radionuclide-conjugated HER4 antibodies, ligands, and derivatives or fragments thereof. In one specific embodiment, such HER4 antibodies may be used for systemic and targeted therapy of certain cancers overexpressing HER2 and/or HER4, such as metastatic breast cancer, with minimal toxicity to normal tissues and organs. Importantly, in this connection, an anti-HER2 monoclonal antibody has been shown to inhibit the growth of human tumor cells overexpressing HER2 (Bacus et al., 1992, Cancer Res. 52: 2580-89). In addition to conjugated antibody therapy, modulation of NDF signaling through HER4 may provide a means to affect the growth and differentiation of cells overexpressing HER2, such as certain breast cancer cells, using HER4-neutralizing monoclonal antibodies, NDF/HER4 antagonists, monoclonal antibodies or ligands which act as super-agonists for HER4 activation, or agents which block the interaction between HER2 and HER4, either by disrupting heterodimer formation or by blocking HER-mediated phosphorylation of the HER2 substrate.

For targeted immunotoxin-mediated cancer therapy, various drugs or toxins may be conjugated to anti-HER4 antibodies and fragments thereof, such as plant and bacterial toxins. For example, ricin, a cytotoxin from the *Ricinis communis* plant may be conjugated to an anti-HER4 antibody using methods known in the art (e.g., Blakey et al., 1988, Prog. Allergy 45: 50-90; Marsh and Neville, 1988, J. Immunol. 140: 3674-78). Once ricin is inside the cell cytoplasm, its A chain inhibits protein synthesis by inactivating the 60S ribosomal subunit (May et al., 1989, EMBO J. 8: 301-08). Immunotoxins of ricin are therefore extremely cytotoxic. However, ricin immunotoxins are not ideally specific because the B chain can bind to virtually all cell surface receptors, and immunotoxins made with ricin A chain alone have increased specificity. Recombinant or deglycosylated forms of the ricin A chain may-result in improved survival (i.e., slower clearance from circulation) of the immunotoxins. Methods for conjugating ricin A chain to antibodies are known (e.g., Vitella and Thorpe, in: Seminars in Cell Biology, pp47-58; Saunders, Philadelphia 1991). Additional toxins which may be used in the formulation of immunotoxins include but are not limited to daunorubicin, methotrexate, ribosome inhibitors (e.g., trichosanthin, trichokirin, gelonin, saporin, mormordin, and pokeweed antiviral protein) and various bacterial toxins (e.g., *Pseudomonas* endotoxin). Immunotoxins for targeted cancer therapy may be administered by any route which will result in antibody interaction with the target cancer cells, including systemic administration and injection directly to the site of tumor.

For targeted radiotherapy using anti-HER4 antibodies, preferred radionuclides for labeling include alpha, beta, and Auger electron emitters. Examples of alpha emitters include astatine 211 and bismuth 212; beta emitters include iodine 131, rhenium 188, copper 67 and yttrium 90; and iodine 125 is an example of an Auger electron emitter.

## 5.7. ASSAYS FOR THE IDENTIFICATION OF HER4 LIGANDS

Cell lines overexpressing a single member of the EGFR-family can be generated by transfection of a variety of parental cell types with an appropriate expression vector as described in section 7., *infra*. Candidate ligands, or partially purified preparations, may be applied to such cells and assayed for receptor binding and/or activation. For example, a CHO-KI cell line transfected with a HER4 expression plasmid and lacking detectable EGFR, HER2, or HER3 may be used to screen for HER4-specific ligands. A particular embodiment of such a cell line is described in Section 7., *infra* and has been deposited with the ATCC (CHO/HER4 21-2). Ligands may be identified by detection of HER4 autophosphorylation, stimulation of DNA synthesis, induction of morphologic differentiation, relief from serum or growth factor requirements in the culture media, and direct binding of labeled purified growth factor. The invention also relates to a bioassay for testing potential analogs of HER4 ligands based on a capacity to affect a biological activity mediated by the HER4 receptor.

## 5.8 HER4 ANALOGUES

The production and use of derivatives, analogues and peptides related to HER4 are also envisioned and are within the scope of the invention. Such derivatives, analogues and peptides may be used to compete with native HER4 for binding of HER4 specific ligand, thereby inhibiting HER4 signal transduction and function. The inhibition of HER4 function may be utilized in several applications, including but not limited to the treatment of cancers in which HER4 biological activity is involved.

In a specific embodiment, a series of deletion mutants in the HER4 nucleotide coding sequence depicted in FIG.1 may be constructed and analyzed to determine the minimum amino acid sequence requirements for binding of a HER4 ligand. Deletion mutants of the HER4 coding sequence may be constructed using methods known in the art which include but are not limited to use of nucleases and/or restriction enzymes; site-directed mutagenesis techniques, PCR, etc. The mutated polypeptides expressed may be assayed for their ability to bind HER4 ligand.

The DNA sequence encoding the desired HER4 analogue may then be cloned into an appropriate expression vector for overexpression in either bacteria or eukaryotic cells. Peptides may be purified from cell extracts in a number of ways including but not limited to ion-exchange chromatography or affinity chromatography using HER4 ligand or antibody. Alternatively, polypeptides may be synthesized by solid phase techniques followed by cleavage from resin and purification by high performance liquid chromatography.

## 6. EXAMPLE: ISOLATION OF cDNAs ENCODING HER4

EGFR and the related proteins, HER2, HER3, and Xmrk exhibit extensive amino acid homology in their tyrosine kinase domains (Kaplan et al., 1991, Nature 350: 158-160; Wen et al., 1992, Cell 69: 559-72; Holmes et al., 1992, Science 256: 1205-10; Hirai et al., 1987, Science 238: 1717-20). In addition, there is strict conservation of the exon-intron boundaries within the genomic regions that encode these catalytic domains (Wen et al., *supra*; Lindberg and Hunter, 1990, Mol. Cell. Biol. 10: 6316-24; and unpublished observations). Degenerate oligonucleotide primers were designed based on conserved amino acids encoded by a single exon or adjacent exons from the kinase domains of these four proteins. These primers were used in a polymerase chain reaction (PCR) to isolate genomic fragments corresponding to murine EGFR, erbB2 and erbB3. In addition, a highly related DNA fragment (designated MER4) was identified as distinct from these other genes. A similar strategy was used to obtain a cDNA clone corresponding to the human homologue of MER4 from the breast cancer cell line, MDA-MB-453. Using this fragment as a probe, several breast cancer cell lines and human heart were found to be an abundant source of the EGFR-related transcript. cDNA libraries were constructed using RNA from human heart and MDA-MB-453 cells, and overlapping clones were isolated spanning the complete open reading frame of HER4/erbB4.

### 6.1. MATERIALS AND METHODS

#### 6.1.1. MOLECULAR CLONING

Several pools of degenerate oligonucleotides were synthesized based on conserved sequences from EGFR-family members (Table I).

5'-ACNGTNTGGGARYTNAYHAC-3' [SEQ ID NO: 14]; 5'-CAYGTNAARATHACNGAYTTYGG-3' [SEQ ID NO: 15); 5'-GACGAATTCCNATHAARTGGATGGC [SEQ ID NO: 16]; 5'-ACAYTTNARDATDATCATRTANAC-3' [SEQ ID NO: 17]; 5'-AANGTCATNARYTCCCA-3' [SEQ ID NO: 18]; 5'-TCCAGNGCGATCCAYTTDATNGG-3' [SEQ ID NO: 19]; 5'-GGRTCDATCATCCARCCT-3' [SEQ ID NO: 20]; 5'-CTGCTGTCAGCATCGATCAT-3' [SEQ ID NO: 21]; TVWELMT [SEQ ID NO: 22]; HVKITDFG [SEQ ID NO: 23]; PIKWMA [SEQ ID NO: 13]; VYMIILK [SEQ ID NO: 24]; WELMTF [SEQ ID NO: 25]; PIKWMALE [SEQ ID NO: 26]; CWMIDP [SEQ ID NO: 27]

Total genomic DNA was isolated from subconfluent murine K1735 melanoma cells and used as a template with these oligonucleotide primers in a 40 cycle PCR amplification. PCR products were resolved on agarose gels and hybridized to $^{32}P$-labeled probes from the kinase domain of human EGFR and HER2. Distinct DNA bands were isolated and subcloned for sequence analysis. Using the degenerate oligonucleotides H4VWELM and H4VYMIIL as primers in a PCR amplification (Plowman et al., 1990, Proc. Natl. Acad. Sci. U.S.A. 87: 4905-09), one clone (MER4-85) was identified that contained a 144 nucleotide insert corresponding to murine erbB4. This $^{32}P$-labeled insert was used to isolate a 17-kilobase fragment from a murine T-cell genomic library (Stratagene, La Jolla, CA) that was found to contain two exons of the murine erbB4 gene. A specific oligonucleotide (4M3070) was synthesized based on the DNA sequence of an erbB4 exon, and used in a PCR protocol with a degenerate 5'-oligonucleotide (H4PIKWMA) on a template of single stranded MDA-MB-453 cDNA. This reaction generated a 260 nucleotide fragment (pMDAPIK) corresponding to human HER4. cDNA libraries were constructed in lambda ZAP II (Stratagene) from oligo(dT)-and specific-primed MDA-MB453 and human heart RNA (Plowman et al., *supra*; Plowman et al., 1990, Mol. Cell. Biol. 10: 1969-81). HER4-specific clones were isolated by probing the libraries with the

$^{32}P$-labeled insert from pMDAPIK. To complete the cloning of the 5'-portion of HER4, we used a PCR strategy to allow for rapid amplification of cDNA ends (Plowman et al., *supra*; Frohman et al., 1988, Proc. Natl. Acad. Sci. U.S.A. 85: 8998-9002). All cDNA clones and several PCR generated clones were sequenced on both strands using T7 polymerase with oligonucleotide primers (Tabor and Richardson, 1987, Proc. Natl. Acad. Sci. U.S.A. 84: 4767-71).

**TABLE I**
**OLIGONUCLEOTIDE PREPARATIONS FOR CLONING HER4**

| Designation | Nucleotide Sequence[1] | Degeneracy | Encoded Sequence | Orientation |
|---|---|---|---|---|
| H4TVWELM | 5'-ACNGTNTGGGARYTNAYHAC-3' | 256-fold | TVWELMT | sense |
| H4KITDFG | 5'-CAYGTNAARATHACNGAYTTYGG-3' | 768-fold | HVKITDFG | sense |
| H4PIKWMA | 5'-GACGAATTCCNATHAARTGGATGGC | 48-fold | PIKWMA | sense |
| H4VYMIIL | 5'-ACAYTTNARDATDATCATRTANAC-3' | 576-fold | VYMIILK | antisense |
| H4WELMTF | 5'-AANGTCATNARYTCCCA-3' | 32-fold | WELMTF | antisense |
| H4PIKWMA | 5'-TCCAGNGCGATCCAYTTDATNGG-3' | 96-fold | PIKWMALE | antisense |
| H4CWMIDP | 5'-GGRTCDATCATCCARCCT-3' | 12-fold | CWMIDP | antisense |
| 4M3070 | 5'-CTGCTGTCAGCATCGATCAT-3' | zero | erbB4 exon | antisense |

[1]Degenerate nucleotide residue designations:
D = A, G, or T;
H = A, C, or T;
N = A, C, G, or T;
R = A or G; and
Y = C or T.

### 6.1.2. NORTHERN BLOT ANALYSIS

3'- and 5'-HER4 specific [$\alpha^{32}P$]UTP-labeled antisense RNA probes were synthesized from the linearized plasmids pHt1B1.6 (containing an 800 bp HER4 fragment beginning at nucleotide 3098) and p5'H4E7 (containing a 1 kb fragment from the 5'-end of the HER4 sequence), respectively. For tissue distribution analysis (Section 6.2.2., *infra*), the Northern blot (Clontech, Palo Alto, CA) contained 2 $\mu$g poly(A)+ mRNA per lane from 8 human tissue samples immobilized on a nylon membrane. The filter was prehybridized at 60°C for several hours in RNA hybridization mixture (50% formamide, 5XSSC, 0.5% SDS, 10X Denhardt's solution, 100 $\mu$g/ml denatured herring sperm DNA, 100 $\mu$g/ml tRNA, and 10 $\mu$g/ml polyadenosine) and hybridized in the same buffer at 60°C, overnight with 1-1.5 x $10^6$ cpm/ml of $^{32}P$-labeled antisense RNA probe. The filters were washed in 0.1XSSC/0.1% SDS, 65°C, and exposed overnight on a phosphorimager (Molecular Dynamics, Sunnyvale, CA).

### 6.1.3. SEMI-OUANTITATIVE PCR DETECTION OF HER4

RNA was isolated from a variety of human cell lines, fresh frozen tissues, and primary tumors. Single stranded cDNA was synthesized from 10 $\mu$g of each RNA by priming with an oligonucleotide containing a $T_{17}$ track on its 3'-end (XSCT17:5'GACTCGAGTCGACATCGATTTTTTTTTTTTTTTTT-3') [SEQ ID NO: 28]. 1% or 5% of each single strand template preparation was then used in a 35 cycle PCR reaction with two HER4-specific oligonucleotides: 4H2674: 5'-GAAGAAAGACGACTCGTTCATCGG-3', [SEQ ID NO: 29], and 4H2965: 5'-GACCATGACCATGTAAACGTCAATA-3') [SEQ ID NO: 30]. Reaction products were electrophoresed on 2% agarose gels, stained with ethidium bromide and photographed on a UV light box. The relative intensity of the 291-bp HER4-specific bands were estimated for each sample as shown in Table II.

### 6.2.1. SEQUENCE ANALYSIS QF cDNA CLQNES ENCQDING HER4

cDNA clones encoding parts of the HER4 coding and non-coding nucleotide sequences were isolated by PCR cloning according to the method outlined in Section 6.1.1., *supra*. The complete HER4 nucleotide sequence assembled from these cDNAs is shown in FIG. 1 and contains a single open reading frame encoding a polypeptide of 1308 amino acids. The HER4 coding region is flanked by a 33 nucleotide 5'-untranslated region and a 1517 nucleotide 3'-untranslated region ending with a poly(A) tail. A 25 amino acid hydrophobic signal sequence follows a consensus initiating methionine at position number 1 in the amino acid sequence depicted in FIG.1. In relation to this signal sequence, the mature HER4 polypeptide would be predicted to begin at amino acid residue number 26 in the sequence depicted in FIG. 1 (Gln), followed by the next 1283 amino acids in the sequence. Thus the prototype mature HER4 of the invention is a polypeptide of 1284 amino acids, having a calculated Mr of 144,260 daltons and an amino acid sequence corresponding to residues 26 through 1309 in FIG. 1.

Comparison of the HER4 nucleotide and deduced amino acid sequences (FIG. 1) with the available DNA and protein sequence databases indicated that the HER4 nucleotide sequence is unique, and revealed a 60/64 amino acid identity with HER2 and a 54/54 amino acid identity to a fragment of a rat EGFR homolog, tyro-2.

### 6.2.2. SEQUENCE ANALYSIS QF RELATED cDNAs

Several cDNAs encoding polypeptides related to the prototype HER4 polypeptide (FIG. 1) were also isolated from the MDA-MB-453 cDNA library and comprised two forms.

The first alternative type of cDNA was identical to the consensus HER4 nucleotide sequence up to nucleotide 3168 (encoding Arg at amino acid position 1045 in the FIG. 1 sequence) and then abruptly diverges into an apparently unrelated sequence (FIG. 2A, FIG. 3A). Downstream from this residue the open reading frame continues for another 13 amino acids before reaching a stop codon followed by a 2 kb 3'-untranslated sequence and poly(A) tail. This cDNA would be predicted to result in a HER4 variant having the C-terminal autophosphorylation domain of the prototype HER4 deleted.

A second type of cDNA was isolated as 4 independent clones each with a 3'-sequence identical to the HER4 consensus, but then diverging on the 5'-side of nucleotide 2335 (encoding Glu at amino acid position 768 in the FIG. 1 sequence), continuing upstream for only another 114-154 nucleotides (FIG. 2B, FIG. 3B). Nucleotide 2335 is the precise location of an intron-exon junction in the HER2 gene (Coussens et al., 1985, Science 230; 1132-39; Semba et al., 1985, Proc. Natl. Acad. Sci. U.S.A. 82: 6497-6501), suggesting these cDNAs could be derived from mRNAs that have initiated from a cryptic promoter within the flanking intron. These 5'-truncated transcripts contain an open reading frame identical to that of the HER4 cDNA sequence of FIG. 1, beginning with the codon for Met at amino acid position 772 in FIG. 1. These cDNAs would be predicted to encode a cytoplasmic HER4 variant polypeptide that initiates just downstream from the ATP-binding domain of the HER4 kinase.

### 6.2.3. HUMAN TISSUE DISTRIBUTIQN QF HER4 EXPRESSIQN

Northern blots of poly(A)+ mRNA from human tissue samples were hybridized with antisense RNA probes to the 3'-end of HER4, encoding the autophosphorylation domain, as described in Section 6.1.2., *supra*. A HER4 mRNA transcript of approximately 6kb was identified, and was found to be most abundant in the heart and skeletal muscle (FIG. 6A). An mRNA of greater than approximately 15 kb was detected in the brain, with lower levels also detected in heart, skeletal muscle, kidney, and pancreas tissue samples.

The same blot was stripped and rehybridized with a probe from the 5'-end of HER4, within the extracellular domain coding region, using identical procedures. This hybridization confirmed the distribution of the 15 kb HER4 mRNA species, and detected a 6.5 kb mRNA species in heart, skeletal muscle, kidney, and pancreas tissue samples (FIG. 6B) with weaker signals in lung, liver, and placenta. In addition, minor transcripts of 1.7-2.6 kb were also detected in pancreas, lung, brain, and skeletal muscle tissue samples. The significance of the different sized RNA transcripts is not known.

Various human tissues were also examined for the presence of HER4 mRNA using the semi-quantitative PCR assay described in Section 6.1.3., *supra*. The results are shown in Table II, together with results of the assay on primary tumor samples and neoplastic cell lines (Section 6.2.4., immediately below). These results correlate well with the Northern and solution hybridization analysis results on the selected RNA samples. The highest levels of HER4 transcript expression were found in heart, kidney, and brain tissue samples. In addition, high levels of HER4 mRNA expression were found in parathyroid, cerebellum, pituitary, spleen, testis, and breast tissue samples. Lower expression levels were found in thymus, lung, salivary gland, and pancreas tissue samples, Finally, low or negative expression was observed in liver, prostate, ovary, adrenal, colon, duodenum, epidermis, and bone marrow samples.

### 6.2.4. HER4 mRNA EXPRESSION IN PRIMARY TUMORS AND VARIOUS CELL LINES OF NEOPLASTIC ORIGIN

HER4 mRNA expression profiles in several primary tumors and a number of cell lines of diverse neoplastic origin were determined with the semi-quantitative PCR assay (Section 6.1.3, *supra*) using primers from sequences in the HER4 kinase domain. The results are included in Table II. This analysis detected the highest expression of HER4 RNA in 4 human mammary adenocarcinoma cell lines (T-47D, MDA-MB-453, BT-474, and H3396), and in neuroblastoma (SK-N-MC), and pancreatic carcinoma (Hs766T) cell lines. Intermediate expression was detected in 3 additional mammary carcinoma cell lines (MCF-7, MDA-MB-330, MDA-MB-361). Low or undetectable expression was found in other cell lines derived from carcinomas of the breast (MDB-MB-231, MDA-MB-157, MDA-MB-468, SK-BR-3), kidney (Caki-1, Caki-2, G-401), liver (SK-HEP-1, HepG2), pancreas (PANC-1, AsPC-1, Capan-1), colon (HT-29), cervix (CaSki), vulva (A-41), ovary (PA-1, Caov-3), melanoma (SK-MEL-28), or in a variety of leukemic cell lines. Finally, high level expression was observed in Wilms (kidney) and breast carcinoma primary tumor samples.

**TABLE II**

**HER4 EXPRESSION BY PRC ANALYSIS**

| VERY STRONG | STRONG | MEDIUM |
|---|---|---|
| T47D (breast) | MDA-MB-453 (breast) | MCF-7 (breast) |
| | BT-474 (breast) | MDA-MB-330 (breast) |
| | H3396 (breast) | MDA-MB-157 (breast) |
| | Hs766T (pancreatic) | JEG-3 (choriocarcinoma) |
| | SK-N-MC (neural) | HEPM (palate) |
| | Wilms Tumor(kidney) | 458(medullablastoma) |
| | | Breast Carcinoma |
| Kidney | Brain | Skeletal Muscle |
| Heart | Cerebellum | Thymus |
| Parathyroid | Pituitary | Pancreas |
| | Breast | Lung |
| | Testis | Salivary Gland |
| | Spleen | |

| WEAK | NEGATIVE |
|---|---|
| MDB-MB-231 (breast) | MDA-MB-468 (breast) |
| MDA-MB-157 (breast) | G-401 (kidney) |
| SK-BR-3 (breast) | HepG2 (liver) |
| A-431 (vulva) | PANC-1 (pancreas) |
| Caki-1 (kidney) | AsPC-1(pancreas) |
| Caki-2 (kidney) | Capan-1 (pancreas) |
| SK-HEP-1 (liver) | HT-29 (colon) |
| THP-1 (macrophage) | CaSki (cervix) |
| | PA-1 (ovary) |
| Prostate | Caov-3 (ovary) |
| Adrenal | SK-MEL-28 (melanoma) |
| Ovary | HUF (fibroblast) |
| Colon | H2981 (lung) |
| Placenta | Ovarian tumor |
| | GEO (colon) |
| | ALL bone marrow |
| | AML bone marrow |
| | Duodenum |
| | Epidermis |
| | Liver |
| | Bone marrow stroma |

## 7. EXAMPLE: RECOMBINANT EXPRESSION OF HER4

### 7.1. MATERIALS AND METHODS

#### 7.1.1. CHO-KI CELLS AND CULTURE CONDITIONS

CHO-KI cells were obtained from the ATCC (Accession Number CCL 61). These cells lack any detectable EGFR, HER2, or HER3 by immunoblot, tyrosine phosphorylation, and $^{35}$S-labeled immunoprecipitation analysis. Transfected cell colonies expressing HER4 were selected in glutamine-free Glasgow modified Eagle's medium (GMEM-S, Gibco) supplemented with 10% dialyzed fetal bovine serum an increasing concentrations of methionine sulfoximine (Bebbington, 1991, in Methods: A Companion to Methods in Enzymology 2: 136-145 Academic Press).

### 7.1.2. EXPRESSION VECTOR CONSTRUCTION AND TRANSFECTIONS

The complete 4 kilobase coding sequence of prototype HER4 was reconstructed and inserted into a glutamine synthetase expression vector, pEE14, under the control of the cytomegalovirus immediate-early promoter (Bebbington, *supra*) to generate the HER4 expression vector pEEHER4. This construct (pEEHER4) was linearized with MluI and transfected into CHO-KI cells by calcium phosphate precipitation using standard techniques. Cells were placed on selective media consisting of GMEM-S supplemented with 10% dialyzed fetal bovine serum and methionine sulfoximine at an initial concentration of 25 $\mu$M (L-MSX) as described in Bebbington, *supra*, for the selection of initial resistant colonies. After 2 weeks, isolated colonies were transferred to 48-well plates and expanded for HER4 expression immunoassays as described immediately below. Subsequent rounds of selection using higher concentrations of MSX were used to isolate cell colonies tolerating the highest concentrations of MSX. A number of CHO/HER4 clones selected at various concentrations of MSX were isolated in this manner.

### 7.1.3. HER4 EXPRESSION IMMUNOASSAY

Confluent cell monolayers were scraped into hypotonic lysis buffer (10 mM Tris pH7.4, 1 mM KCl, 2 mM $MgCl_2$) at 4°C, dounce homogenized with 30 strokes, and the cell debris was removed by centrifugation at 3500 x g, 5 min. Membrane fractions were collected by centrifugation at 100,000 x g, 20 min, and the pellet was resuspended in hot Laemmli sample buffer with 2-mercaptoethanol. Expression of the HER4 polypeptide was detected by immunoblot analysis on solubilized cells or membrane preparations using HER2 immunoreagents generated to either a 19 amino acid region of the HER2 kinase domain, which coincidentally is identical to the HER4 sequence (residues 927-945), or to the C-terminal 14 residues of HER2, which share a stretch of 7 consecutive residues with a region near the C-terminus of HER4. On further amplification, HER4 was detected from solubilized cell extracts by immunoblot analysis with PY20 anti-phosphotyrosine antibody (ICN Biochemicals), presumably reflecting autoactivation and autophosphorylation of HER4 due to receptor aggregation resulting from abberantly high receptor density. More specifically, expression was detected by immunobloting with a primary murine monoclonal antibody to HER2 (Neu-Ab3, Oncogene Science) diluted 1:50 in blotto (2.5% dry milk, 0.2% NP40 in PBS) using $^{125}$I-goat anti-mouse Ig F(ab')2 (Amersham, UK) diluted 1:500 in blotto as a second antibody. Alternatively, a sheep polyclonal antipeptide antibody against HER2 residues 929-947 (Cambridge Research Biochemicals, Valleystream, NY) was used as a primary immunoreagent diluted 1:100 in blotto with $^{125}$I-Protein G (Amersham) diluted 1:200 in blotto as a second antibody. Filters were washed with blotto and exposed overnight on a phosphorimager (Molecular Dynamics).

### 7.2. RESULTS

CHO-KI cells transfected with a vector encoding the complete human prototype HER4 polypeptide were selected for amplified expression in media containing increasing concentrations of methionine sulfoximine as outlined in Section 7.1., et seq., *supra*. Expression of HER4 was evaluated using the immunoassay described in Section 7.1.3., *supra*. Several transfected CHO-KI cell clones stably expressing HER4 were isolated. One particular clone, CHO/HER4 21-2, was selected in media supplemented with 250 $\mu$M MSX, and expresses high levels of HER4. CHO/HER4 21-2 cells have been deposited with the ATCC.

Recombinant HER4 expressed in CHO/HER4 cells migrated with an apparent Mr of 180,000, slightly less than HER2, whereas the parental CHO cells showed no cross-reactive bands (FIG. 7A). In addition, a 130 kDa band was also detected in the CHO/HER4 cells, and presumably represents a degradation product of the 180 kDa mature protein. CHO/HER4 cells were used to identify ligand specific binding and autophosphorylation of the HER4 tyrosine kinase (see Section 9., et seq., *infra*).

## 8. EXAMPLE: ASSAY FOR DETECTING EGFR-FAMILY LIGANDS

### 8.1. CELL LINES

A panel of four recombinant cell lines, each expressing a single member of the human EGFR-family, were generated for use in the tyrosine kinase stimulatory assay described in Section 8.2., below. The cell line CHO/HER4 3 was generated as described in Section 7.1.2, *supra*.

CHO/HER2 cells (clone 1-2500) were selected to express high levels of recombinant human $p185^{erbB2}$ by dihydrofolate reductase-induced gene amplification in dhfr-deficient CHO cells. The HER2 expression plasmid, cDNeu, was generated by insertion of a full length HER2 coding sequence into a modified pCDM8 (Invitrogen, San Diego, CA) expression vector (Seed and Aruffo, 1987, Proc. Natl. Adad. Sci. U.S.A. 84: 3365-69) in which an expression cassette from pSV2DHFR (containing the murine dhfr cDNA driven by the SV40 early promoter) has been inserted at the pCDM8 vector's unique BamHI site. This construct drives HER2 expression from the CMV immediate-early promoter.

NRHER5 cells (Velu et al., 1987, Science 1408-10) were obtained from Dr. Hsing-Jien Kung (Case Western Reserve University, Cleveland, OH). This murine cell line was clonally isolated from NR6 cells infected with a retrovirus stock carrying the human EGFR, and was found to have approximately $10^6$ human EGFRs per cell.

The cell line 293/HER3 was selected for high level expression of $p160^{erbB3}$. The parental cell line, 293 human embryonic kidney cells, constitutively expresses adenovirus E1a and have low levels of EGFR expression. This line was established by cotransfection of linearized cHER3 (Plowman et al., 1990, Proc. Natl. Acad. Sci. U.S.A. 87: 4905-09) and pMC1neoPolyA (neomycin selectable marker with an Herpes simplex thymidine kinase promoter, Stratagene), with selection in DMEM/F12 media containing 500µg/ml G418.

### 8.2. TYROSINE KINASE STIMULATION ASSAY

Cells were plated in 6-well tissue culture plates (Falcon), and allowed to attach at 37°C for 18-24 hr. Prior to the assay, the cells were changed to serum-free media for at least 1 hour. Cell monolayers were then incubated with the amounts of ligand preparations indicated in Section 7.3., below for 5 min at 37°C. Cells were then washed with PBS and solubilized on ice with 0.5 ml PBSTDS containing phosphatase inhibitors (10 mM NaHPO4, 7.25, 150 mM NaCl, 1% Triton X-100, 0.5% deoxycholate, 0.1% SDS, 0.2% sodium azide, 1 mM NaF, 1 mM EGTA, 4 mM sodium orthovanadate, 1% aprotinin, 5 µg/ml leupeptin). Cell debris was removed by centrifugation (12000 x g, 15 min, 4°C) and the cleared supernatant reacted with 1 µg murine monoclonal antibody to phosphotyrosine (PY20, ICN Biochemicals, Cleveland, Ohio) for CHO/HER4 and 293/HER3 cells, or 1 µg murine monoclonal antibody to HER2 (Neu-Ab3, Oncogene Sciences) for CHO/HER2 cells, or 1 µg murine monoclonal antibody EGFR-1 to human EGFR (Amersham) for NRHER5 cells. Following a 1 hr incubation at 4°C, 30 µl of a 1:1 slurry (in PBSTDS) of anti-mouse IgG-agarose (for PY20 and Neu-Ab3 antibodies) or protein A-sepharose (for EGFR-R1 antibody) was added and the incubation was allowed to continue an additional 30 minutes. The beads were washed 3 times in PBSTDS and the complexes resolved by electrophoresis on reducing 7% SDS-polyacrylamide gels. The gels were transferred to nitrocellulose and blocked in TNET (10 mM Tris pH7.4, 75 mM NaCl, 0.1% Tween-20, 1 mM EDTA). PY20 antiphosphotyrosine antibody diluted 1:1000 in TNET was used as the primary antibody followed by $^{125}$I-goat anti-mouse Ig F(ab')2 diluted 1:500 in TNET. Blots were washed with TNET and exposed on a phosphorimager (Molecular Dynamics).

### 8.3. RESULTS

Several EGF-family member polypeptide and ligand preparations were tested for their ability to stimulate tyrosine phosphorylation of each of four EGFR-family receptors expressed in recombinant CHO cells using the tyrosine phosphorylation stimulation assay described in Section 8.2., above. The particular preparations tested for each of the four recombinant cell lines and the results obtained in the assay are tabulated below, and autoradiographs of some of these results are shown in FIG. 8.

TABLE III

| STIMULATION OF TYR PHOSPHORYLATION OF EGFR-FAMILY RECEPTORS | | | | |
|---|---|---|---|---|
| PREPARATION | RECOMBINANT CELLS | | | |
| | CHO/HER4#3 | CHO/HER2 | NRHER5 | 293/HER3 |
| EGF | - | - | + | - |
| AMPHIREGULIN | - | - | + | - |
| TGF-α | - | - | + | - |
| HB-EGF | - | - | + | - |
| FRACTION 17* | + | - | - | - |
| FRACTION 14* | - | - | - | - |

* The identification of the HER4 tyrosine kinase stimulatory activity within the conditioned media of HepG2 cells and the isolation of these preparations is described in Section 9, *infra*.

The results indicate that EGF, AR, TGF-α, and HB-EGF, four related ligands which mediate their growth regulatory signals in part through interaction with EGFR, were able to stimulate tyrosine phosphorylation of EGFR expressed in recombinant NIH3T3 cells (for EGF, see FIG. 8C, lane 2), but not HER4, HER2, or HER3 expressed in recombinant CHO or 293 cells (FIG. 8A, B, D, lanes 2 and 3). Additionally, as discussed in more detail below, the assay identified a HepG2-derived preparation (fraction 17) as a HER4 ligand capable of specifically stimulating tyrosine phoshorylation of HER4 expressed in CHO/HER4 cells alone.

## 9. EXAMPLE: ISOLATION OF A HER4 LIGAND

### 9.1. MATERIALS AND METHODS

#### 9.1.1. CELL DIFFERENTIATION ASSAY

For the identification of ligands specific for HER2, HER3 or HER4, the receptor expression profile of MDA-MB-453 cells offers an excellent indicator for morphologic differentiation inducing activity. This cell line is known to express HER2 and HER3, but contains no detectable EGFR. The results of the semi-quantitative PCR assays (Table III) indicated high level expression of HER4 in MDA-MB-453 cells. In addition, cDNA encoding the prototype HER4 polypeptide of the invention was first isolated from this cell line (Section 6., *supra*).

MDA-MB-453 cells (7500/well) were grown in 50 ml DMEM supplemented with 5% FBS and 1x essential amino acids. Cells were allowed to adhere to 96-well plates for 24 hr. Samples were diluted in the above medium, added to the cell monolayer in 50 ml final volume, and the incubation continued for an additional 3 days. Cells were then examined by inverted light microscopy for morphologic changes.

#### 9.1.2. SOURCE CELLS

Serum free media from a panel of cultures human cancer cells were screened for growth regulatory activity on MDA-MB-453 cells. A human hepatocarcinoma cell line, HepG2, was identified as a source of a factor which induced dramatic morphologic differentiation of the MDA-MB-453 cells.

#### 9.1.3. PURIFICATION OF HER4 LIGAND

The cell differentiation assay described in Section 10.1.1., *supra*, was used throughout the purification procedure to monitor the column fractions that induce morphological changes in MDA-MB-453 cells. For large-scale production of conditioned medium, HepG2 cells were cultured in DMEM containing 10% fetal bovine serum using Nunc cell factories. At about 70% confluence, cells were washed then incubated with serum-free DMEM. Conditioned medium (HepG2-CM) was collected 3 days later, and fresh serum-free medium added to the cells. Two additional harvests of HepG2-CM were collected per cell factory. The medium was centrifuged and stored at -20°C in the presence of 500 mM PMSF.

Ten litres of HepG2-CM were concentrated 16-fold using an Amicon ultrafiltration unit (10,000 molecular weight cutoff membrane), and subjected to sequential precipitation with 20% and 60% ammonium sulfate. After centrifugation at 15,000 x g, the supernatant was extensively dialyzed against PBS and passed through a DEAE-sepharose (Pharmacia) column pre-equilibrated with PBS. The flow-through fraction was then applied onto a 4 ml heparin-acrylic (Bio-Rad) column equilibrated with PBS. Differentiation inducing activity eluted from the heparin column between 0.4 and 0.8 M NaCl. Active heparin fractions were pooled, brought to 2.0 M ammonium sulfate, centrifuged at 12,000 x g for 5 min, and the resulting supernatant was loaded onto a phenyl-5PW column (8 x 75 mm, Waters). Bound proteins were eluted with a decreasing gradient from 2.0 M ammonium sulfate in 0.1 M Na2HPO4, pH 7.4 to 0.1 M Na2HPO4. Dialyzed fractions were assayed for tyrosine phosphorylation of MDA-MB-453 cells, essentially as described (Wen et al., 1992, Cell 69: 559-72), except PY20 was used as the primary antibody and horseradish peroxidase-conjugated goat F(ab')2 anti-mouse Ig (Cappell) and chemiluminescence were used for detection. Phosphorylation signals were analyzed using the Molecular Dynamics personal densitometer.

## 9.2. RESULTS

Semi-purified HepG2-derived factor demonstrated a capacity to induce differentiation in MDA-MB-453 cells (FIG. 9). With reference to the micrographs shown in FIG. 9, untreated MDA-MB-453 cells are moderately adherent and show a rounded morphology (FIG. 9A). In contrast, the addition of semi-purified HepG2-derived factor induces these cells to display a noticeably flattened morphology with larger nuclei and increased cytoplasm (FIG. 9B and 9C). This HepG2-derived factor preparation also binds to heparin, a property which was utilized for purifying the activity.

On further purification, the HepG2-derived factor was found to elute from a phenyl hydrophobic interaction column at 1.0M ammonium sulfate (fractions 16 to 18). FIG. 9D shows the phenyl column elution profile. Tyrosine phosphorylation assays of the phenyl column fractions revealed that the same fractions found to induce differentiation of the human breast carcinoma cells are also able to stimulate tyrosine phosphorylation of a 185 K protein in MDA-MB-453 cells (FIG. 9E). In particular, fraction 16 induced a 4.5-fold increase in the phosphorylation signal compared to the baseline signal observed in unstimulated cells, as determined by densitometry analysis (FIG. 9F).

The phenyl fractions were also tested against the panel of cell lines which each overexpress a single member of the EGFR-family (Section 9.1., *supra*). Fraction 17 induced a significant and specific activation of the HER4 kinase ( FIG. 8A, lane 4) without directly affecting the phosphorylation of HER2, EGFR, or HER3 (FIGS. 8B, 8C, and 8D, lane 4). Adjacent fraction 14 was used as a control and had no effect on the phosphorylation of any of the EGFR-family receptors (FIGS. 8A, B, C, D, lane 5). Further purification and analysis of the factor present in fraction 17 indicates that it is a glycoprotein of 40 to 45 kDa, approximately the same size as NDF and HRG. The HepG2-derived factor also has functional properties similar to NDF and HRG, inasmuch as it stimulates tyrosine phosphorylation of HER2/p185 in MDA-MB-453 cells, but not EGFR in NR5 cells, and induces morphologic differentiation of HER2 overexpressing human breast cancer cells.

Recently, several groups have reported the identification of specific ligands for HER2 (see Section 2., *supra*., including NDF and HRG-$\alpha$. In contrast to these molecules, the HepG2-derived factor described herein failed to stimulate phosphorylation of HER2 in CHO/HER2 cells, but did stimulate phosphorylation of HER4 in CHO/HER4 cells. These findings are intriguing in view of the ability of the HepG2-derived factor to stimulate phosphorylation of MDA-MD-453 cells, a cell line known to overexpress HER2 and HER3 and the source from which HER4 was cloned. Since EGFR and HER2 have been shown to act synergistically, it is conceivable that HER4 may also interact with other EGFR-family members. In this connection, these results suggest that NDF may bind to HER4 in MDA-MB-453 cells resulting in the activation of HER2. The results described in Section 10., immediately below, provide evidence that NDF interacts directly with HER4, resulting in activation of HER2.

## 10. EXAMPLE: RECOMBINANT NDF-INDUCED, HER4 MEDIATED PHOSPHORYLATION OF HER2

Recombinant NDF was expressed in COS cells and tested for its activity on HER4 in an assay system essentially devoid of other known members of the EGFR-family, notably EGFR and HER2.

A full length rat NDF cDNA was isolated from normal rat kidney RNA and inserted into a cDM8-based expression vector to generate cNDF1.6. This construct was transiently expressed in COS cells, and conditioned cell supernatants were tested for NDF activity using the tyrosine kinase stimulation assay described in Section 8.2., *supra*. Supernatants from cNDF1.6 transfected cells upregulated tyrosine phosphorylation in MDA-MB-453 cells relative to mock transfected COS media FIG. 10A. Phosphorylation peaked 10-15 minutes after addition on NDF.

The crude NDF supernatants were also tested for the ability to phosphorylate EGFR (NR5 cells), HER2 (CHO/HER2 1-2500 cells), and HER4 (CHO/HER4 21-2 cells). The NDF preparation had no effect on phosphorylation of EGFR, or HER2 containing cells, but induced a 2.4 to 4 fold increase in tyrosine phosphorylation of HER4 after 15 minutes incubation (see FIG. 10B) . These findings provide preliminary evidence that NDF/HRG-$\alpha$ mediate their effects not through direct binding to HER2, but instead by means of a direct interaction with HER4. In cell lines expressing both HER2 and HER4, such as MDA-MB-453 cells and other breast carcinoma cells, binding of NDF to HER4 may stimulate HER2 either by heterodimer formation of these two related transmembrane receptors, or by intracellular crosstalk. Formal proof of the direct interaction between NDF and HER4 will require crosslinking of $^{125}$I-NDF to CHO/HER4 cells and a detailed analysis of its binding characteristics.

## 11. EXAMPLE: CHROMOSOMAL MAPPING OF THE HER4 GENE

A HER4 cDNA probe corresponding to the 5' portion of the gene (nucleotide positions 34-1303) was used for *in situ* hybridization mapping of the HER4 gene. *In situ* hybridization to metaphase chromosomes from lymphocytes of two normal male donors was conducted using the HER4 probe labeled with $^{3}$H to a specific activity of 2.6 x $10^7$ cpm/$\mu$g as described (Marth et al, 1986, Proc. Natl. Acad. Sci. U.S.A. 83:7400-04). The final probe concentration was 0.05 $\mu$g/$\mu$l of hybridization mixture. Slides were exposed for one month. Chromosomes were identified by Q banding.

### 11.2 RESULTS

A total of 58 metaphase cells with autoradiographic grains were examined. Of the 124 hybridization sites scored, 38 (31%) were located on the distal portion of the long arm of chromosome 2 (FIG. 11). The greatest number of grains (21 grains) was located at band q33, with significant numbers of grains on bands q34 (10 grains) and q35 (7 grains). No significant hybridization on other human chromosomes was detected.

## 12. MICROORGANISM AND CELL DEPOSITS

The following microorganisms and cell lines have been deposited with the American Type Culture Collection, and have been assigned the following accession numbers:

| Microorganism | Plasmid | Accession Number |
|---|---|---|
| *Escherichia coli* SCS-1 | pBSHER4Y | 69 131 |
| (containing the complete human HER4 coding sequence) | | |

| Cell Lines | Accession Number |
|---|---|
| CHO/HER4 21-2 | CRL 11205 |

The present invention is not to be limited in scope by the microorganisms and cell lines deposited or the embodiments disclosed herein, which are intended as single illustrations of one aspect of the invention, and any which are functionally equivalent are within the scope of the invention. Indeed, various modifications of the invention, in addition to those shown and described herein, will become apparent to those skilled in the art from the foregoing description. Such modifications are intended to fall within the scope of the appended claims. All base pair and amino acid residue numbers and sizes given for polynucleotides and polypeptides are approximate and used for the purpose of description.

## Claims

1. A recombinant polynucleotide comprising a sequence of at least about 200 nucleotides having greater than 80 % homology to a contiguous portion of the HER4 nucleotide sequence depicted in FIG. 1 or its complement.

2. The recombinant polynucleotide of claim 1 comprising a sequence of nucleotides encoding at least about 70 contiguous amino acids within the HER4 amino acid sequence depicted in FIG. 1.

3. The recombinant polynucleotide of claim 1 comprising a contiguous sequence of at least about 200 nucleotides within the HER4 nucleotide coding sequence depicted in FIG. 1 or its complement.

4. The recombinant polynucleotide of claim 1 comprising the HER4 nucleotide coding sequence depicted in FIG. 1 or its complement.

5. A recombinant polynucleotide which encodes a polypeptide having structural characteristics equivalent to that of HER4, which polynucleotide is obtained by single or multiple base addition, deletion and/or substitution in a nucleotide sequence of one of the claims 1 to 4, or which is obtained by selective hybridization with a nucleotide sequence of one of the claims 1 to 4.

6. A recombinant polynucleotide according to one of the claims 1 to 5 which is a DNA polynucleotide.

7. A recombinant polynucleotide according to one of the claims 1 to 5 which is a RNA polynucleotide.

**8.** An assay kit comprising a recombinant polynucleotide according to one of the claims 1 to 5 to which a detectable label has been added.

**9.** A polymerase chain reaction (PCR) kit comprising a pair of primers capable of priming cDNA synthesis in a PCR reaction, wherein each primer is a polynucleotide according to claim 6.

**10.** The PCR kit according to claim 9 further comprising a polynucleotide probe capable of hybridizing to a region of the HER4 gene between and not including the nucleotide sequences to which the primers hybridize.

**11.** A polypeptide comprising a sequence of at least about 80 amino acids having greater than 90 % identity to a contiguous portion of the HER4 amino acid sequence depicted in FIG. 1.

**12.** A HER4 polypeptide comprising
- the amino acid sequence depicted in FIG. 1 from amino acid residues 1 through 1308, or
- the amino acid sequence depicted in FIG. 1 from amino acid residues 26 through 1308; or
- the amino acid sequence depicted in FIG. 1 from amino acid residues 1 through 1045; or
- the amino acid sequence depicted in FIG. 1 from amino acid residues 26 through 1045; or
- the amino acid sequence depicted in FIG. 2A, or
- the amino acid sequence depicted in FIG. 1 from amino acid residues 772 through 1308; or
- the amino acid sequence depicted in FIG. 2B.

**13.** A polypeptide having structural and/or functional features equivalent to HER4, obtainable by single or multiple amino acid addition, deletion and/or substitution in a sequence of one of the claims 11 or 12.

**14.** An antibody capable of inhibiting the interaction of a soluble polypeptide and human HER4.

**15.** An antibody according to claim 14 wherein the soluble polypeptide is a heregulin.

**16.** An antibody capable of
a) stimulating HER4 tyrosine autophosphorylation; or
b) inducing a HER4-mediated signal in a cell, which signal results in modulation of growth and/or differentiation of the cell; or
c) inhibiting HepG2 fraction 17-stimulated tyrosine phosphorylation of HER4 expressed in CHO/HER4 21-2 cells as deposited with the ATCC (accession number CRL 11205).

**17.** An antibody which immunospecifically binds to human HER4.

**18.** An antibody according to claim 17 which
a) resides on the cell surface after binding to HER4; or
b) is internalized into the cell after binding to HER4; or
c) immunospecifically binds to human HER4 expressed in CHO/HER4 21-2 cells as deposited with the ATCC (accession number CRL 11205); or
d) neutralizes HER4 biological activity; or
e) is conjugated to a drug or toxin; or
f) is radiolabeled.

**19.** Plasmid pBSHER4Y as deposited with the ATCC and having the accession number ATCC 69131.

**20.** A recombinant vector comprising a nucleotide sequence encoding a polypeptide according to one of the claims 11 to 13.

**21.** A host cell transfected with a recombinant vector according to claim 20.

**22.** A recombinant vector comprising a nucleotide sequence encoding a polypeptide according to one of the claims 11 to 13 wherein the coding sequence is operably linked to a control sequence which is capable of directing the expression of the coding sequence in a host cell transfected therewith.

**23.** A host cell transfected with a recombinant vector according to claim 22.

**24.** Cell line CHO/HER4 21-2 as deposited with the ATCC and having the accession number CRL 11205.

**25.** An assay for detecting the presence of a HER4 ligand in a sample comprising:
(a) applying the sample to cells which have been engineered to overexpress HER4; and
(b) detecting an ability of the ligand to affect an activity mediated by HER4.

**26.** The method according to claim 25, wherein the cells are CHO/HER4 21-2 cells as deposited with the ATCC and having the accession number CRL 11205.

**27.** The method according to claim 25, wherein the activity detected is HER4 tyrosine phosphorylation, or morphologic differentiation.

**28.** A ligand for HER4 comprising a polypeptide which binds to HER4, stimulates tyrosine phosphorlation of HER4, and affects a biological activity mediated by HER4.

**29.** A ligand according to claim 28 which is capable of inducing morphological differentiation when added to cultured MDA-MB-453 cells; and/or which is obtained from cultured HepG2 cell conditioned media.

**30.** An immunoassay for detecting HER4 comprising:
a) providing an antibody according to claim 17 or 18;
b) incubating a biological sample with the antibody under conditions which allow for the binding of the antibody to HER4; and
c) determining the amount of antibody present as a HER4-antibody complex.

**31.** The use of at least one antibody according to one of the claims 17 or 18 for preparing a pharmaceutical composition for the in vivo delivery of a drug or toxin to cells expressing HER4.

**32.** The use of claim 31, which comprises conjugating at least one antibody according to claim 17 or 18, or an active fragment thereof, to the drug or toxin, for delivering the resulting conjugate to an individual by using a formulation, dose, and route of administration such that the conjugate binds to HER4.

HER4 cDNA

```
  1                                                MetLysProAlaThrGlyLeuTrpValTrp ValSerLeuLeuValAlaAlaGlyThr
  1     AATTGTCAGCACGGGATCTGAGACTTCCAAAAA ATGAAGCCGGCGACAGGACTTTGGGTCTGG GTGAGCCTTCTCGTGGCGGCGGGGACC

 20 Val GlnProSerAspSerGlnSerValCysAla GlyThrGluAsnLysLeuSerSerLeuSer AspLeuGluGlnGlnTyrArgAlaLeu
 91 GTC CAGCCCAGCGATTCTCAGTCAGTGTGTGCA GGAACGGAGAATAAACTGAGCTCTCTCTCT GACCTGGAACAGCAGTACCGAGCCTTG

 50 Arg LysTyrTyrGluAsnCysGluValValMet GlyAsnLeuGluIleThrSerIleGluHis AsnArgAspLeuSerPheLeuArgSer
181 CGC AAGTACTATGAAAACTGTGAGGTTGTCATG GGCAACCTGGAGATAACCAGCATTGAGCAC AACCGGGACCTCTCCTTCCTGCGGTCT

 80 Val ArgGluValThrGlyTyrValLeuValAla LeuAsnGlnPheArgTyrLeuProLeuGlu AsnLeuArgIleIleArgGlyThrLys
271 GTT CGAGAAGTCACAGGCTACGTGTTAGTGGCT CTTAATCAGTTTCGTTACCTGCCTCTGGAG AATTTACGCATTATTCGTGGGACAAAA

110 Leu TyrGluAspArgTyrAlaLeuAlaIlePhe LeuAsnTyrArgLysAspGlyAsnPheGly LeuGlnGluLeuGlyLeuLysAsnLeu
361 CTT TATGAGGATCGATATGCCTTGGCAATATTT TTAAACTACAGAAAAGATGGAAACTTTGGA CTTCAAGAACTTGGATTAAAGAACTTG

140 Thr GluIleLeuAsnGlyGlyValTyrValAsp GlnAsnLysPheLeuCysTyrAlaAspThr IleHisTrpGlnAspIleValArgAsn
451 ACA GAAATCCTAAATGGTGGAGTCTATGTAGAC CAGAACAAATTCCTTTGTTATGCAGACACC ATTCATTGGCAAGATATTGTTCGGAAC

170 Pro TrpProSerAsnLeuThrLeuValSerThr AsnGlySerSerGlyCysGlyArgCysHis LysSerCysThrGlyArgCysTrpGly
541 CCA TGGCCTTCCAACTTGACTCTTGTGTCAACA AATGGTAGTTCAGGATGTGGACGTTGCCAT AAGTCCTGTACTGGCCGTTGCTGGGGA

200 Pro ThrGluAsnHisCysGlnThrLeuThrArg ThrValCysAlaGluGlnCysAspGlyArg CysTyrGlyProTyrValSerAspCys
631 CCC ACAGAAAATCATTGCCAGACTTTGACAAGG ACGGTGTGTGCAGAACAATGTGACGGCAGA TGCTACGGACCTTACGTCAGTGACTGC

230 Cys HisArgGluCysAlaGlyGlyCysSerGly ProLysAspThrAspCysPheAlaCysMet AsnPheAsnAspSerGlyAlaCysVal
721 TGC CATCGAGAATGTGCTGGAGGCTGCTCAGGA CCTAAGGACACAGACTGCTTTGCCTGCATG AATTTCAATGACAGTGGAGCATGTGTT

260 Thr GlnCysProGlnThrPheValTyrAsnPro ThrThrPheGlnLeuGluHisAsnPheAsn AlaLysTyrThrTyrGlyAlaPheCys
811 ACT CAGTGTCCCCAAACCTTTGTCTACAATCCA ACCACCTTTCAACTGGAGCACAATTTCAAT GCAAAGTACACATATGGAGCATTCTGT

290 Val LysLysCysProHisAsnPheValValAsp SerSerSerCysValArgAlaCysProSer SerLysMetGluValGluGluAsnGly
901 GTC AAGAAATGTCCACATAACTTTGTGGTAGAT TCCAGTTCTTGTGTGCGTGCCTGCCCTAGT TCCAAGATGGAAGTAGAAGAAAATGGG

320 Ile LysMetCysLysProCysThrAspIleCys ProLysAlaCysAspGlyIleGlyThrGly SerLeuMetSerAlaGlnThrValAsp
991 ATT AAAATGTGTAAACCTTGCACTGACATTTGC CCAAAAGCTTGTGATGGCATTGGCACAGGA TCATTGATGTCAGCTCAGACTGTGGAT
```

Figure 1

```
 350  Ser SerAsnIleAspLysPheIleAsnCysThr LysIleAsnGlyAsnLeuIlePheLeuVal ThrGlyIleHisGlyAspProTyrAsn
1081  TCC AGTAACATTGACAAATTCATAAACTGTACC AAGATCAATGGGAATTTGATCTTTCTAGTC ACTGGTATTCATGGGGACCCTTACAAT

 380  Ala IleGluAlaIleAspProGluLysLeuAsn ValPheArgThrValArgGluIleThrGly PheLeuAsnIleGlnSerTrpProPro
1171  GCA ATTGAAGCCATAGACCCAGAGAAACTGAAC GTCTTTCGGACAGTCAGAGAGATAACAGGT TTCCTGAACATACAGTCATGGCCACCA

 410  Asn MetThrAspPheSerValPheSerAsnLeu ValThrIleGlyGlyArgValLeuTyrSer GlyLeuSerLeuLeuIleLeuLysGln
1261  AAC ATGACTGACTTCAGTGTTTTTCTAACCTG GTGACCATTGGTGGAAGAGTACTCTATAGT GGCCTGTCCTTGCTTATCCTCAAGCAA

 440  Gln GlyIleThrSerLeuGlnPheGlnSerLeu LysGluIleSerAlaGlyAsnIleTyrIle ThrAspAsnSerAsnLeuCysTyrTyr
1351  CAG GGCATCACCTCTCTACAGTTCCAGTCCCTG AAGGAAATCAGCGCAGGAAACATCTATATT ACTGACAACAGCAACCTGTGTTATTAT

 470  His ThrIleAsnTrpThrThrLeuPheSerThr IleAsnGlnArgIleValIleArgAspAsn ArgLysAlaGluAsnCysThrAlaGlu
1441  CAT ACCATTAACTGGACAACACTCTTCAGCACA ATCAACCAGAGAATAGTAATCCGGGACAAC AGAAAAGCTGAAAATTGTACTGCTGAA

 500  Gly MetValCysAsnHisLeuCysSerSerAsp GlyCysTrpGlyProGlyProAspGlnCys LeuSerCysArgArgPheSerArgGly
1531  GGA ATGGTGTGCAACCATCTGTGTTCCAGTGAT GGCTGTTGGGGACCTGGGCCAGACCAATGT CTGTCGTGTCGCCGCTTCAGTAGAGGA

 530  Arg IleCysIleGluSerCysAsnLeuTyrAsp GlyGluPheArgGluPheGluAsnGlySer IleCysValGluCysAspProGlnCys
1621  AGG ATCTGCATAGAGTCTTGTAACCTCTATGAT GGTGAATTTCGGGAGTTTGAGAATGGCTCC ATCTGTGTGGAGTGTGACCCCCAGTGT

 560  Glu LysMetGluAspGlyLeuLeuThrCysHis GlyProGlyProAspAsnCysThrLysCys SerHisPheLysAspGlyProAsnCys
1711  GAG AAGATGGAAGATGGCCTCCTCACATGCCAT GGACCGGGTCCTGACAACTGTACAAAGTGC TCTCATTTTAAAGATGGCCCAAACTGT

 590  Val GluLysCysProAspGlyLeuGlnGlyAla AsnSerPheIlePheLysTyrAlaAspPro AspArgGluCysHisProCysHisPro
1801  GTG GAAAAATGTCCAGATGGCTTACAGGGGGCA AACAGTTTCATTTTCAAGTATGCTGATCCA GATCGGGAGTGCCACCCATGCCATCCA

 620  Asn CysThrGlnGlyCysAsnGlyProThrSer HisAspCysIleTyrTyrProTrpThrGly HisSerThrLeuProGlnHisAlaArg
1891  AAC TGCACCCAAGGGTGTAACGGTCCCACTAGT CATGACTGCATTTACTACCCATGGACGGGC CATTCCACTTTACCACAACATGCTAGA

 650  Thr ProLeuIleAlaAlaGlyValIleGlyGly LeuPheIleLeuValIleValGlyLeuThr PheAlaValTyrValArgArgLysSer
1981  ACT CCCCTGATTGCAGCTGGAGTAATTGGTGGG CTCTTCATTCTGGTCATTGTGGGTCTGACA TTTGCTGTTTATGTTAGAAGGAAGAGC

 680  Ile LysLysLysArgAlaLeuArgArgPheLeu GluThrGluLeuValGluProLeuThrPro SerGlyThrAlaProAsnGlnAlaGln
2071  ATC AAAAAGAAAAGAGCCTTGAGAAGATTCTTG GAAACAGAGTTGGTGGAACCATTAACTCCC AGTGGCACAGCACCCAATCAAGCTCAA
```

Figure 1

(continued)

```
 710  Leu RgIleLeuLysGluThrGluLeuLysArg ValLysValLeuGlySerGlyAlaPheGly ThrValTyrLysGlyIleTrpValPro
2161  CTT CGTATTTTGAAAGAAACTGAGCTGAAGAGG GTAAAAGTCCTTGGCTCAGGTGCTTTTGGA ACGGTTTATAAAGGTATTTGGGTACCT

 740  Glu GlyGluThrValLysIleProValAlaIle LysIleLeuAsnGluThrThrGlyProLys AlaAsnValGluPheMetAspGluAla
2251  GAA GGAGAAACTGTGAAGATTCCTGTGGCTATT AAGATTCTTAATGAGACAACTGGTCCCAAG GCAAATGTGGAGTTCATGGATGAAGCT

 770  Leu IleMetAlaSerMetAspHisProHisLeu ValArgLeuLeuGlyValCysLeuSerPro ThrIleGlnLeuValThrGlnLeuMet
2341  CTG ATCATGGCAAGTATGGATCATCCACACCTA GTCCGGTTGCTGGGTGTGTGTCTGAGCCCA ACCATCCAGCTGGTTACTCAACTTATG

 800  Pro HisGlyCysLeuLeuGluTyrValHisGlu HisLysAspAsnIleGlySerGlnLeuLeu LeuAsnTrpCysValGlnIleAlaLys
2431  CCC CATGGCTGCCTGTTGGAGTATGTCCACGAG CACAAGGATAACATTGGATCACAACTGCTG CTTAACTGGTGTGTCCAGATAGCTAAG

 830  Gly MetMetTyrLeuGluGluArgArgLeuVal HisArgAspLeuAlaAlaArgAsnValLeu ValLysSerProAsnHisValLysIle
2521  GGA ATGATGTACCTGGAAGAAAGACGACTCGTT CATCGGGATTTGGCAGCCCGTAATGTCTTA GTGAAATCTCCAAACCATGTGAAAATC

 860  Thr AspPheGlyLeuAlaArgLeuLeuGluGly AspGluLysGluTyrAsnAlaAspGlyGly LysMetProIleLysTrpMetAlaLeu
2611  ACA GATTTTGGGCTAGCCAGACTCTTGGAAGGA GATGAAAAAGAGTACAATGCTGATGGAGGA AAGATGCCAATTAAATGGATGGCTCTG

 890  Glu CysIleHisTyrArgLysPheThrHisGln SerAspValTrpSerTyrGlyValThrIle TrpGluLeuMetThrPheGlyGlyLys
2701  GAG TGTATACATTACAGGAAATTCACCCATCAG AGTGACGTTTGGAGCTATGGAGTTACTATA TGGGAACTGATGACCTTTGGAGGAAAA

 920  Pro TyrAspGlyIleProThrArgGluIlePro AspLeuLeuGluLysGlyGluArgLeuPro GlnProProIleCysThrIleAspVal
2791  CCC TATGATGGAATTCCAACGCGAGAAATCCCT GATTTATTAGAGAAAGGAGAACGTTTGCCT CAGCCTCCCATCTGCACTATTGACGTT

 950  Tyr MetValMetValLysCysTrpMetIleAsp AlaAspSerArgProLysPheLysGluLeu AlaAlaGluPheSerArgMetAlaArg
2881  TAC ATGGTCATGGTCAAATGTTGGATGATTGAT GCTGACAGTAGACCTAAATTTAAGGAACTG GCTGCTGAGTTTTCAAGGATGGCTCGA

 980  Asp ProGlnArgTyrLeuValIleGlnGlyAsp AspArgMetLysLeuProSerProAsnAsp SerLysPhePheGlnAsnLeuLeuAsp
2971  GAC CCTCAAAGATACCTAGTTATTCAGGGTGAT GATCGTATGAAGCTTCCCAGTCCAAATGAC AGCAAGTTCTTTCAGAATCTCTTGGAT

1010  Glu GluAspLeuGluAspMetMetAspAlaGlu GluTyrLeuValProGlnAlaPheAsnIle ProProProIleTyrThrSerArgAla
3061  GAA GAGGATTTGGAAGATATGATGGATGCTGAG GAGTACTTGGTCCCTCAGGCTTTCAACATC CCACCTCCCATCTATACTTCCAGAGCA

1040  Arg IleAspSerAsnArgSerGluIleGlyHis SerProProProAlaTyrThrProMetSer GlyAsnGlnPheValTyrArgAspGly
3151  AGA ATTGACTCGAATAGGAGTGAAATTGGACAC AGCCCTCCTCCTGCCTACACCCCCATGTCA GGAAACCAGTTTGTATACCGAGATGGA

1070  Gly PheAlaAlaGluGlnGlyValSerValPro TyrArgAlaProThrSerThrIleProGlu AlaProValAlaGlnGlyAlaThrAla
3241  GGT TTTGCTGCTGAACAAGGAGTGTCTGTGCCC TACAGAGCCCCAACTAGCACAATTCCAGAA GCTCCTGTGGCACAGGGTGCTACTGCT

1100  Glu IlePheAspAspSerCysCysAsnGlyThr LeuArgLysProValAlaProHisValGln GluAspSerSerThrGlnArgTyrSer
3331  GAG ATTTTTGATGACTCCTGCTGTAATGGCACC CTACGCAAGCCAGTGGCACCCCATGTCCAA GAGGACAGTAGCACCCAGAGGTACAGT
```

Figure 1
(continued)

```
1130  Ala AspProThrValPheAlaProGluArgSer ProArgGlyGluLeuAspGluGluGlyTyr MetThrProMetArgAspLysProLys
3421  GCT GACCCCACCGTGTTTGCCCCAGAACGGAGC CCACGAGGAGAGCTGGATGAGGAAGGTTAC ATGACTCCTATGCGAGACAAACCCAAA

1160  Gln GluTyrLeuAsnProValGluGluAsnPro PheValSerArgArgLysAsnGlyAspLeu GlnAlaLeuAspAsnProGluTyrHis
3511  CAA GAATACCTGAATCCAGTGGAGGAGAACCCT TTTGTTTCTCGGAGAAAAAATGGAGACCTT CAAGCATTGGATAATCCCGAATATCAC

1190  Asn AlaSerAsnGlyProProLysAlaGluAsp GluTyrValAsnGluProLeuTyrLeuAsn ThrPheAlaAsnThrLeuGlyLysAla
3601  AAT GCATCCAATGGTCCACCCAAGGCCGAGGAT GAGTATGTGAATGAGCCACTGTACCTCAAC ACCTTTGCCAACACCTTGGGAAAAGCT

1220  Glu TyrLeuLysAsnAsnIleLeuSerMetPro GluLysAlaLysLysAlaPheAspAsnPro AspTyrTrpAsnHisSerLeuProPro
3691  GAG TACCTGAAGAACAACATACTGTCAATGCCA GAGAAGGCCAAGAAAGCGTTTGACAACCCT GACTACTGGAACCACAGCCTGCCACCT

1250  Arg SerThrLeuGlnHisProAspTyrLeuGln GluTyrSerThrLysTyrPheTyrLysGln AsnGlyArgIleArgProIleValAla
3781  CGG AGCACCCTTCAGCACCCAGACTACCTGCAG GAGTACAGCACAAAATATTTTTATAAACAG AATGGGCGGATCCGGCCTATTGTGGCA

1280  Glu AsnProGluTyrLeuSerGluPheSerLeu LysProGlyThrValLeuProProProPro TyrArgHisArgAsnThrValVal***
3871  GAG AATCCTGAATACCTCTCTGAGTTCTCCCTG AAGCCAGGCACTGTGCTGCCGCCTCCACCT TACAGACACCGGAATACTGTGGTGTAA

3961  GCTCAGTTGTGGTTTTTTAGGTGGAGAGACACACCTGCTCCAATTTCCCCACCCCCCTCTCTTTCTCTGGTGGTCTTCCTTCTACCCCAAGGC
4054  CAGTAGTTTTGACACTTCCCAGTGGAAGATACAGAGATGCAATGATAGTTATGTGCTTACCTAACTTGAACATTAGAGGGAAAGACTGAAAGA
4147  GAAAGATAGGAGGAACCACAATGTTTCTTCATTTCTCTGCATGGGTTGGTCAGGAGAATGAAACAGCTAGAGAAGGACCAGAAAATGTAAGGC
4240  AATGCTGCCTACTATCAAACTAGCTGTCACTTTTTTTCTTTTTCTTTTTCTTTCTTTGTTTCTTTCTTCCTCTTCTTTTTTTTTTTTTTTTA
4333  AAGCAGATGGTTGAAACACCCATGCTATCTGTTCCTATCTGCAGGAACTGATGTGTGCATATTTAGCATCCCTGGAAATCATAATAAAGTTTC
4426  CATTAGAACAAAAGAATAACATTTTCTATAACATATGATAGTGTCTGAAATTGAGAATCCAGTTTCTTTCCCCAGCAGTTTCTGTCCTAGCAA
4519  GTAAGAATGGCCAACTCAACTTTCATAATTTAAAAATCTCCATTAAAGTTATAACTAGTAATTATGTTTTCAACACTTTTTGGTTTTTTTCAT
4612  TTTGTTTTGCTCTGACCGATTCCTTTATATTTGCTCCCCTATTTTTGGCTTTAATTTCTAATTGCAAAGATGTTTACATCAAAGCTTCTTCAC
4705  AGAATTTAAGCAAGAAATATTTTAATATAGTGAAATGGCCACTACTTTAAGTATACAATCTTTAAAATAAGAAAGGGAGGCTAATATTTTTCA
4798  TGCTATCAAATTATCTTCACCCTCATCCTTTACATTTTTCAACATTTTTTTTCTCCATAAATGACACTACTTGATAGGCCGTTGGTTGTCTG
4891  AAGAGTAGAAGGGAAACTAAGAGACAGTTCTCTGTGGTTCAGGAAAACTACTGATACTTTCAGGGGTGGCCCAATGAGGGAATCCATTGAACT
4984  GGAAGAAACACACTGGATTGGGTATGTCTACCTGGCAGATACTCAGAAATGTAGTTTGCACTTAAGCTGTAATTTTATTTGTTCTTTTTCTGA
5077  ACTCCATTTTGGATTTTGAATCAAGCAATATGGAAGCAACCAGCAAATTAACTAATTTAAGTACATTTTTAAAAAAAGAGCTAAGATAAAGAC
5170  TGTGGAAATGCCAAACCAAGCAAATTAGGAACCTTGCAACGGTATCCAGGGACTATGATGAGAGGCCAGCACATTATCTTCATATGTCACCTT
5263  TGCTACGCAAGGAAATTTGTTCAGTTCGTATACTTCGTAAGAAGGAATGCGAGTAAGGATTGGCTTGAATTCCATGGAATTTCTAGTATGAGA
5356  CTATTTATATGAAGTAGAAGGTAACTCTTTGCACATAAATTGGTATAATAAAAAGAAAAACACAAACATTCAAAGCTTAGGGATAGGTCCTTG
5449  GGTCAAAAGTTGTAAATAAATGTGAAACATCTTCTCAAAAAAAAAAAAAAAAA
```

Figure 1

(continued)

HER4 with alternate 3'-end without AP domain

```
  1                                          MetLysProAlaThrGlyLeuTrpValTrp ValSerLeuLeuValAlaAlaGlyThr
  1  AATTGTCAGCACGGGATCTGAGACTTCCAAAAA ATGAAGCCGGCGACAGGACTTTGGGTCTGG GTGAGCCTTCTCGTGGCGGCGGGGACC

 20  Val GlnProSerAspSerGlnSerValCysAla GlyThrGluAsnLysLeuSerSerLeuSer AspLeuGluGlnGlnTyrArgAlaLeu
 91  GTC CAGCCCAGCGATTCTCAGTCAGTGTGTGCA GGAACGGAGAATAAACTGAGCTCTCTCTCT GACCTGGAACAGCAGTACCGAGCCTTG

 50  Arg LysTyrTyrGluAsnCysGluValValMet GlyAsnLeuGluIleThrSerIleGluHis AsnArgAspLeuSerPheLeuArgSer
181  CGC AAGTACTATGAAAACTGTGAGGTTGTCATG GGCAACCTGGAGATAACCAGCATTGAGCAC AACCGGGACCTCTCCTTCCTGCGGTCT

 80  Val ArgGluValThrGlyTyrValLeuValAla LeuAsnGlnPheArgTyrLeuProLeuGlu AsnLeuArgIleIleArgGlyThrLys
271  GTT CGAGAAGTCACAGGCTACGTGTTAGTGGCT CTTAATCAGTTTCGTTACCTGCCTCTGGAG AATTTACGCATTATTCGTGGGACAAAA

110  Leu TyrGluAspArgTyrAlaLeuAlaIlePhe LeuAsnTyrArgLysAspGlyAsnPheGly LeuGlnGluLeuGlyLeuLysAsnLeu
361  CTT TATGAGGATCGATATGCCTTGGCAATATTT TTAAACTACAGAAAAGATGGAAACTTTGGA CTTCAAGAACTTGGATTAAAGAACTTG

140  Thr GluIleLeuAsnGlyGlyValTyrValAsp GlnAsnLysPheLeuCysTyrAlaAspThr IleHisTrpGlnAspIleValArgAsn
451  ACA GAAATCCTAAATGGTGGAGTCTATGTAGAC CAGAACAAATTCCTTTGTTATGCAGACACC ATTCATTGGCAAGATATTGTTCGGAAC

170  Pro TrpProSerAsnLeuThrLeuValSerThr AsnGlySerSerGlyCysGlyArgCysHis LysSerCysThrGlyArgCysTrpGly
541  CCA TGGCCTTCCAACTTGACTCTTGTGTCAACA AATGGTAGTTCAGGATGTGGACGTTGCCAT AAGTCCTGTACTGGCCGTTGCTGGGGA

200  Pro ThrGluAsnHisCysGlnThrLeuThrArg ThrValCysAlaGluGlnCysAspGlyArg CysTyrGlyProTyrValSerAspCys
631  CCC ACAGAAAATCATTGCCAGACTTTGACAAGG ACGGTGTGTGCAGAACAATGTGACGGCAGA TGCTACGGACCTTACGTCAGTGACTGC

230  Cys HisArgGluCysAlaGlyGlyCysSerGly ProLysAspThrAspCysPheAlaCysMet AsnPheAsnAspSerGlyAlaCysVal
721  TGC CATCGAGAATGTGCTGGAGGCTGCTCAGGA CCTAAGGACACAGACTGCTTTGCCTGCATG AATTTCAATGACAGTGGAGCATGTGTT

260  Thr GlnCysProGlnThrPheValTyrAsnPro ThrThrPheGlnLeuGluHisAsnPheAsn AlaLysTyrThrTyrGlyAlaPheCys
811  ACT CAGTGTCCCCAAACCTTTGTCTACAATCCA ACCACCTTTCAACTGGAGCACAATTTCAAT GCAAAGTACACATATGGAGCATTCTGT

290  Val LysLysCysProHisAsnPheValValAsp SerSerSerCysValArgAlaCysProSer SerLysMetGluValGluGluAsnGly
901  GTC AAGAAATGTCCACATAACTTTGTGGTAGAT TCCAGTTCTTGTGTGCGTGCCTGCCCTAGT TCCAAGATGGAAGTAGAAGAAAATGGG

320  Ile LysMetCysLysProCysThrAspIleCys ProLysAlaCysAspGlyIleGlyThrGly SerLeuMetSerAlaGlnThrValAsp
991  ATT AAAATGTGTAAACCTTGCACTGACATTTGC CCAAAAGCTTGTGATGGCATTGGCACAGGA TCATTGATGTCAGCTCAGACTGTGGAT
```

Figure 2A

```
 350  Ser SerAsnIleAspLysPheIleAsnCysThr LysIleAsnGlyAsnLeuIlePheLeuVal ThrGlyIleHisGlyAspProTyrAsn
1081  TCC AGTAACATTGACAAATTCATAAACTGTACC AAGATCAATGGGAATTTGATCTTTCTAGTC ACTGGTATTCATGGGGACCCTTACAAT

 380  Ala IleGluAlaIleAspProGluLysLeuAsn ValPheArgThrValArgGluIleThrGly PheLeuAsnIleGlnSerTrpProPro
1171  GCA ATTGAAGCCATAGACCCAGAGAAACTGAAC GTCTTTCGGACAGTCAGAGAGATAACAGGT TTCCTGAACATACAGTCATGGCCACCA

 410  Asn MetThrAspPheSerValPheSerAsnLeu ValThrIleGlyGlyArgValLeuTyrSer GlyLeuSerLeuLeuIleLeuLysGln
1261  AAC ATGACTGACTTCAGTGTTTTTTCTAACCTG GTGACCATTGGTGGAAGAGTACTCTATAGT GGCCTGTCCTTGCTTATCCTCAAGCAA

 440  Gln GlyIleThrSerLeuGlnPheGlnSerLeu LysGluIleSerAlaGlyAsnIleTyrIle ThrAspAsnSerAsnLeuCysTyrTyr
1351  CAG GGCATCACCTCTCTACAGTTCCAGTCCCTG AAGGAAATCAGCGCAGGAAACATCTATATT ACTGACAACAGCAACCTGTGTTATTAT

 470  His ThrIleAsnTrpThrThrLeuPheSerThr IleAsnGlnArgIleValIleArgAspAsn ArgLysAlaGluAsnCysThrAlaGlu
1441  CAT ACCATTAACTGGACAACACTCTTCAGCACA ATCAACCAGAGAATAGTAATCCGGGACAAC AGAAAAGCTGAAAATTGTACTGCTGAA

 500  Gly MetValCysAsnHisLeuCysSerSerAsp GlyCysTrpGlyProGlyProAspGlnCys LeuSerCysArgArgPheSerArgGly
1531  GGA ATGGTGTGCAACCATCTGTGTTCCAGTGAT GGCTGTTGGGGACCTGGGCCAGACCAATGT CTGTCGTGTCGCCGCTTCAGTAGAGGA

 530  Arg IleCysIleGluSerCysAsnLeuTyrAsp GlyGluPheArgGluPheGluAsnGlySer IleCysValGluCysAspProGlnCys
1621  AGG ATCTGCATAGAGTCTTGTAACCTCTATGAT GGTGAATTTCGGGAGTTTGAGAATGGCTCC ATCTGTGTGGAGTGTGACCCCCAGTGT

 560  Glu LysMetGluAspGlyLeuLeuThrCysHis GlyProGlyProAspAsnCysThrLysCys SerHisPheLysAspGlyProAsnCys
1711  GAG AAGATGGAAGATGGCCTCCTCACATGCCAT GGACCGGGTCCTGACAACTGTACAAAGTGC TCTCATTTTAAAGATGGCCCAAACTGT

 590  Val GluLysCysProAspGlyLeuGlnGlyAla AsnSerPheIlePheLysTyrAlaAspPro AspArgGluCysHisProCysHisPro
1801  GTG GAAAAATGTCCAGATGGCTTACAGGGGGCA AACAGTTTCATTTTCAAGTATGCTGATCCA GATCGGGAGTGCCACCCATGCCATCCA

 620  Asn CysThrGlnGlyCysAsnGlyProThrSer HisAspCysIleTyrTyrProTrpThrGly HisSerThrLeuProGlnHisAlaArg
1891  AAC TGCACCCAAGGGTGTAACGGTCCCACTAGT CATGACTGCATTTACTACCCATGGACGGGC CATTCCACTTTACCACAACATGCTAGA

 650  Thr ProLeuIleAlaAlaGlyValIleGlyGly LeuPheIleLeuValIleValGlyLeuThr PheAlaValTyrValArgArgLysSer
1981  ACT CCCCTGATTGCAGCTGGAGTAATTGGTGGG CTCTTCATTCTGGTCATTGTGGGTCTGACA TTTGCTGTTTATGTTAGAAGGAAGAGC
```

Figure 2A
(continued)

```
680  Ile LysLysLysArgAlaLeuArgArgPheLeu GluThrGluLeuValGluProLeuThrPro SerGlyThrAlaProAsnGlnAlaGln
2071 ATC AAAAAGAAAAGAGCCTTGAGAAGATTCTTG GAAACAGAGTTGGTGGAACCATTAACTCCC AGTGGCACAGCACCCAATCAAGCTCAA

710  Leu ArgIleLeuLysGluThrGluLeuLysArg ValLysValLeuGlySerGlyAlaPheGly ThrValTyrLysGlyIleTrpValPro
2161 CTT CGTATTTTGAAAGAAACTGAGCTGAAGAGG GTAAAAGTCCTTGGCTCAGGTGCTTTTGGA ACGGTTTATAAAGGTATTTGGGTACCT

740  Glu GlyGluThrValLysIleProValAlaIle LysIleLeuAsnGluThrThrGlyProLys AlaAsnValGluPheMetAspGluAla
2251 GAA GGAGAAACTGTGAAGATTCCTGTGGCTATT AAGATTCTTAATGAGACAACTGGTCCCAAG GCAAATGTGGAGTTCATGGATGAAGCT

770  Leu IleMetAlaSerMetAspHisProHisLeu ValArgLeuLeuGlyValCysLeuSerPro ThrIleGlnLeuValThrGlnLeuMet
2341 CTG ATCATGGCAAGTATGGATCATCCACACCTA GTCCGGTTGCTGGGTGTGTGTCTGAGCCCA ACCATCCAGCTGGTTACTCAACTTATG

800  Pro HisGlyCysLeuLeuGluTyrValHisGlu HisLysAspAsnIleGlySerGlnLeuLeu LeuAsnTrpCysValGlnIleAlaLys
2431 CCC CATGGCTGCCTGTTGGAGTATGTCCACGAG CACAAGGATAACATTGGATCACAACTGCTG CTTAACTGGTGTGTCCAGATAGCTAAG

830  Gly MetMetTyrLeuGluGluArgArgLeuVal HisArgAspLeuAlaAlaArgAsnValLeu ValLysSerProAsnHisValLysIle
2521 GGA ATGATGTACCTGGAAGAAAGACGACTCGTT CATCGGGATTTGGCAGCCCGTAATGTCTTA GTGAAATCTCCAAACCATGTGAAAATC

860  Thr AspPheGlyLeuAlaArgLeuLeuGluGly AspGluLysGluTyrAsnAlaAspGlyGly LysMetProIleLysTrpMetAlaLeu
2611 ACA GATTTTGGGCTAGCCAGACTCTTGGAAGGA GATGAAAAAGAGTACAATGCTGATGGAGGA AAGATGCCAATTAAATGGATGGCTCTG

890  Glu CysIleHisTyrArgLysPheThrHisGln SerAspValTrpSerTyrGlyValThrIle TrpGluLeuMetThrPheGlyGlyLys
2701 GAG TGTATACATTACAGGAAATTCACCCATCAG AGTGACGTTTGGAGCTATGGAGTTACTATA TGGGAACTGATGACCTTTGGAGGAAAA

920  Pro TyrAspGlyIleProThrArgGluIlePro AspLeuLeuGluLysGlyGluArgLeuPro GlnProProIleCysThrIleAspVal
2791 CCC TATGATGGAATTCCAACGCGAGAAATCCCT GATTTATTAGAGAAAGGAGAACGTTTGCCT CAGCCTCCCATCTGCACTATTGACGTT

950  Tyr MetValMetValLysCysTrpMetIleAsp AlaAspSerArgProLysPheLysGluLeu AlaAlaGluPheSerArgMetAlaArg
2881 TAC ATGGTCATGGTCAAATGTTGGATGATTGAT GCTGACAGTAGACCTAAATTTAAGGAACTG GCTGCTGAGTTTTCAAGGATGGCTCGA

980  Asp ProGlnArgTyrLeuValIleGlnGlyAsp AspArgMetLysLeuProSerProAsnAsp SerLysPhePheGlnAsnLeuLeuAsp
2971 GAC CCTCAAAGATACCTAGTTATTCAGGGTGAT GATCGTATGAAGCTTCCCAGTCCAAATGAC AGCAAGTTCTTTCAGAATCTCTTGGAT

1010 Glu GluAspLeuGluAspMetMetAspAlaGlu GluTyrLeuValProGlnAlaPheAsnIle ProProProIleTyrThrSerArgAla
3061 GAA GAGGATTTGGAAGATATGATGGATGCTGAG GAGTACTTGGTCCCTCAGGCTTTCAACATC CCACCTCCCATCTATACTTCCAGAGCA
```

Figure 2A
(continued)

1040 Arg IleAspSerAsnArgSerValArgAsnAsn TyrIleHisIleSerTyrSerPhe***
3151 AGA ATTGACTCGAATAGGAGTGTAAGAAATAAT TATATACACATATCATATTCTTTCTGA

3211 GATATAAAATCATGTAATAGTTCATAAGCACTAACATTTCAAAATAATTATATAGCTCAAATCAATGTGATGCCTAGATTAAAAATATAC
3301 CATACCCACAAAAGATGTGCCAATCTTGCTATATGTAGTTAATTTTGGAAGACAAGCATGGACAATACAACATGTACTCTGAAATACCTT
3391 CAAGATTTCAGAAGCAAAACATTTTCCTCATCTTAATTTATTTAAAACAAATCTTAACTTTAAAAAACAATTCCAACTAATAAAACCATT
3481 ATGTGTATATAAATAAATGAAAATTCCTACCAAGTAGGCTTTCTACTTTTCTTTCTTAAAAAGATATTATGATATATTAGTCAAGAAGTA
3571 ATACAAGTATAAATCTCTTTCACTTATTTAAGAAAAATTAAATATTTTCTGTCAAGTTGAAGTAGAAACACAGAAAACCGTGCAGTCCTT
3661 TGAACCTAATCACATCGAAAAGGCTGCTGAGAAGTAGATTTTTGTTTTTAAGAAGTAGATTTAAGTTTTGAAGGAAGTTTCTGAAAACAC
3751 TTTACATTTTAAATGTTAAACCTACTCTATATGAATTCCATTCTTTCTTTGAAAGCTGTCAAATCCATGCATTTATTTTTATAAATTCAT
3841 TCCTCATACATTCAACATATATTGAGTACCACTGTATGTGAAGCATTAGTATACATTTAAGACTCAAAGAATTTTGATACAACTTCTGCT
3931 TTCAAGAAGTGAAAACCTTAATCAAAGAATCATACAGATAGAGGGACTGCATAGTAAGTGCTGTAATCCAGTATTCACTGACCAGTACGG
4021 AGCATGAAGAAGTAGTAAATTTGTGTCTGTAATCAGTTTCTTCCATTGATAAGATATAAACATGATGCTTAATTTTTTCTAGAAGATAAT
4111 TCTTTTCTCTTAATCTAAGAACATTATCATAGCTAGTAGAACCGACAGCATCCGATTTCTCTTGACCATAGCCATAAGAATATCTTCAAC
4201 TTGCTGCTCATTATCTAACAAACATAATTTCTTTATTTCATATTGATTGTAATAAGTAATATCCCCCTGGAAGTTTACTATTCAACACA
4291 TATATGTTAACCTCCTTAATTCCTTAAACAAACTTCATGAGGTTCTATTATTATCATCCCCTTCTTTCAAAGGAAGAAACTTGCCACAGA
4381 GAAGTCAGGTGATATGACTGGTGTCACACAGCTAGTCAGTGGAAGAGAGGAATAAGTAATCTAGATATCTGCCTACTACACTGTAGGTTT
4471 GCTTCAAAGTTACTGAAGYCATGTTATTTCCATGATGTGATTAGAGTCTGGGACTTGTCTTGTTTGGGAAATTTCCCAGGTGGTTTTCTT
4561 ATAAAATGCATCTCAAATCTGCTCTACACCTTTTACTCATCTACCTCCATTTAGAAGATCTGATATGGAAAGAGACAAAGATGGAGACCT
4651 CAATTATTTTTTCTTTTCTGTTAAAAATATTATAGTACAACTGAAACTTATCACATGCCAATGGGGAATAGATAACTAAAAGTTTAAAAT
4741 TAGATCAATGGATAGGTAAATGAATAATCNTTCTTTTGCTTGTGAGAGGGGAAGGAAAAGCGGTTAAGGTGGTATAAAGGAGGCTCCTCT
4831 GTACACTTGCAAAATGATCAAATTATATACCCTTGTATTTATAATTTTAAGTGACAAATTCATTACTTCTGGTTACAACAGTGAAATTTA
4921 AAAAAAAATAGTTTTTCTTTCTTAGCTTGCAATGCTATAAATCTTTTTCTTTTTATAAGAATTCTTACATTTCAGCTTTTTGTTCATTTT
5011 AATTTATAATTCTCAGTGCAAGAAATTCTTAATAAAGGTTTGAGCTAGCTAGATGGAATTATTGAGACAAAGTCTAAATCACCCGTGGAC
5101 TTATTTGACCTTTAGCCATCATTTCTTATTCCACATTATAAAACAATGTTACCTGTAGATTTCTTTTTACTTTTTCAGTCCTTGGAAAAG
5191 AAATGGTGATTAAATATCATTATATCATTTTATGTTCAGGCATTTAAAAAGCTTTATTTGTCATCTATATTGTCCTAATAGTTTTCAGTC
5281 TGGCTTTACGTAACTTTTACGGAAATTTCTAACATGTACAAATGCCATGTTCCTCCTTTCTTTCCTACATGGCTGAATTAGAAAACAAAT
5371 TACTTCCATTTTAAGTTTGGCTAAATTAGAAAACAAATTACTACCATTTTAAGTTTGGTGGCTAAATAACGTGCTAAGGGAACATCTTAA
5461 AAAGTGAATTTTGATCAAATATTTCTTAAGCATATGTGATAGACTTTGAAACCAAAAAAAAAAAAAAAAAAAAAAAAAAAAAAAAAAAAA
5551 AAAAA

Figure 2A
(continued)

```
     HER4 N-terminal truncated with AP domain

   1 CATTAGCTGCAATTGATCAAGTGACTGAGAGAAGGGCAACATTCCATGCAACAGTATAGTGGTATGGAAAGCCCTGGATGTTGA
  85 AATCTAGCTTCAAAAAGCCTGTCTGGAAATGTAGTTAATTGGATGAAGTGAGAAGAGATAAAACCAGAGAG GAAGCTCTGATC

   1 MetAlaSerMetAspHisProHisLeuVal ArgLeuLeuGlyValCysLeuSerProThr IleGlnLeuValThrGlnLeuMetProHis
 168 ATGGCAAGTATGGATCATCCACACCTAGTC CGGTTGCTGGGTGTGTGTCTGAGCCCAACC ATCCAGCTGGTTACTCAACTTATGCCCCAT

  31 GlyCysLeuLeuGluTyrValHisGluHis LysAspAsnIleGlySerGlnLeuLeuLeu AsnTrpCysValGlnIleAlaLysGlyMet
 258 GGCTGCCTGTTGGAGTATGTCCACGAGCAC AAGGATAACATTGGATCACAACTGCTGCTT AACTGGTGTGTCCAGATAGCTAAGGGAATG

  61 MetTyrLeuGluGluArgArgLeuValHis ArgAspLeuAlaAlaArgAsnValLeuVal LysSerProAsnHisValLysIleThrAsp
 348 ATGTACCTGGAAGAAAGACGACTCGTTCAT CGGGATTTGGCAGCCCGTAATGTCTTAGTG AAATCTCCAAACCATGTGAAAATCACAGAT

  91 PheGlyLeuAlaArgLeuLeuGluGlyAsp GluLysGluTyrAsnAlaAspGlyGlyLys MetProIleLysTrpMetAlaLeuGluCys
 438 TTTGGGCTAGCCAGACTCTTGGAAGGAGAT GAAAAAGAGTACAATGCTGATGGAGGAAAG ATGCCAATTAAATGGATGGCTCTGGAGTGT

 121 IleHisTyrArgLysPheThrHisGlnSer AspValTrpSerTyrGlyValThrIleTrp GluLeuMetThrPheGlyGlyLysProTyr
 528 ATACATTACAGGAAATTCACCCATCAGAGT GACGTTTGGAGCTATGGAGTTACTATATGG GAACTGATGACCTTTGGAGGAAAACCCTAT

 151 AspGlyIleProThrArgGluIleProAsp LeuLeuGluLysGlyGluArgLeuProGln ProProIleCysThrIleAspValTyrMet
 618 GATGGAATTCCAACGCGAGAAATCCCTGAT TTATTAGAGAAAGGAGAACGTTTGCCTCAG CCTCCCATCTGCACTATTGACGTTTACATG

 181 ValMetValLysCysTrpMetIleAspAla AspSerArgProLysPheLysGluLeuAla AlaGluPheSerArgMetAlaArgAspPro
 708 GTCATGGTCAAATGTTGGATGATTGATGCT GACAGTAGACCTAAATTTAAGGAACTGGCT GCTGAGTTTTCAAGGATGGCTCGAGACCCT

 211 GlnArgTyrLeuValIleGlnGlyAspAsp ArgMetLysLeuProSerProAsnAspSer LysPhePheGlnAsnLeuLeuAspGluGlu
 798 CAAAGATACCTAGTTATTCAGGGTGATGAT CGTATGAAGCTTCCCAGTCCAAATGACAGC AAGTTCTTTCAGAATCTCTTGGATGAAGAG

 241 AspLeuGluAspMetMetAspAlaGluGlu TyrLeuValProGlnAlaPheAsnIlePro ProProIleTyrThrSerArgAlaArgIle
 888 GATTTGGAAGATATGATGGATGCTGAGGAG TACTTGGTCCCTCAGGCTTTCAACATCCCA CCTCCCATCTATACTTCCAGAGCAAGAATT

 271 AspSerAsnArgSerGluIleGlyHisSer ProProProAlaTyrThrProMetSerGly AsnGlnPheValTyrArgAspGlyGlyPhe
 978 GACTCGAATAGGAGTGAAATTGGACACAGC CCTCCTCCTGCCTACACCCCCATGTCAGGA AACCAGTTTGTATACCGAGATGGAGGTTTT

 301 AlaAlaGluGlnGlyValSerValProTyr ArgAlaProThrSerThrIleProGluAla ProValAlaGlnGlyAlaThrAlaGluIle
1068 GCTGCTGAACAAGGAGTGTCTGTGCCCTAC AGAGCCCCAACTAGCACAATTCCAGAAGCT CCTGTGGCACAGGGTGCTACTGCTGAGATT
```

Figure 2B

```
 331  PheAspAspSerCysCysAsnGlyThrLeu ArgLysProValAlaProHisValGlnGlu AspSerSerThrGlnArgTyrSerAlaAsp
1158  TTTGATGACTCCTGCTGTAATGGCACCCTA CGCAAGCCAGTGGCACCCCATGTCCAAGAG GACAGTAGCACCCAGAGGTACAGTGCTGAC

 361  ProThrValPheAlaProGluArgSerPro ArgGlyGluLeuAspGluGluGlyTyrMet ThrProMetArgAspLysProLysGlnGlu
1248  CCCACCGTGTTTGCCCCAGAACGGAGCCCA CGAGGAGAGCTGGATGAGGAAGGTTACATG ACTCCTATGCGAGACAAACCCAAACAAGAA

 391  TyrLeuAsnProValGluGluAsnProPhe ValSerArgArgLysAsnGlyAspLeuGln AlaLeuAspAsnProGluTyrHisAsnAla
1338  TACCTGAATCCAGTGGAGGAGAACCCTTTT GTTTCTCGGAGAAAAAATGGAGACCTTCAA GCATTGGATAATCCCGAATATCACAATGCA

 421  SerAsnGlyProProLysAlaGluAspGlu TyrValAsnGluProLeuTyrLeuAsnThr PheAlaAsnThrLeuGlyLysAlaGluTyr
1428  TCCAATGGTCCACCCAAGGCCGAGGATGAG TATGTGAATGAGCCACTGTACCTCAACACC TTTGCCAACACCTTGGGAAAAGCTGAGTAC

 451  LeuLysAsnAsnIleLeuSerMetProGlu LysAlaLysLysAlaPheAspAsnProAsp TyrTrpAsnHisSerLeuProProArgSer
1518  CTGAAGAACAACATACTGTCAATGCCAGAG AAGGCCAAGAAAGCGTTTGACAACCCTGAC TACTGGAACCACAGCCTGCCACCTCGGAGC

 481  ThrLeuGlnHisProAspTyrLeuGlnGlu TyrSerThrLysTyrPheTyrLysGlnAsn GlyArgIleArgProIleValAlaGluAsn
1608  ACCCTTCAGCACCCAGACTACCTGCAGGAG TACAGCACAAAATATTTTTATAAACAGAAT GGGCGGATCCGGCCTATTGTGGCAGAGAAT

 511  ProGluTyrLeuSerGluPheSerLeuLys ProGlyThrValLeuProProProProTyr ArgHisArgAsnThrValVal***
1698  CCTGAATACCTCTCTGAGTTCTCCCTGAAG CCAGGCACTGTGCTGCCGCCTCCACCTTAC AGACACCGGAATACTGTGGTGTAA

1783  GCTCAGTTGTGGTTTTTAGGTGGAGAGACACACCTGCTCCAATTTCCCCACCCCCCTCTCTTTCTCTGGTGGTCTTCCTTCTACCCCAAGGC
1876  CAGTAGTTTTGACACTTCCCAGTGGAAGATACAGAGATGCAATGATAGTTATGTGCTTACCTAACTTGAACATTAGAGGGAAAGACTGAAAGA
1969  GAAAGATAGGAGGAACCACAATGTTTCTTCATTTCTCTGCATGGGTTGGTCAGGAGAATGAAACAGCTAGAGAAGGACCAGAAAATGTAAGGC
2062  AATGCTGCCTACTATCAAACTAGCTGTCACTTTTTTTCTTTTTCTTTTTCTTTCTTTGTTTCTTTCTTCCTCTTCTTTTTTTTTTTTTTTTA
2155  AAGCAGATGGTTGAAACACCCATGCTATCTGTTCCTATCTGCAGGAACTGATGTGTGCATATTTAGCATCCCTGGAAATCATAATAAAGTTTC
2248  CATTAGAACAAAAGAATAACATTTTCTATAACATATGATAGTGTCTGAAATTGAGAATCCAGTTTCTTTCCCCAGCAGTTTCTGTCCTAGCAA
2341  GTAAGAATGGCCAACTCAACTTTCATAATTTAAAAATCTCCATTAAAGTTATAACTAGTAATTATGTTTTCAACACTTTTTGGTTTTTTTCAT
2434  TTTGTTTTGCTCTGACCGATTCCTTTATATTTGCTCCCCTATTTTTGGCTTTAATTTCTAATTGCAAAGATGTTTACATCAAAGCTTCTTCAC
2527  AGAATTTAAGCAAGAAATATTTTAATATAGTGAAATGGCCACTACTTTAAGTATACAATCTTTAAAATAAGAAAGGGAGGCTAATATTTTTCA
2620  TGCTATCAAATTATCTTCACCCTCATCCTTTACATTTTTCAACATTTTTTTTTCTCCATAAATGACACTACTTGATAGGCCGTTGGTTGTCTG
2713  AAGAGTAGAAGGGAAACTAAGAGACAGTTCTCTGTGGTTCAGGAAAACTACTGATACTTTCAGGGGTGGCCCAATGAGGGAATCCATTGAACT
2806  GGAAGAAACACACTGGATTGGGTATGTCTACCTGGCAGATACTCAGAAATGTAGTTTGCACTTAAGCTGTAATTTTATTTGTTCTTTTTCTGA
2899  ACTCCATTTTGGATTTTGAATCAAGCAATATGGAAGCAACCAGCAAATTAACTAATTTAAGTACATTTTTAAAAAAAGAGCTAAGATAAAGAC
2992  TGTGGAAATGCCAAACCAAGCAAATTAGGAACCTTGCAACGGTATCCAGGGACTATGATGAGAGGCCAGCACATTATCTTCATATGTCACCTT
3085  TGCTACGCAAGGAAATTTGTTCAGTTCGTATACTTCGTAAGAAGGAATGCGAGTAAGGATTGGCTTGAATTCCATGGAATTTCTAGTATGAGA
3178  CTATTTATATGAAGTAGAAGGTAACTCTTTGCACATAAATTGGTATAATAAAAAGAAAAACACAAACATTCAAAGCTTAGGGATAGGTCCTTG
3271  GGTCAAAAGTTGTAAATAAATGTGAAACATCTTCTCAAAAAAAAAAAAAAAA
```

Figure 2B

(continued)

HER4
HER4 with alternate 3'-end without Autophosphorylation domain

```
MKPATGLWVWVSLLVAAGTVQPSDSQSVCAGTENKLSSLSDLEQQYRALRKYYENCEVVM      60
::::::::::::::::::::::::::::::::::::::::::::::::::::::::::::
MKPATGLWVWVSLLVAAGTVQPSDSQSVCAGTENKLSSLSDLEQQYRALRKYYENCEVVM      60

GNLEITSIEHNRDLSFLRSVREVTGYVLVALNQFRYLPLENLRIIRGTKLYEDRYALAIF     120
::::::::::::::::::::::::::::::::::::::::::::::::::::::::::::
GNLEITSIEHNRDLSFLRSVREVTGYVLVALNQFRYLPLENLRIIRGTKLYEDRYALAIF     120

LNYRKDGNFGLQELGLKNLTEILNGGVYVDQNKFLCYADTIHWQDIVRNPWPSNLTLVST     180
::::::::::::::::::::::::::::::::::::::::::::::::::::::::::::
LNYRKDGNFGLQELGLKNLTEILNGGVYVDQNKFLCYADTIHWQDIVRNPWPSNLTLVST     180

NGSSGCGRCHKSCTGRCWGPTENHCQTLTRTVCAEQCDGRCYGPYVSDCCHRECAGGCSG     240
::::::::::::::::::::::::::::::::::::::::::::::::::::::::::::
NGSSGCGRCHKSCTGRCWGPTENHCQTLTRTVCAEQCDGRCYGPYVSDCCHRECAGGCSG     240

PKDTDCFACMNFNDSGACVTQCPQTFVYNPTTFQLEHNFNAKYTYGAFCVKKCPHNFVVD     300
::::::::::::::::::::::::::::::::::::::::::::::::::::::::::::
PKDTDCFACMNFNDSGACVTQCPQTFVYNPTTFQLEHNFNAKYTYGAFCVKKCPHNFVVD     300

SSSCVRACPSSKMEVEENGIKMCKPCTDICPKACDGIGTGSLMSAQTVDSSNIDKFINCT     360
::::::::::::::::::::::::::::::::::::::::::::::::::::::::::::
SSSCVRACPSSKMEVEENGIKMCKPCTDICPKACDGIGTGSLMSAQTVDSSNIDKFINCT     360

KINGNLIFLVTGIHGDPYNAIEAIDPEKLNVFRTVREITGFLNIQSWPPNMTDFSVFSNL     420
::::::::::::::::::::::::::::::::::::::::::::::::::::::::::::
KINGNLIFLVTGIHGDPYNAIEAIDPEKLNVFRTVREITGFLNIQSWPPNMTDFSVFSNL     420

VTIGGRVLYSGLSLLILKQQGITSLQFQSLKEISAGNIYITDNSNLCYYHTINWTTLFST     480
::::::::::::::::::::::::::::::::::::::::::::::::::::::::::::
VTIGGRVLYSGLSLLILKQQGITSLQFQSLKEISAGNIYITDNSNLCYYHTINWTTLFST     480

INQRIVIRDNRKAENCTAEGMVCNHLCSSDGCWGPGPDQCLSCRRFSRGRICIESCNLYD     540
::::::::::::::::::::::::::::::::::::::::::::::::::::::::::::
INQRIVIRDNRKAENCTAEGMVCNHLCSSDGCWGPGPDQCLSCRRFSRGRICIESCNLYD     540

GEFREFENGSICVECDPQCEKMEDGLLTCHGPGPDNCTKCSHFKDGPNCVEKCPDGLQGA     600
::::::::::::::::::::::::::::::::::::::::::::::::::::::::::::
GEFREFENGSICVECDPQCEKMEDGLLTCHGPGPDNCTKCSHFKDGPNCVEKCPDGLQGA     600
```

Figure 3A

```
NSFIFKYADPDRECHPCHPNCTQGCNGPTSHDCIYYPWTGHSTLPQHARTPLIAAGVIGG   660
::::::::::::::::::::::::::::::::::::::::::::::::::::::::::::
NSFIFKYADPDRECHPCHPNCTQGCNGPTSHDCIYYPWTGHSTLPQHARTPLIAAGVIGG   660

LFILVIVGLTFAVYVRRKSIKKKRALRRFLETELVEPLTPSGTAPNQAQLRILKETELKR   720
::::::::::::::::::::::::::::::::::::::::::::::::::::::::::::
LFILVIVGLTFAVYVRRKSIKKKRALRRFLETELVEPLTPSGTAPNQAQLRILKETELKR   720

VKVLGSGAFGTVYKGIWVPEGETVKIPVAIKILNETTGPKANVEFMDEALIMASMDHPHL   780
::::::::::::::::::::::::::::::::::::::::::::::::::::::::::::
VKVLGSGAFGTVYKGIWVPEGETVKIPVAIKILNETTGPKANVEFMDEALIMASMDHPHL   780

VRLLGVCLSPTIQLVTQLMPHGCLLEYVHEHKDNIGSQLLLNWCVQIAKGMMYLEERRLV   840
::::::::::::::::::::::::::::::::::::::::::::::::::::::::::::
VRLLGVCLSPTIQLVTQLMPHGCLLEYVHEHKDNIGSQLLLNWCVQIAKGMMYLEERRLV   840

HRDLAARNVLVKSPNHVKITDFGLARLLEGDEKEYNADGGKMPIKWMALECIHYRKFTHQ   900
::::::::::::::::::::::::::::::::::::::::::::::::::::::::::::
HRDLAARNVLVKSPNHVKITDFGLARLLEGDEKEYNADGGKMPIKWMALECIHYRKFTHQ   900

SDVWSYGVTIWELMTFGGKPYDGIPTREIPDLLEKGERLPQPPICTIDVYMVMVKCWMID   960
::::::::::::::::::::::::::::::::::::::::::::::::::::::::::::
SDVWSYGVTIWELMTFGGKPYDGIPTREIPDLLEKGERLPQPPICTIDVYMVMVKCWMID   960

ADSRPKFKELAAEFSRMARDPQRYLVIQGDDRMKLPSPNDSKFFQNLLDEEDLEDMMDAE  1020
::::::::::::::::::::::::::::::::::::::::::::::::::::::::::::
ADSRPKFKELAAEFSRMARDPQRYLVIQGDDRMKLPSPNDSKFFQNLLDEEDLEDMMDAE  1020

EYLVPQAFNIPPPIYTSRARIDSNRSEIGHSPPPAYTPMSGNQFVYRDGGFAAEQGVSVP  1080
::::::::::::::::::::::::::        :.
EYLVPQAFNIPPPIYTSRARIDSNRVRNNYIHIS-YSF                        1057

YRAPTSTIPEAPVAQGATAEIFDDSCCNGTLRKPVAPHVQEDSSTQRYSADPTVFAPERS  1140
PRGELDEEGYMTPMRDKPKQEYLNPVEENPFVSRRKNGDLQALDNPEYHNASNGPPKAED  1200
EYVNEPLYLNTFANTLGKAEYLKNNILSMPEKAKKAFDNPDYWNHSLPPRSTLQHPDYLQ  1260
EYSTKYFYKQNGRIRPIVAENPEYLSEFSLKPGTVLPPPPYRHRNTVV              1308
```

Aligned 1058, Matches 1046, Mismatches 12, Score 132, Homology 98%

Figure 3A
(continued)

HER4
HER4 N-terminal truncated with autophosphorylation domain

```
MKPATGLWVWVSLLVAAGTVQPSDSQSVCAGTENKLSSLSDLEQQYRALRKYYENCEVVM   60
GNLEITSIEHNRDLSFLRSVREVTGYVLVALNQFRYLPLENLRIIRGTKLYEDRYALAIF  120
LNYRKDGNFGLQELGLKNLTEILNGGVYVDQNKFLCYADTIHWQDIVRNPWPSNLTLVST  180
NGSSGCGRCHKSCTGRCWGPTENHCQTLTRTVCAEQCDGRCYGPYVSDCCHRECAGGCSG  240
PKDTDCFACMNFNDSGACVTQCPQTFVYNPTTFQLEHNFNAKYTYGAFCVKKCPHNFVVD  300
SSSCVRACPSSKMEVEENGIKMCKPCTDICPKACDGIGTGSLMSAQTVDSSNIDKFINCT  360
KINGNLIFLVTGIHGDPYNAIEAIDPEKLNVFRTVREITGFLNIQSWPPNMTDFSVFSNL  420
VTIGGRVLYSGLSLLILKQQGITSLQFQSLKEISAGNIYITDNSNLCYYHTINWTTLFST  480
INQRIVIRDNRKAENCTAEGMVCNHLCSSDGCWGPGPDQCLSCRRFSRGRICIESCNLYD  540
GEFREFENGSICVECDPQCEKMEDGLLTCHGPGPDNCTKCSHFKDGPNCVEKCPDGLQGA  600
NSFIFKYADPDRECHPCHPNCTQGCNGPTSHDCIYYPWTGHSTLPQHARTPLIAAGVIGG  660

VKVLGSGAFGTVYKGIWVPEGETVKIPVAIKILNETTGPKANVEFMDEALIMASMDHPHL  780
                                                ::::::::::::
                                                EALIMASMDHPHL  13

VRLLGVCLSPTIQLVTQLMPHGCLLEYVHEHKDNIGSQLLLNWCVQIAKGMMYLEERRLV  840
::::::::::::::::::::::::::::::::::::::::::::::::::::::::::::
VRLLGVCLSPTIQLVTQLMPHGCLLEYVHEHKDNIGSQLLLNWCVQIAKGMMYLEERRLV   73

HRDLAARNVLVKSPNHVKITDFGLARLLEGDEKEYNADGGKMPIKWMALECIHYRKFTHQ  900
::::::::::::::::::::::::::::::::::::::::::::::::::::::::::::
HRDLAARNVLVKSPNHVKITDFGLARLLEGDEKEYNADGGKMPIKWMALECIHYRKFTHQ  133

SDVWSYGVTIWELMTFGGKPYDGIPTREIPDLLEKGERLPQPPICTIDVYMVMVKCWMID  960
::::::::::::::::::::::::::::::::::::::::::::::::::::::::::::
SDVWSYGVTIWELMTFGGKPYDGIPTREIPDLLEKGERLPQPPICTIDVYMVMVKCWMID  193

ADSRPKFKELAAEFSRMARDPQRYLVIQGDDRMKLPSPNDSKFFQNLLDEEDLEDMMDAE 1020
::::::::::::::::::::::::::::::::::::::::::::::::::::::::::::
ADSRPKFKELAAEFSRMARDPQRYLVIQGDDRMKLPSPNDSKFFQNLLDEEDLEDMMDAE  253

EYLVPQAFNIPPPIYTSRARIDSNRSEIGHSPPPAYTPMSGNQFVYRDGGFAAEQGVSVP 1080
::::::::::::::::::::::::::::::::::::::::::::::::::::::::::::
EYLVPQAFNIPPPIYTSRARIDSNRSEIGHSPPPAYTPMSGNQFVYRDGGFAAEQGVSVP  313

YRAPTSTIPEAPVAQGATAEIFDDSCCNGTLRKPVAPHVQEDSSTQRYSADPTVFAPERS 1140
::::::::::::::::::::::::::::::::::::::::::::::::::::::::::::
YRAPTSTIPEAPVAQGATAEIFDDSCCNGTLRKPVAPHVQEDSSTQRYSADPTVFAPERS  373

PRGELDEEGYMTPMRDKPKQEYLNPVEENPFVSRRKNGDLQALDNPEYHNASNGPPKAED 1200
::::::::::::::::::::::::::::::::::::::::::::::::::::::::::::
PRGELDEEGYMTPMRDKPKQEYLNPVEENPFVSRRKNGDLQALDNPEYHNASNGPPKAED  433

EYVNEPLYLNTFANTLGKAEYLKNNILSMPEKAKKAFDNPDYWNHSLPPRSTLQHPDYLQ 1260
::::::::::::::::::::::::::::::::::::::::::::::::::::::::::::
EYVNEPLYLNTFANTLGKAEYLKNNILSMPEKAKKAFDNPDYWNHSLPPRSTLQHPDYLQ  493

EYSTKYFYKQNGRIRPIVAENPEYLSEFSLKPGTVLPPPPYRHRNTVV             1308
::::::::::::::::::::::::::::::::::::::::::::::::
EYSTKYFYKQNGRIRPIVAENPEYLSEFSLKPGTVLPPPPYRHRNTVV              541
```

Aligned 541, Matches 541, Mismatches 0, Score 130, Homology 100%

Figure 3B

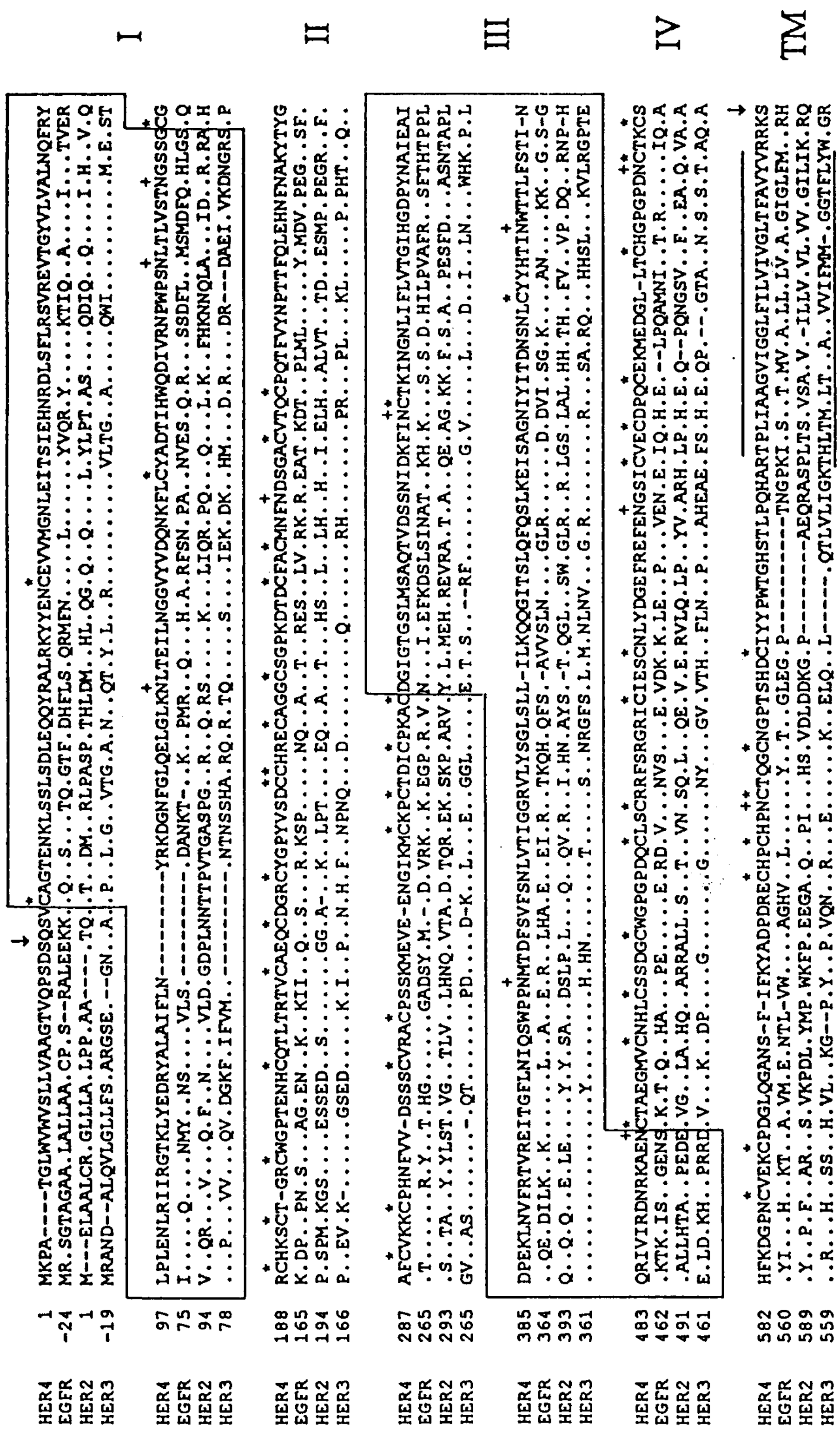


Figure 4

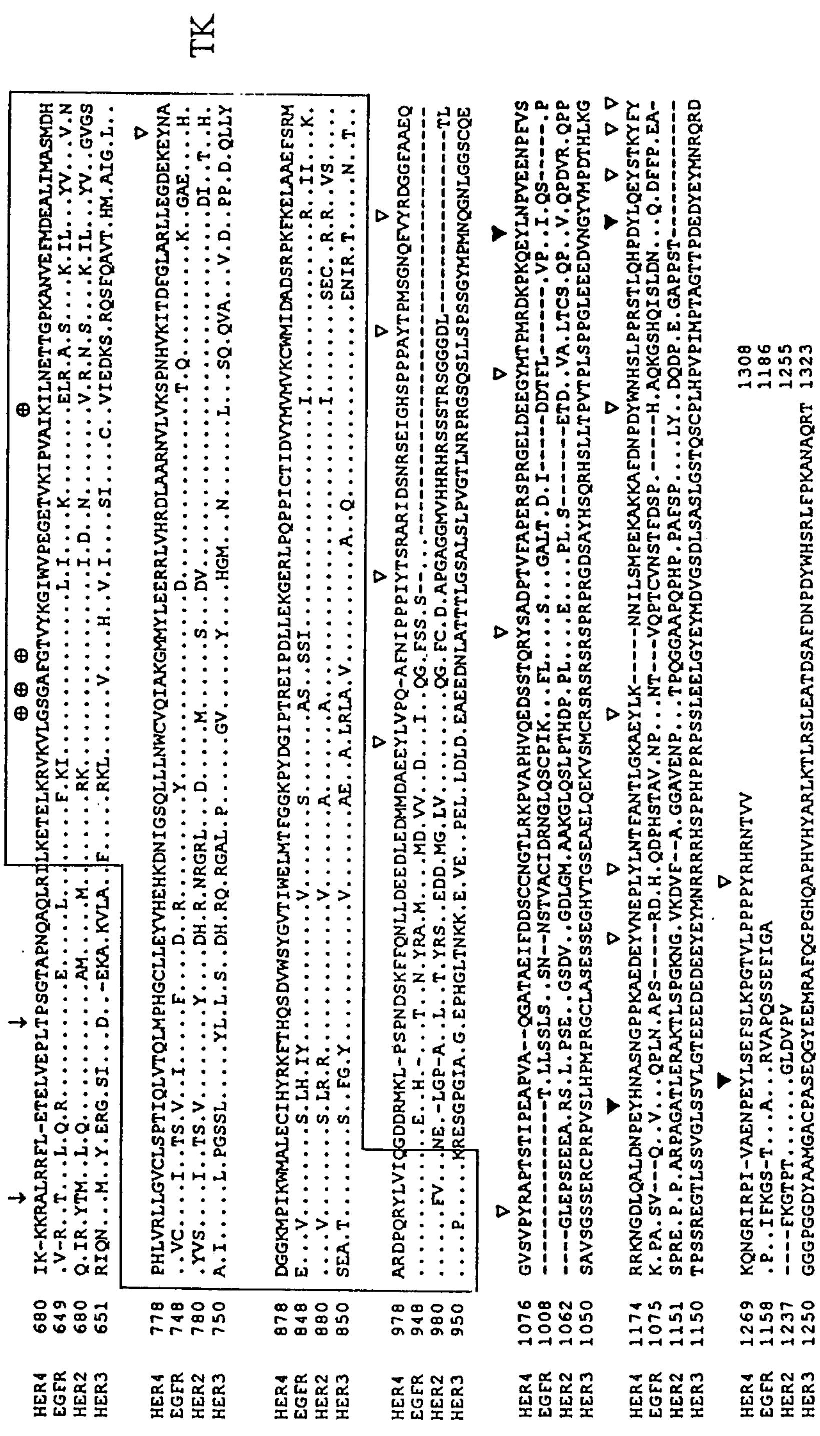


Figure 4

(continued)

FIGURE 5

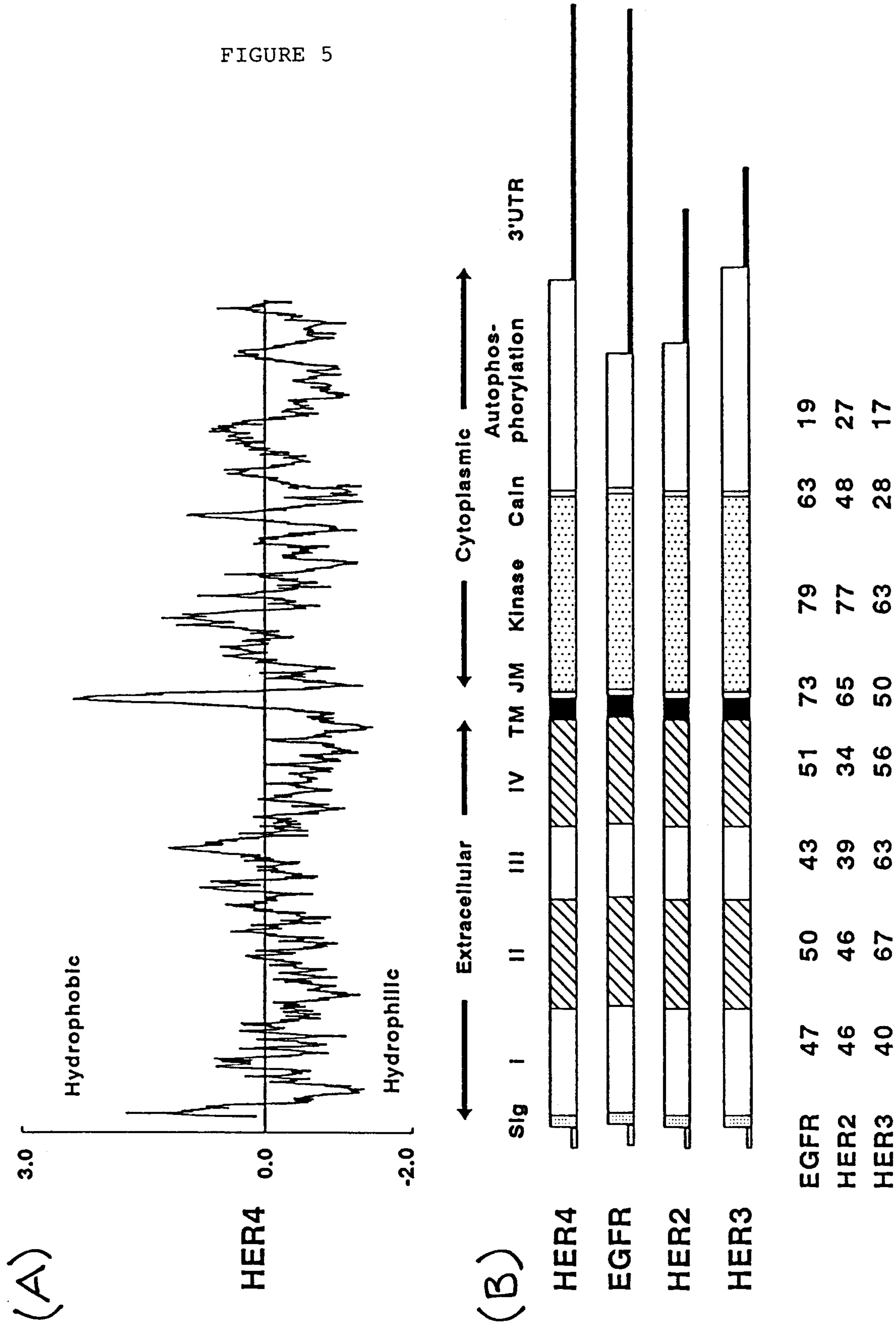
(A)
HER4
3.0
0.0
-2.0
Hydrophobic
Hydrophilic
(B)
Extracellular
Cytoplasmic
SIg
I
II
III
IV
TM
JM
Kinase
Caln
Autophos-
phorylation
3'UTR
HER4
EGFR
HER2
HER3
EGFR 47 50 43 51 73 79 63 19
HER2 46 46 39 34 65 77 48 27
HER3 40 67 63 56 50 63 28 17

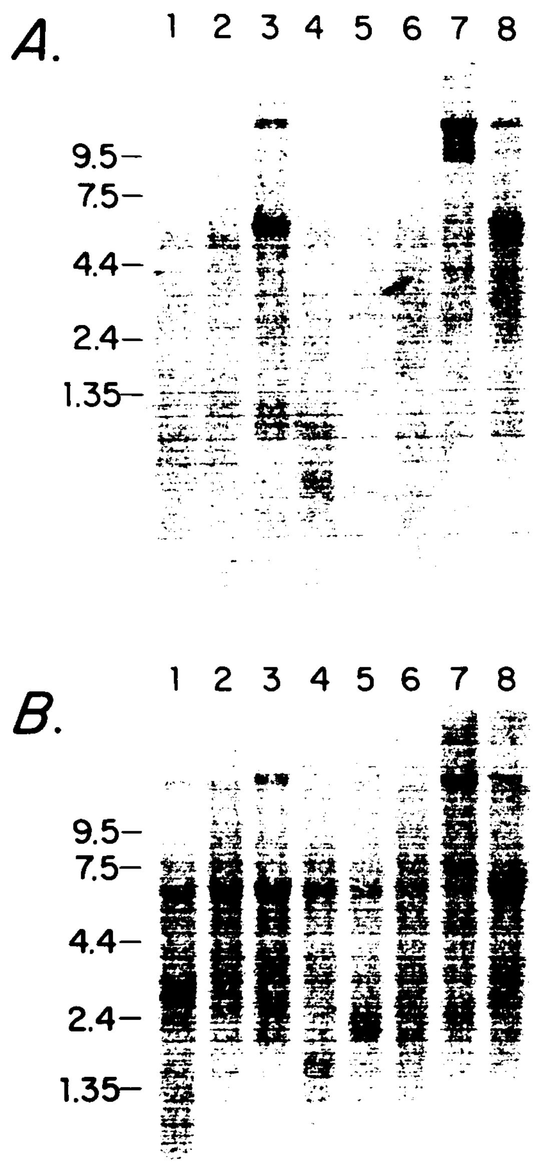


FIGURE 6

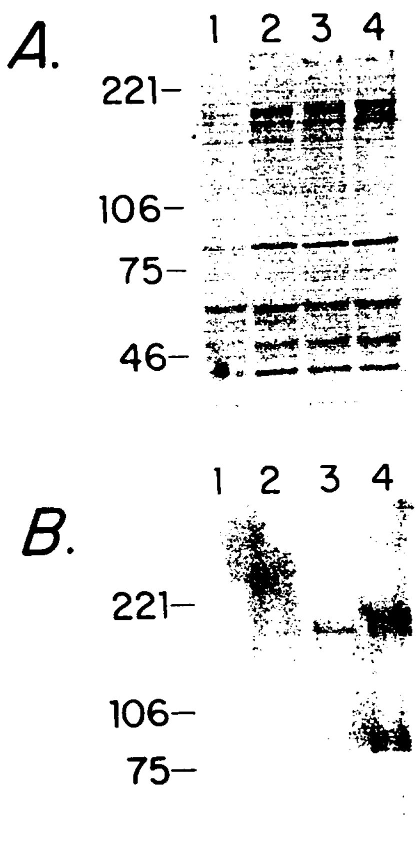


FIGURE 7

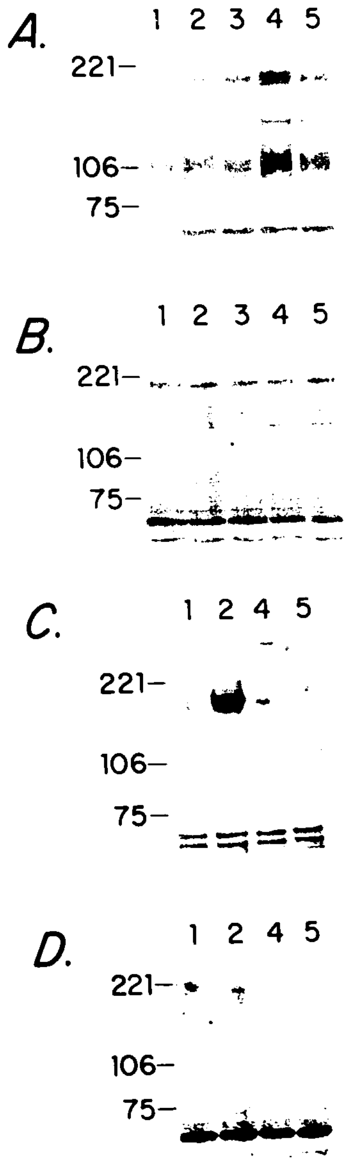
A.
1 2 3 4 5
221—
106—
75—
B.
1 2 3 4 5
221—
106—
75—
C.
1 2 4 5
221—
106—
75—
D.
1 2 4 5
221—
106—
75—

FIGURE 8

# Biological and Biochemical Properties of the MDA-MB-453-cell Differentiation Activity

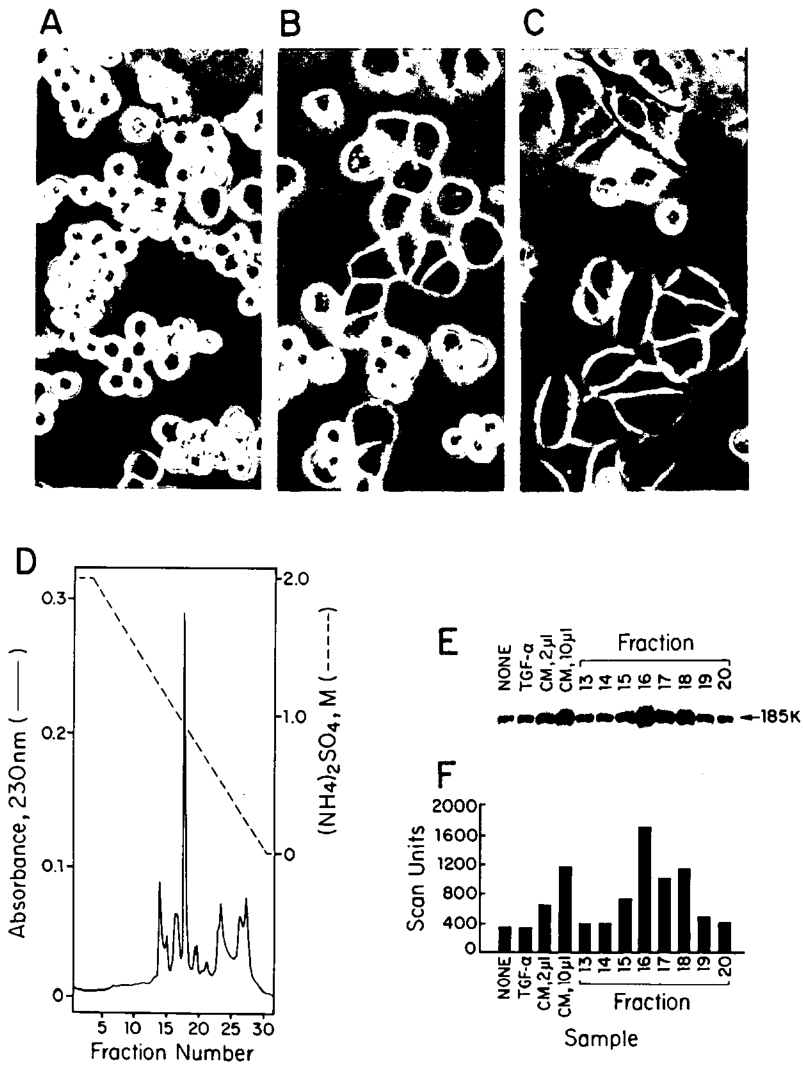


FIGURE 9

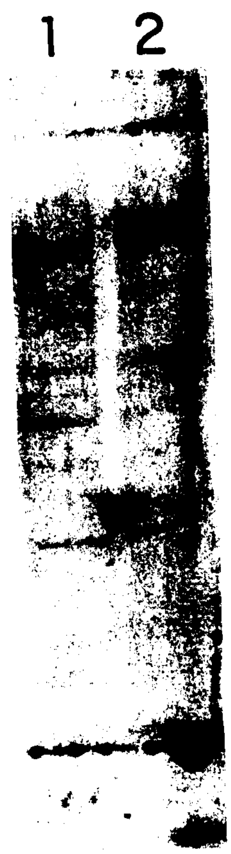


FIGURE 10A

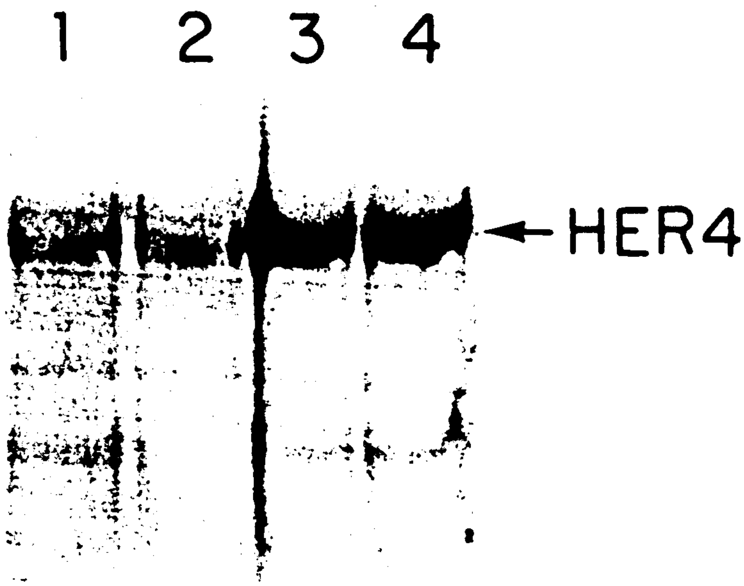


FIGURE 10B

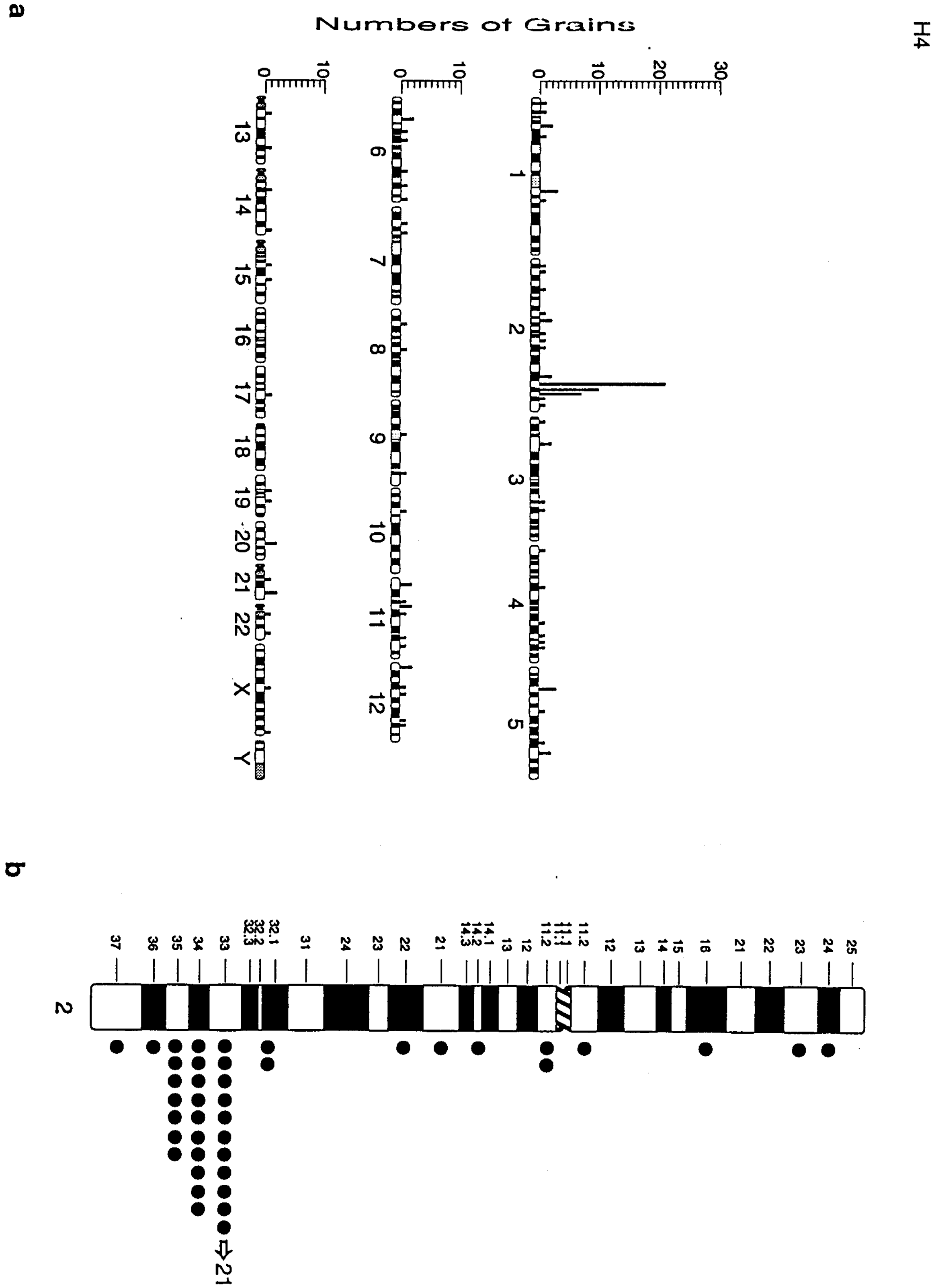


Figure 11

HER4-Ig
HER4 extracellular domain-human Ig fusion construct

MKPATGLWVWVSLLVAAGTVQPSDSQSVCAGTENKLSSLSDLEQQYRALRKYYENCEVVM
GNLEITSIEHNRDLSFLRSVREVTGYVLVALNQFRYLPLENLRIIRGTKLYEDRYALAIF
LNYRKDGNFGLQELGLKNLTEILNGGVYVDQNKFLCYADTIHWQDIVRNPWPSNLTLVST
NGSSGCGRCHKSCTGRCWGPTENHCQTLTRTVCAEQCDGRCYGPYVSDCCHRECAGGCSG
PKDTDCFACMNFNDSGACVTQCPQTFVYNPTTFQLEHNFNAKYTYGAFCVKKCPHNFVVD
SSSCVRACPSSKMEVEENGIKMCKPCTDICPKACDGIGTGSLMSAQTVDSSNIDKFINCT
KINGNLIFLVTGIHGDPYNAIEAIDPEKLNVFRTVREITGFLNIQSWPPNMTDFSVFSNL
VTIGGRVLYSGLSLLILKQQGITSLQFQSLKEISAGNIYITDNSNLCYYHTINWTTLFST
INQRIVIRDNRKAENCTAEGMVCNHLCSSDGCWGPGPDQCLSCRRFSRGRICIESCNLYD
GEFREFENGSICVECDPQCEKMEDGLLTCHGPGPDNCTKCSHFKDGPNCVEKCPDGLQGA
NSFIFKYADPDRECHPCHPNCTQGCNGPTSHDCIYYPWTGHSTLPQDPVKVKALEGFPRL
VGPDFFGCAEPANTFLDPEEPKSCDKTHTCPPCPAPELLGGPSVFLFPPKPKDTLMISRT
PEVTCVVVDVSHEDPEVKFNWYVDGVEVHVAKTKPREEQYNSTYRVVSVLTVLHQDWLNG
KEYKCKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHEALHNHY
TQKSLSLSPGK

Bold = Signal Sequence
= Immunoglobin domain

Lower case = HER4 ECD

Figure 12

European Patent Office

# PARTIAL EUROPEAN SEARCH REPORT

which under Rule 45 of the European Patent Convention shall be considered, for the purposes of subsequent proceedings, as the European search report

Application Number EP 93 11 8837

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| P,X | WO-A-92 20798 (GENENTECH, US) 26 November 1992<br>* Abstract, claims *<br>--- | 14,15, 28,29 | C12N15/12<br>C07K13/00<br>C12P21/08<br>C12N5/10<br>G01N33/68<br>G01N33/577<br>A61K39/395 |
| P,X | PROCEEDINGS OF THE NATIONAL ACADEMY OF SCIENCES OF USA.<br>vol. 90 , 1 March 1993 , WASHINGTON US<br>pages 1746 - 1750<br>PLOWMAN GD;CULOUSCOU JM;WHITNEY GS;GREEN JM;CARLTON GW;FOY L;NEUBAUER MG;SHOYAB M; 'Ligand-specific activation of HER4/p180erbB4, a fourth member of the epidermal growth factor receptor family.'<br>* the whole document *<br>--- | 1-32 | |
| A | SCIENCE<br>vol. 256 , 22 May 1992 , LANCASTER, PA<br>pages 1205 - 1210<br>HOLMES, W.E. ET AL.; 'Identification of heregulin, a specific activator of p185erbb2'<br>* the whole document *<br>---<br>-/-- | 14,15 | |

| TECHNICAL FIELDS SEARCHED (Int.Cl.5) |
|---|
| C12N<br>C07K<br>G01N |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 9 March 1994 | Nauche, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 93 11 8837

| Category | Citation of document with indication, where appropriate, of relevant passages | Relevant to claim | CLASSIFICATION OF THE APPLICATION (Int.Cl.5) |
|---|---|---|---|
| P,X | JOURNAL OF BIOLOGICAL CHEMISTRY. vol. 268, no. 25 , September 1993 , BALTIMORE US pages 18407 - 18410 CULOUSCOU JM;PLOWMAN GD;CARLTON GW;GREEN JM;SHOYAB M; 'Characterization of a breast cancer cell differentiation factor that specifically activates the HER4/p180erbB4 receptor.' * page 18410, column 1, line 4 - page 18410, column 2, line 2 * | 14,15 | |
| A | EP-A-0 444 961 (BRISTOL-MYERS SQUIBB COMPANY) 4 September 1991 * the whole document * | 1-32 | TECHNICAL FIELDS SEARCHED (Int.Cl.5) |
| A | WO-A-90 14357 (GENENTECH, US) 29 November 1990 | | |

EPO FORM 1503 03.82 (P04C10)

SHEET C

EP 93118837.9

Remark : Although claim 32 is directed to a method of treatment of human/animal body, the search has been carried out and based on the alleged effects of the compound/composition.

SEQUENCE LISTING

(1) GENERAL INFORMATION:

(i) APPLICANT:
(A) NAME: BRISTOL-MYERS SQUIBB COMPANY
(B) STREET: 345 Park Avenue
(C) CITY: New York
(D) STATE: New York
(E) COUNTRY: U.S.A.
(F) POSTAL CODE (ZIP): 10154

(ii) TITLE OF INVENTION: HER4 HUMAN RECEPTOR TYROSINE KINASE

(iii) NUMBER OF SEQUENCES: 30

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(2) INFORMATION FOR SEQ ID NO:1:

(i) SEQUENCE CHARACTERISTICS:
(A) LENGTH: 5501 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: CDS
(B) LOCATION: 34..3961

(xi) SEQUENCE DESCRIPTION: SEQ ID NO:1:

```
AATTGTCAGC ACGGGATCTG AGACTTCCAA AAA ATG AAG CCG GCG ACA GGA CTT      54
                                     Met Lys Pro Ala Thr Gly Leu
                                      1               5

TGG GTC TGG GTG AGC CTT CTC GTG GCG GCG GGG ACC GTC CAG CCC AGC      102
Trp Val Trp Val Ser Leu Leu Val Ala Ala Gly Thr Val Gln Pro Ser
            10                  15                  20

GAT TCT CAG TCA GTG TGT GCA GGA ACG GAG AAT AAA CTG AGC TCT CTC      150
Asp Ser Gln Ser Val Cys Ala Gly Thr Glu Asn Lys Leu Ser Ser Leu
    25                  30                  35

TCT GAC CTG GAA CAG CAG TAC CGA GCC TTG CGC AAG TAC TAT GAA AAC      198
Ser Asp Leu Glu Gln Gln Tyr Arg Ala Leu Arg Lys Tyr Tyr Glu Asn
40                  45                  50                  55

TGT GAG GTT GTC ATG GGC AAC CTG GAG ATA ACC AGC ATT GAG CAC AAC      246
Cys Glu Val Val Met Gly Asn Leu Glu Ile Thr Ser Ile Glu His Asn
                60                  65                  70

CGG GAC CTC TCC TTC CTG CGG TCT GTT CGA GAA GTC ACA GGC TAC GTG      294
Arg Asp Leu Ser Phe Leu Arg Ser Val Arg Glu Val Thr Gly Tyr Val
                75                  80                  85

TTA GTG GCT CTT AAT CAG TTT CGT TAC CTG CCT CTG GAG AAT TTA CGC      342
Leu Val Ala Leu Asn Gln Phe Arg Tyr Leu Pro Leu Glu Asn Leu Arg
            90                  95                  100

ATT ATT CGT GGG ACA AAA CTT TAT GAG GAT CGA TAT GCC TTG GCA ATA      390
Ile Ile Arg Gly Thr Lys Leu Tyr Glu Asp Arg Tyr Ala Leu Ala Ile
    105                 110                 115

TTT TTA AAC TAC AGA AAA GAT GGA AAC TTT GGA CTT CAA GAA CTT GGA      438
Phe Leu Asn Tyr Arg Lys Asp Gly Asn Phe Gly Leu Gln Glu Leu Gly
120                 125                 130                 135

TTA AAG AAC TTG ACA GAA ATC CTA AAT GGT GGA GTC TAT GTA GAC CAG      486
Leu Lys Asn Leu Thr Glu Ile Leu Asn Gly Gly Val Tyr Val Asp Gln
                140                 145                 150

AAC AAA TTC CTT TGT TAT GCA GAC ACC ATT CAT TGG CAA GAT ATT GTT      534
Asn Lys Phe Leu Cys Tyr Ala Asp Thr Ile His Trp Gln Asp Ile Val
            155                 160                 165

CGG AAC CCA TGG CCT TCC AAC TTG ACT CTT GTG TCA ACA AAT GGT AGT      582
Arg Asn Pro Trp Pro Ser Asn Leu Thr Leu Val Ser Thr Asn Gly Ser
            170                 175                 180

TCA GGA TGT GGA CGT TGC CAT AAG TCC TGT ACT GGC CGT TGC TGG GGA      630
Ser Gly Cys Gly Arg Cys His Lys Ser Cys Thr Gly Arg Cys Trp Gly
    185                 190                 195

CCC ACA GAA AAT CAT TGC CAG ACT TTG ACA AGG ACG GTG TGT GCA GAA      678
Pro Thr Glu Asn His Cys Gln Thr Leu Thr Arg Thr Val Cys Ala Glu
200                 205                 210                 215
```

```
CAA TGT GAC GGC AGA TGC TAC GGA CCT TAC GTC AGT GAC TGC TGC CAT      726
Gln Cys Asp Gly Arg Cys Tyr Gly Pro Tyr Val Ser Asp Cys Cys His
                220                 225                 230

CGA GAA TGT GCT GGA GGC TGC TCA GGA CCT AAG GAC ACA GAC TGC TTT      774
Arg Glu Cys Ala Gly Gly Cys Ser Gly Pro Lys Asp Thr Asp Cys Phe
            235                 240                 245

GCC TGC ATG AAT TTC AAT GAC AGT GGA GCA TGT GTT ACT CAG TGT CCC      822
Ala Cys Met Asn Phe Asn Asp Ser Gly Ala Cys Val Thr Gln Cys Pro
        250                 255                 260

CAA ACC TTT GTC TAC AAT CCA ACC ACC TTT CAA CTG GAG CAC AAT TTC      870
Gln Thr Phe Val Tyr Asn Pro Thr Thr Phe Gln Leu Glu His Asn Phe
    265                 270                 275

AAT GCA AAG TAC ACA TAT GGA GCA TTC TGT GTC AAG AAA TGT CCA CAT      918
Asn Ala Lys Tyr Thr Tyr Gly Ala Phe Cys Val Lys Lys Cys Pro His
280                 285                 290                 295

AAC TTT GTG GTA GAT TCC AGT TCT TGT GTG CGT GCC TGC CCT AGT TCC      966
Asn Phe Val Val Asp Ser Ser Ser Cys Val Arg Ala Cys Pro Ser Ser
                300                 305                 310

AAG ATG GAA GTA GAA GAA AAT GGG ATT AAA ATG TGT AAA CCT TGC ACT     1014
Lys Met Glu Val Glu Glu Asn Gly Ile Lys Met Cys Lys Pro Cys Thr
            315                 320                 325

GAC ATT TGC CCA AAA GCT TGT GAT GGC ATT GGC ACA GGA TCA TTG ATG     1062
Asp Ile Cys Pro Lys Ala Cys Asp Gly Ile Gly Thr Gly Ser Leu Met
        330                 335                 340

TCA GCT CAG ACT GTG GAT TCC AGT AAC ATT GAC AAA TTC ATA AAC TGT     1110
Ser Ala Gln Thr Val Asp Ser Ser Asn Ile Asp Lys Phe Ile Asn Cys
    345                 350                 355

ACC AAG ATC AAT GGG AAT TTG ATC TTT CTA GTC ACT GGT ATT CAT GGG     1158
Thr Lys Ile Asn Gly Asn Leu Ile Phe Leu Val Thr Gly Ile His Gly
360                 365                 370                 375

GAC CCT TAC AAT GCA ATT GAA GCC ATA GAC CCA GAG AAA CTG AAC GTC     1206
Asp Pro Tyr Asn Ala Ile Glu Ala Ile Asp Pro Glu Lys Leu Asn Val
                380                 385                 390

TTT CGG ACA GTC AGA GAG ATA ACA GGT TTC CTG AAC ATA CAG TCA TGG     1254
Phe Arg Thr Val Arg Glu Ile Thr Gly Phe Leu Asn Ile Gln Ser Trp
            395                 400                 405

CCA CCA AAC ATG ACT GAC TTC AGT GTT TTT TCT AAC CTG GTG ACC ATT     1302
Pro Pro Asn Met Thr Asp Phe Ser Val Phe Ser Asn Leu Val Thr Ile
        410                 415                 420

GGT GGA AGA GTA CTC TAT AGT GGC CTG TCC TTG CTT ATC CTC AAG CAA     1350
Gly Gly Arg Val Leu Tyr Ser Gly Leu Ser Leu Leu Ile Leu Lys Gln
    425                 430                 435
```

```
CAG GGC ATC ACC TCT CTA CAG TTC CAG TCC CTG AAG GAA ATC AGC GCA       1398
Gln Gly Ile Thr Ser Leu Gln Phe Gln Ser Leu Lys Glu Ile Ser Ala
440                 445                 450                 455

GGA AAC ATC TAT ATT ACT GAC AAC AGC AAC CTG TGT TAT TAT CAT ACC       1446
Gly Asn Ile Tyr Ile Thr Asp Asn Ser Asn Leu Cys Tyr Tyr His Thr
            460                 465                 470

ATT AAC TGG ACA ACA CTC TTC AGC ACA ATC AAC CAG AGA ATA GTA ATC       1494
Ile Asn Trp Thr Thr Leu Phe Ser Thr Ile Asn Gln Arg Ile Val Ile
        475                 480                 485

CGG GAC AAC AGA AAA GCT GAA AAT TGT ACT GCT GAA GGA ATG GTG TGC       1542
Arg Asp Asn Arg Lys Ala Glu Asn Cys Thr Ala Glu Gly Met Val Cys
    490                 495                 500

AAC CAT CTG TGT TCC AGT GAT GGC TGT TGG GGA CCT GGG CCA GAC CAA       1590
Asn His Leu Cys Ser Ser Asp Gly Cys Trp Gly Pro Gly Pro Asp Gln
505                 510                 515

TGT CTG TCG TGT CGC CGC TTC AGT AGA GGA AGG ATC TGC ATA GAG TCT       1638
Cys Leu Ser Cys Arg Arg Phe Ser Arg Gly Arg Ile Cys Ile Glu Ser
520                 525                 530                 535

TGT AAC CTC TAT GAT GGT GAA TTT CGG GAG TTT GAG AAT GGC TCC ATC       1686
Cys Asn Leu Tyr Asp Gly Glu Phe Arg Glu Phe Glu Asn Gly Ser Ile
            540                 545                 550

TGT GTG GAG TGT GAC CCC CAG TGT GAG AAG ATG GAA GAT GGC CTC CTC       1734
Cys Val Glu Cys Asp Pro Gln Cys Glu Lys Met Glu Asp Gly Leu Leu
        555                 560                 565

ACA TGC CAT GGA CCG GGT CCT GAC AAC TGT ACA AAG TGC TCT CAT TTT       1782
Thr Cys His Gly Pro Gly Pro Asp Asn Cys Thr Lys Cys Ser His Phe
    570                 575                 580

AAA GAT GGC CCA AAC TGT GTG GAA AAA TGT CCA GAT GGC TTA CAG GGG       1830
Lys Asp Gly Pro Asn Cys Val Glu Lys Cys Pro Asp Gly Leu Gln Gly
585                 590                 595

GCA AAC AGT TTC ATT TTC AAG TAT GCT GAT CCA GAT CGG GAG TGC CAC       1878
Ala Asn Ser Phe Ile Phe Lys Tyr Ala Asp Pro Asp Arg Glu Cys His
600                 605                 610                 615

CCA TGC CAT CCA AAC TGC ACC CAA GGG TGT AAC GGT CCC ACT AGT CAT       1926
Pro Cys His Pro Asn Cys Thr Gln Gly Cys Asn Gly Pro Thr Ser His
            620                 625                 630

GAC TGC ATT TAC TAC CCA TGG ACG GGC CAT TCC ACT TTA CCA CAA CAT       1974
Asp Cys Ile Tyr Tyr Pro Trp Thr Gly His Ser Thr Leu Pro Gln His
        635                 640                 645

GCT AGA ACT CCC CTG ATT GCA GCT GGA GTA ATT GGT GGG CTC TTC ATT       2022
Ala Arg Thr Pro Leu Ile Ala Ala Gly Val Ile Gly Gly Leu Phe Ile
    650                 655                 660
```

```
CTG GTC ATT GTG GGT CTG ACA TTT GCT GTT TAT GTT AGA AGG AAG AGC      2070
Leu Val Ile Val Gly Leu Thr Phe Ala Val Tyr Val Arg Arg Lys Ser
    665                 670                 675

ATC AAA AAG AAA AGA GCC TTG AGA AGA TTC TTG GAA ACA GAG TTG GTG      2118
Ile Lys Lys Lys Arg Ala Leu Arg Arg Phe Leu Glu Thr Glu Leu Val
680                 685                 690                 695

GAA CCA TTA ACT CCC AGT GGC ACA GCA CCC AAT CAA GCT CAA CTT CGT      2166
Glu Pro Leu Thr Pro Ser Gly Thr Ala Pro Asn Gln Ala Gln Leu Arg
                700                 705                 710

ATT TTG AAA GAA ACT GAG CTG AAG AGG GTA AAA GTC CTT GGC TCA GGT      2214
Ile Leu Lys Glu Thr Glu Leu Lys Arg Val Lys Val Leu Gly Ser Gly
            715                 720                 725

GCT TTT GGA ACG GTT TAT AAA GGT ATT TGG GTA CCT GAA GGA GAA ACT      2262
Ala Phe Gly Thr Val Tyr Lys Gly Ile Trp Val Pro Glu Gly Glu Thr
        730                 735                 740

GTG AAG ATT CCT GTG GCT ATT AAG ATT CTT AAT GAG ACA ACT GGT CCC      2310
Val Lys Ile Pro Val Ala Ile Lys Ile Leu Asn Glu Thr Thr Gly Pro
    745                 750                 755

AAG GCA AAT GTG GAG TTC ATG GAT GAA GCT CTG ATC ATG GCA AGT ATG      2358
Lys Ala Asn Val Glu Phe Met Asp Glu Ala Leu Ile Met Ala Ser Met
760                 765                 770                 775

GAT CAT CCA CAC CTA GTC CGG TTG CTG GGT GTG TGT CTG AGC CCA ACC      2406
Asp His Pro His Leu Val Arg Leu Leu Gly Val Cys Leu Ser Pro Thr
                780                 785                 790

ATC CAG CTG GTT ACT CAA CTT ATG CCC CAT GGC TGC CTG TTG GAG TAT      2454
Ile Gln Leu Val Thr Gln Leu Met Pro His Gly Cys Leu Leu Glu Tyr
            795                 800                 805

GTC CAC GAG CAC AAG GAT AAC ATT GGA TCA CAA CTG CTG CTT AAC TGG      2502
Val His Glu His Lys Asp Asn Ile Gly Ser Gln Leu Leu Leu Asn Trp
        810                 815                 820

TGT GTC CAG ATA GCT AAG GGA ATG ATG TAC CTG GAA GAA AGA CGA CTC      2550
Cys Val Gln Ile Ala Lys Gly Met Met Tyr Leu Glu Glu Arg Arg Leu
    825                 830                 835

GTT CAT CGG GAT TTG GCA GCC CGT AAT GTC TTA GTG AAA TCT CCA AAC      2598
Val His Arg Asp Leu Ala Ala Arg Asn Val Leu Val Lys Ser Pro Asn
840                 845                 850                 855

CAT GTG AAA ATC ACA GAT TTT GGG CTA GCC AGA CTC TTG GAA GGA GAT      2646
His Val Lys Ile Thr Asp Phe Gly Leu Ala Arg Leu Leu Glu Gly Asp
                860                 865                 870

GAA AAA GAG TAC AAT GCT GAT GGA GGA AAG ATG CCA ATT AAA TGG ATG      2694
Glu Lys Glu Tyr Asn Ala Asp Gly Gly Lys Met Pro Ile Lys Trp Met
            875                 880                 885
```

```
GCT CTG GAG TGT ATA CAT TAC AGG AAA TTC ACC CAT CAG AGT GAC GTT       2742
Ala Leu Glu Cys Ile His Tyr Arg Lys Phe Thr His Gln Ser Asp Val
        890                 895                 900

TGG AGC TAT GGA GTT ACT ATA TGG GAA CTG ATG ACC TTT GGA GGA AAA       2790
Trp Ser Tyr Gly Val Thr Ile Trp Glu Leu Met Thr Phe Gly Gly Lys
    905                 910                 915

CCC TAT GAT GGA ATT CCA ACG CGA GAA ATC CCT GAT TTA TTA GAG AAA       2838
Pro Tyr Asp Gly Ile Pro Thr Arg Glu Ile Pro Asp Leu Leu Glu Lys
920                 925                 930                 935

GGA GAA CGT TTG CCT CAG CCT CCC ATC TGC ACT ATT GAC GTT TAC ATG       2886
Gly Glu Arg Leu Pro Gln Pro Pro Ile Cys Thr Ile Asp Val Tyr Met
                940                 945                 950

GTC ATG GTC AAA TGT TGG ATG ATT GAT GCT GAC AGT AGA CCT AAA TTT       2934
Val Met Val Lys Cys Trp Met Ile Asp Ala Asp Ser Arg Pro Lys Phe
            955                 960                 965

AAG GAA CTG GCT GCT GAG TTT TCA AGG ATG GCT CGA GAC CCT CAA AGA       2982
Lys Glu Leu Ala Ala Glu Phe Ser Arg Met Ala Arg Asp Pro Gln Arg
        970                 975                 980

TAC CTA GTT ATT CAG GGT GAT GAT CGT ATG AAG CTT CCC AGT CCA AAT       3030
Tyr Leu Val Ile Gln Gly Asp Asp Arg Met Lys Leu Pro Ser Pro Asn
    985                 990                 995

GAC AGC AAG TTC TTT CAG AAT CTC TTG GAT GAA GAG GAT TTG GAA GAT       3078
Asp Ser Lys Phe Phe Gln Asn Leu Leu Asp Glu Glu Asp Leu Glu Asp
1000                1005                1010                1015

ATG ATG GAT GCT GAG GAG TAC TTG GTC CCT CAG GCT TTC AAC ATC CCA       3126
Met Met Asp Ala Glu Glu Tyr Leu Val Pro Gln Ala Phe Asn Ile Pro
                1020                1025                1030

CCT CCC ATC TAT ACT TCC AGA GCA AGA ATT GAC TCG AAT AGG AGT GAA       3174
Pro Pro Ile Tyr Thr Ser Arg Ala Arg Ile Asp Ser Asn Arg Ser Glu
            1035                1040                1045

ATT GGA CAC AGC CCT CCT CCT GCC TAC ACC CCC ATG TCA GGA AAC CAG       3222
Ile Gly His Ser Pro Pro Pro Ala Tyr Thr Pro Met Ser Gly Asn Gln
        1050                1055                1060

TTT GTA TAC CGA GAT GGA GGT TTT GCT GCT GAA CAA GGA GTG TCT GTG       3270
Phe Val Tyr Arg Asp Gly Gly Phe Ala Ala Glu Gln Gly Val Ser Val
    1065                1070                1075

CCC TAC AGA GCC CCA ACT AGC ACA ATT CCA GAA GCT CCT GTG GCA CAG       3318
Pro Tyr Arg Ala Pro Thr Ser Thr Ile Pro Glu Ala Pro Val Ala Gln
1080                1085                1090                1095

GGT GCT ACT GCT GAG ATT TTT GAT GAC TCC TGC TGT AAT GGC ACC CTA       3366
Gly Ala Thr Ala Glu Ile Phe Asp Asp Ser Cys Cys Asn Gly Thr Leu
                1100                1105                1110
```

```
CGC AAG CCA GTG GCA CCC CAT GTC CAA GAG GAC AGT AGC ACC CAG AGG     3414
Arg Lys Pro Val Ala Pro His Val Gln Glu Asp Ser Ser Thr Gln Arg
            1115                1120                1125

TAC AGT GCT GAC CCC ACC GTG TTT GCC CCA GAA CGG AGC CCA CGA GGA     3462
Tyr Ser Ala Asp Pro Thr Val Phe Ala Pro Glu Arg Ser Pro Arg Gly
        1130                1135                1140

GAG CTG GAT GAG GAA GGT TAC ATG ACT CCT ATG CGA GAC AAA CCC AAA     3510
Glu Leu Asp Glu Glu Gly Tyr Met Thr Pro Met Arg Asp Lys Pro Lys
    1145                1150                1155

CAA GAA TAC CTG AAT CCA GTG GAG GAG AAC CCT TTT GTT TCT CGG AGA     3558
Gln Glu Tyr Leu Asn Pro Val Glu Glu Asn Pro Phe Val Ser Arg Arg
1160                1165                1170                1175

AAA AAT GGA GAC CTT CAA GCA TTG GAT AAT CCC GAA TAT CAC AAT GCA     3606
Lys Asn Gly Asp Leu Gln Ala Leu Asp Asn Pro Glu Tyr His Asn Ala
                1180                1185                1190

TCC AAT GGT CCA CCC AAG GCC GAG GAT GAG TAT GTG AAT GAG CCA CTG     3654
Ser Asn Gly Pro Pro Lys Ala Glu Asp Glu Tyr Val Asn Glu Pro Leu
            1195                1200                1205

TAC CTC AAC ACC TTT GCC AAC ACC TTG GGA AAA GCT GAG TAC CTG AAG     3702
Tyr Leu Asn Thr Phe Ala Asn Thr Leu Gly Lys Ala Glu Tyr Leu Lys
        1210                1215                1220

AAC AAC ATA CTG TCA ATG CCA GAG AAG GCC AAG AAA GCG TTT GAC AAC     3750
Asn Asn Ile Leu Ser Met Pro Glu Lys Ala Lys Lys Ala Phe Asp Asn
    1225                1230                1235

CCT GAC TAC TGG AAC CAC AGC CTG CCA CCT CGG AGC ACC CTT CAG CAC     3798
Pro Asp Tyr Trp Asn His Ser Leu Pro Pro Arg Ser Thr Leu Gln His
1240                1245                1250                1255

CCA GAC TAC CTG CAG GAG TAC AGC ACA AAA TAT TTT TAT AAA CAG AAT     3846
Pro Asp Tyr Leu Gln Glu Tyr Ser Thr Lys Tyr Phe Tyr Lys Gln Asn
                1260                1265                1270

GGG CGG ATC CGG CCT ATT GTG GCA GAG AAT CCT GAA TAC CTC TCT GAG     3894
Gly Arg Ile Arg Pro Ile Val Ala Glu Asn Pro Glu Tyr Leu Ser Glu
            1275                1280                1285

TTC TCC CTG AAG CCA GGC ACT GTG CTG CCG CCT CCA CCT TAC AGA CAC     3942
Phe Ser Leu Lys Pro Gly Thr Val Leu Pro Pro Pro Pro Tyr Arg His
        1290                1295                1300

CGG AAT ACT GTG GTG TAAGCTCAGT TGTGGTTTTT TAGGTGGAGA GACACACCTG     3997
Arg Asn Thr Val Val
    1305

CTCCAATTTC CCCACCCCCC TCTCTTTCTC TGGTGGTCTT CCTTCTACCC CAAGGCCAGT   4057

AGTTTTGACA CTTCCCAGTG GAAGATACAG AGATGCAATG ATAGTTATGT GCTTACCTAA   4117

CTTGAACATT AGAGGGAAAG ACTGAAAGAG AAAGATAGGA GGAACCACAA TGTTTCTTCA   4177
```

```
TTTCTCTGCA TGGGTTGGTC AGGAGAATGA AACAGCTAGA GAAGGACCAG AAAATGTAAG    4237
GCAATGCTGC CTACTATCAA ACTAGCTGTC ACTTTTTTTC TTTTTCTTTT TCTTTCTTTG    4297
TTTCTTTCTT CCTCTTCTTT TTTTTTTTTT TTTTAAAGCA GATGGTTGAA ACACCCATGC    4357
TATCTGTTCC TATCTGCAGG AACTGATGTG TGCATATTTA GCATCCCTGG AAATCATAAT    4417
AAAGTTTCCA TTAGAACAAA AGAATAACAT TTTCTATAAC ATATGATAGT GTCTGAAATT    4477
GAGAATCCAG TTTCTTTCCC CAGCAGTTTC TGTCCTAGCA AGTAAGAATG GCCAACTCAA    4537
CTTTCATAAT TTAAAAATCT CCATTAAAGT TATAACTAGT AATTATGTTT TCAACACTTT    4597
TTGGTTTTTT TCATTTTGTT TTGCTCTGAC CGATTCCTTT ATATTTGCTC CCCTATTTTT    4657
GGCTTTAATT TCTAATTGCA AAGATGTTTA CATCAAAGCT TCTTCACAGA ATTTAAGCAA    4717
GAAATATTTT AATATAGTGA AATGGCCACT ACTTTAAGTA TACAATCTTT AAAATAAGAA    4777
AGGGAGGCTA ATATTTTTCA TGCTATCAAA TTATCTTCAC CCTCATCCTT TACATTTTTC    4837
AACATTTTTT TTTCTCCATA AATGACACTA CTTGATAGGC CGTTGGTTGT CTGAAGAGTA    4897
GAAGGGAAAC TAAGAGACAG TTCTCTGTGG TTCAGGAAAA CTACTGATAC TTTCAGGGGT    4957
GGCCCAATGA GGGAATCCAT TGAACTGGAA GAAACACACT GGATTGGGTA TGTCTACCTG    5017
GCAGATACTC AGAAATGTAG TTTGCACTTA AGCTGTAATT TTATTTGTTC TTTTTCTGAA    5077
CTCCATTTTG GATTTTGAAT CAAGCAATAT GGAAGCAACC AGCAAATTAA CTAATTTAAG    5137
TACATTTTTA AAAAAAGAGC TAAGATAAAG ACTGTGGAAA TGCCAAACCA AGCAAATTAG    5197
GAACCTTGCA ACGGTATCCA GGGACTATGA TGAGAGGCCA GCACATTATC TTCATATGTC    5257
ACCTTTGCTA CGCAAGGAAA TTTGTTCAGT TCGTATACTT CGTAAGAAGG AATGCGAGTA    5317
AGGATTGGCT TGAATTCCAT GGAATTTCTA GTATGAGACT ATTTATATGA AGTAGAAGGT    5377
AACTCTTTGC ACATAAATTG GTATAATAAA AAGAAAAACA CAAACATTCA AAGCTTAGGG    5437
ATAGGTCCTT GGGTCAAAAG TTGTAAATAA ATGTGAAACA TCTTCTCAAA AAAAAAAAAA    5497
AAAA                                                                 5501
```

(2) INFORMATION FOR SEQ ID NO:2:

(i) SEQUENCE CHARACTERISTICS:
- (A) LENGTH: 1308 amino acids
- (B) TYPE: amino acid
- (D) TOPOLOGY: linear

(ii) MOLECULE TYPE: protein

(xi) SEQUENCE DESCRIPTION: SEQ ID NO:2:

Met Lys Pro Ala Thr Gly Leu Trp Val Trp Val Ser Leu Leu Val Ala
1 5 10 15

Ala Gly Thr Val Gln Pro Ser Asp Ser Gln Ser Val Cys Ala Gly Thr
20 25 30

Glu Asn Lys Leu Ser Ser Leu Ser Asp Leu Glu Gln Gln Tyr Arg Ala
35 40 45

Leu Arg Lys Tyr Tyr Glu Asn Cys Glu Val Val Met Gly Asn Leu Glu
50 55 60

Ile Thr Ser Ile Glu His Asn Arg Asp Leu Ser Phe Leu Arg Ser Val
65 70 75 80

Arg Glu Val Thr Gly Tyr Val Leu Val Ala Leu Asn Gln Phe Arg Tyr
85 90 95

Leu Pro Leu Glu Asn Leu Arg Ile Ile Arg Gly Thr Lys Leu Tyr Glu
100 105 110

Asp Arg Tyr Ala Leu Ala Ile Phe Leu Asn Tyr Arg Lys Asp Gly Asn
115 120 125

Phe Gly Leu Gln Glu Leu Gly Leu Lys Asn Leu Thr Glu Ile Leu Asn
130 135 140

Gly Gly Val Tyr Val Asp Gln Asn Lys Phe Leu Cys Tyr Ala Asp Thr
145 150 155 160

Ile His Trp Gln Asp Ile Val Arg Asn Pro Trp Pro Ser Asn Leu Thr
165 170 175

Leu Val Ser Thr Asn Gly Ser Ser Gly Cys Gly Arg Cys His Lys Ser
180 185 190

Cys Thr Gly Arg Cys Trp Gly Pro Thr Glu Asn His Cys Gln Thr Leu
195 200 205

Thr Arg Thr Val Cys Ala Glu Gln Cys Asp Gly Arg Cys Tyr Gly Pro
210 215 220

Tyr Val Ser Asp Cys Cys His Arg Glu Cys Ala Gly Gly Cys Ser Gly
225 230 235 240

Pro Lys Asp Thr Asp Cys Phe Ala Cys Met Asn Phe Asn Asp Ser Gly
245 250 255

Ala Cys Val Thr Gln Cys Pro Gln Thr Phe Val Tyr Asn Pro Thr Thr
260 265 270

Phe Gln Leu Glu His Asn Phe Asn Ala Lys Tyr Thr Tyr Gly Ala Phe
275 280 285

Cys Val Lys Lys Cys Pro His Asn Phe Val Val Asp Ser Ser Ser Cys
290 295 300

Val Arg Ala Cys Pro Ser Ser Lys Met Glu Val Glu Glu Asn Gly Ile
305 310 315 320

Lys Met Cys Lys Pro Cys Thr Asp Ile Cys Pro Lys Ala Cys Asp Gly
325 330 335

Ile Gly Thr Gly Ser Leu Met Ser Ala Gln Thr Val Asp Ser Ser Asn
340 345 350

Ile Asp Lys Phe Ile Asn Cys Thr Lys Ile Asn Gly Asn Leu Ile Phe
355 360 365

Leu Val Thr Gly Ile His Gly Asp Pro Tyr Asn Ala Ile Glu Ala Ile
370 375 380

Asp Pro Glu Lys Leu Asn Val Phe Arg Thr Val Arg Glu Ile Thr Gly
385 390 395 400

Phe Leu Asn Ile Gln Ser Trp Pro Pro Asn Met Thr Asp Phe Ser Val
405 410 415

Phe Ser Asn Leu Val Thr Ile Gly Gly Arg Val Leu Tyr Ser Gly Leu
420 425 430

Ser Leu Leu Ile Leu Lys Gln Gln Gly Ile Thr Ser Leu Gln Phe Gln
435 440 445

Ser Leu Lys Glu Ile Ser Ala Gly Asn Ile Tyr Ile Thr Asp Asn Ser
450 455 460

Asn Leu Cys Tyr Tyr His Thr Ile Asn Trp Thr Thr Leu Phe Ser Thr
465 470 475 480

Ile Asn Gln Arg Ile Val Ile Arg Asp Asn Arg Lys Ala Glu Asn Cys
485 490 495

Thr Ala Glu Gly Met Val Cys Asn His Leu Cys Ser Ser Asp Gly Cys
500 505 510

Trp Gly Pro Gly Pro Asp Gln Cys Leu Ser Cys Arg Arg Phe Ser Arg
515 520 525

Gly Arg Ile Cys Ile Glu Ser Cys Asn Leu Tyr Asp Gly Glu Phe Arg
530 535 540

Glu Phe Glu Asn Gly Ser Ile Cys Val Glu Cys Asp Pro Gln Cys Glu
545 550 555 560

Lys Met Glu Asp Gly Leu Leu Thr Cys His Gly Pro Gly Pro Asp Asn
565 570 575

Cys Thr Lys Cys Ser His Phe Lys Asp Gly Pro Asn Cys Val Glu Lys
580 585 590

Cys Pro Asp Gly Leu Gln Gly Ala Asn Ser Phe Ile Phe Lys Tyr Ala
595 600 605

```
Asp Pro Asp Arg Glu Cys His Pro Cys His Pro Asn Cys Thr Gln Gly
    610                 615                 620

Cys Asn Gly Pro Thr Ser His Asp Cys Ile Tyr Tyr Pro Trp Thr Gly
625                 630                 635                 640

His Ser Thr Leu Pro Gln His Ala Arg Thr Pro Leu Ile Ala Ala Gly
                645                 650                 655

Val Ile Gly Gly Leu Phe Ile Leu Val Ile Val Gly Leu Thr Phe Ala
            660                 665                 670

Val Tyr Val Arg Arg Lys Ser Ile Lys Lys Lys Arg Ala Leu Arg Arg
        675                 680                 685

Phe Leu Glu Thr Glu Leu Val Glu Pro Leu Thr Pro Ser Gly Thr Ala
    690                 695                 700

Pro Asn Gln Ala Gln Leu Arg Ile Leu Lys Glu Thr Glu Leu Lys Arg
705                 710                 715                 720

Val Lys Val Leu Gly Ser Gly Ala Phe Gly Thr Val Tyr Lys Gly Ile
                725                 730                 735

Trp Val Pro Glu Gly Glu Thr Val Lys Ile Pro Val Ala Ile Lys Ile
            740                 745                 750

Leu Asn Glu Thr Thr Gly Pro Lys Ala Asn Val Glu Phe Met Asp Glu
        755                 760                 765

Ala Leu Ile Met Ala Ser Met Asp His Pro His Leu Val Arg Leu Leu
    770                 775                 780

Gly Val Cys Leu Ser Pro Thr Ile Gln Leu Val Thr Gln Leu Met Pro
785                 790                 795                 800

His Gly Cys Leu Leu Glu Tyr Val His Glu His Lys Asp Asn Ile Gly
                805                 810                 815

Ser Gln Leu Leu Leu Asn Trp Cys Val Gln Ile Ala Lys Gly Met Met
            820                 825                 830

Tyr Leu Glu Glu Arg Arg Leu Val His Arg Asp Leu Ala Ala Arg Asn
        835                 840                 845

Val Leu Val Lys Ser Pro Asn His Val Lys Ile Thr Asp Phe Gly Leu
    850                 855                 860

Ala Arg Leu Leu Glu Gly Asp Glu Lys Glu Tyr Asn Ala Asp Gly Gly
865                 870                 875                 880

Lys Met Pro Ile Lys Trp Met Ala Leu Glu Cys Ile His Tyr Arg Lys
                885                 890                 895

Phe Thr His Gln Ser Asp Val Trp Ser Tyr Gly Val Thr Ile Trp Glu
            900                 905                 910
```

```
Leu Met Thr Phe Gly Gly Lys Pro Tyr Asp Gly Ile Pro Thr Arg Glu
        915                 920                     925

Ile Pro Asp Leu Leu Glu Lys Gly Glu Arg Leu Pro Gln Pro Pro Ile
    930                 935                     940

Cys Thr Ile Asp Val Tyr Met Val Met Val Lys Cys Trp Met Ile Asp
945                 950                     955                 960

Ala Asp Ser Arg Pro Lys Phe Lys Glu Leu Ala Ala Glu Phe Ser Arg
                965                     970                 975

Met Ala Arg Asp Pro Gln Arg Tyr Leu Val Ile Gln Gly Asp Asp Arg
            980                 985                     990

Met Lys Leu Pro Ser Pro Asn Asp Ser Lys Phe Phe Gln Asn Leu Leu
        995                 1000                    1005

Asp Glu Glu Asp Leu Glu Asp Met Met Asp Ala Glu Glu Tyr Leu Val
    1010                1015                    1020

Pro Gln Ala Phe Asn Ile Pro Pro Pro Ile Tyr Thr Ser Arg Ala Arg
1025                1030                    1035                1040

Ile Asp Ser Asn Arg Ser Glu Ile Gly His Ser Pro Pro Pro Ala Tyr
                1045                    1050                1055

Thr Pro Met Ser Gly Asn Gln Phe Val Tyr Arg Asp Gly Gly Phe Ala
            1060                1065                    1070

Ala Glu Gln Gly Val Ser Val Pro Tyr Arg Ala Pro Thr Ser Thr Ile
        1075                1080                    1085

Pro Glu Ala Pro Val Ala Gln Gly Ala Thr Ala Glu Ile Phe Asp Asp
    1090                1095                    1100

Ser Cys Cys Asn Gly Thr Leu Arg Lys Pro Val Ala Pro His Val Gln
1105                1110                    1115                1120

Glu Asp Ser Ser Thr Gln Arg Tyr Ser Ala Asp Pro Thr Val Phe Ala
                1125                    1130                1135

Pro Glu Arg Ser Pro Arg Gly Glu Leu Asp Glu Glu Gly Tyr Met Thr
            1140                1145                    1150

Pro Met Arg Asp Lys Pro Lys Gln Glu Tyr Leu Asn Pro Val Glu Glu
        1155                1160                    1165

Asn Pro Phe Val Ser Arg Arg Lys Asn Gly Asp Leu Gln Ala Leu Asp
    1170                1175                    1180

Asn Pro Glu Tyr His Asn Ala Ser Asn Gly Pro Pro Lys Ala Glu Asp
1185                1190                    1195                1200

Glu Tyr Val Asn Glu Pro Leu Tyr Leu Asn Thr Phe Ala Asn Thr Leu
                1205                    1210                1215
```

```
Gly Lys Ala Glu Tyr Leu Lys Asn Asn Ile Leu Ser Met Pro Glu Lys
            1220                1225                1230

Ala Lys Lys Ala Phe Asp Asn Pro Asp Tyr Trp Asn His Ser Leu Pro
        1235                1240                1245

Pro Arg Ser Thr Leu Gln His Pro Asp Tyr Leu Gln Glu Tyr Ser Thr
    1250                1255                1260

Lys Tyr Phe Tyr Lys Gln Asn Gly Arg Ile Arg Pro Ile Val Ala Glu
1265                1270                1275                1280

Asn Pro Glu Tyr Leu Ser Glu Phe Ser Leu Lys Pro Gly Thr Val Leu
                1285                1290                1295

Pro Pro Pro Pro Tyr Arg His Arg Asn Thr Val Val
            1300                1305
```

(2) INFORMATION FOR SEQ ID NO:3:

(i) SEQUENCE CHARACTERISTICS:
(A) LENGTH: 5555 base pairs
(B) TYPE: nucleic acid
(C) STRANDEDNESS: single
(D) TOPOLOGY: unknown

(ii) MOLECULE TYPE: DNA (genomic)

(ix) FEATURE:
(A) NAME/KEY: CDS
(B) LOCATION: 34..3210

(xi) SEQUENCE DESCRIPTION: SEQ ID NO:3:

```
AATTGTCAGC ACGGGATCTG AGACTTCCAA AAA ATG AAG CCG GCG ACA GGA CTT      54
                                     Met Lys Pro Ala Thr Gly Leu
                                       1               5

TGG GTC TGG GTG AGC CTT CTC GTG GCG GCG GGG ACC GTC CAG CCC AGC      102
Trp Val Trp Val Ser Leu Leu Val Ala Ala Gly Thr Val Gln Pro Ser
         10                  15                  20

GAT TCT CAG TCA GTG TGT GCA GGA ACG GAG AAT AAA CTG AGC TCT CTC      150
Asp Ser Gln Ser Val Cys Ala Gly Thr Glu Asn Lys Leu Ser Ser Leu
     25                  30                  35

TCT GAC CTG GAA CAG CAG TAC CGA GCC TTG CGC AAG TAC TAT GAA AAC      198
Ser Asp Leu Glu Gln Gln Tyr Arg Ala Leu Arg Lys Tyr Tyr Glu Asn
 40                  45                  50                  55

TGT GAG GTT GTC ATG GGC AAC CTG GAG ATA ACC AGC ATT GAG CAC AAC      246
Cys Glu Val Val Met Gly Asn Leu Glu Ile Thr Ser Ile Glu His Asn
                 60                  65                  70
```

```
CGG GAC CTC TCC TTC CTG CGG TCT GTT CGA GAA GTC ACA GGC TAC GTG      294
Arg Asp Leu Ser Phe Leu Arg Ser Val Arg Glu Val Thr Gly Tyr Val
            75                  80                  85

TTA GTG GCT CTT AAT CAG TTT CGT TAC CTG CCT CTG GAG AAT TTA CGC      342
Leu Val Ala Leu Asn Gln Phe Arg Tyr Leu Pro Leu Glu Asn Leu Arg
        90                  95                  100

ATT ATT CGT GGG ACA AAA CTT TAT GAG GAT CGA TAT GCC TTG GCA ATA      390
Ile Ile Arg Gly Thr Lys Leu Tyr Glu Asp Arg Tyr Ala Leu Ala Ile
    105                 110                 115

TTT TTA AAC TAC AGA AAA GAT GGA AAC TTT GGA CTT CAA GAA CTT GGA      438
Phe Leu Asn Tyr Arg Lys Asp Gly Asn Phe Gly Leu Gln Glu Leu Gly
120                 125                 130                 135

TTA AAG AAC TTG ACA GAA ATC CTA AAT GGT GGA GTC TAT GTA GAC CAG      486
Leu Lys Asn Leu Thr Glu Ile Leu Asn Gly Gly Val Tyr Val Asp Gln
                140                 145                 150

AAC AAA TTC CTT TGT TAT GCA GAC ACC ATT CAT TGG CAA GAT ATT GTT      534
Asn Lys Phe Leu Cys Tyr Ala Asp Thr Ile His Trp Gln Asp Ile Val
            155                 160                 165

CGG AAC CCA TGG CCT TCC AAC TTG ACT CTT GTG TCA ACA AAT GGT AGT      582
Arg Asn Pro Trp Pro Ser Asn Leu Thr Leu Val Ser Thr Asn Gly Ser
        170                 175                 180

TCA GGA TGT GGA CGT TGC CAT AAG TCC TGT ACT GGC CGT TGC TGG GGA      630
Ser Gly Cys Gly Arg Cys His Lys Ser Cys Thr Gly Arg Cys Trp Gly
    185                 190                 195

CCC ACA GAA AAT CAT TGC CAG ACT TTG ACA AGG ACG GTG TGT GCA GAA      678
Pro Thr Glu Asn His Cys Gln Thr Leu Thr Arg Thr Val Cys Ala Glu
200                 205                 210                 215

CAA TGT GAC GGC AGA TGC TAC GGA CCT TAC GTC AGT GAC TGC TGC CAT      726
Gln Cys Asp Gly Arg Cys Tyr Gly Pro Tyr Val Ser Asp Cys Cys His
                220                 225                 230

CGA GAA TGT GCT GGA GGC TGC TCA GGA CCT AAG GAC ACA GAC TGC TTT      774
Arg Glu Cys Ala Gly Gly Cys Ser Gly Pro Lys Asp Thr Asp Cys Phe
            235                 240                 245

GCC TGC ATG AAT TTC AAT GAC AGT GGA GCA TGT GTT ACT CAG TGT CCC      822
Ala Cys Met Asn Phe Asn Asp Ser Gly Ala Cys Val Thr Gln Cys Pro
        250                 255                 260

CAA ACC TTT GTC TAC AAT CCA ACC ACC TTT CAA CTG GAG CAC AAT TTC      870
Gln Thr Phe Val Tyr Asn Pro Thr Thr Phe Gln Leu Glu His Asn Phe
    265                 270                 275

AAT GCA AAG TAC ACA TAT GGA GCA TTC TGT GTC AAG AAA TGT CCA CAT      918
Asn Ala Lys Tyr Thr Tyr Gly Ala Phe Cys Val Lys Lys Cys Pro His
280                 285                 290                 295
```

```
AAC TTT GTG GTA GAT TCC AGT TCT TGT GTG CGT GCC TGC CCT AGT TCC     966
Asn Phe Val Val Asp Ser Ser Ser Cys Val Arg Ala Cys Pro Ser Ser
                300                 305                 310

AAG ATG GAA GTA GAA GAA AAT GGG ATT AAA ATG TGT AAA CCT TGC ACT    1014
Lys Met Glu Val Glu Glu Asn Gly Ile Lys Met Cys Lys Pro Cys Thr
            315                 320                 325

GAC ATT TGC CCA AAA GCT TGT GAT GGC ATT GGC ACA GGA TCA TTG ATG    1062
Asp Ile Cys Pro Lys Ala Cys Asp Gly Ile Gly Thr Gly Ser Leu Met
        330                 335                 340

TCA GCT CAG ACT GTG GAT TCC AGT AAC ATT GAC AAA TTC ATA AAC TGT    1110
Ser Ala Gln Thr Val Asp Ser Ser Asn Ile Asp Lys Phe Ile Asn Cys
    345                 350                 355

ACC AAG ATC AAT GGG AAT TTG ATC TTT CTA GTC ACT GGT ATT CAT GGG    1158
Thr Lys Ile Asn Gly Asn Leu Ile Phe Leu Val Thr Gly Ile His Gly
360                 365                 370                 375

GAC CCT TAC AAT GCA ATT GAA GCC ATA GAC CCA GAG AAA CTG AAC GTC    1206
Asp Pro Tyr Asn Ala Ile Glu Ala Ile Asp Pro Glu Lys Leu Asn Val
                380                 385                 390

TTT CGG ACA GTC AGA GAG ATA ACA GGT TTC CTG AAC ATA CAG TCA TGG    1254
Phe Arg Thr Val Arg Glu Ile Thr Gly Phe Leu Asn Ile Gln Ser Trp
            395                 400                 405

CCA CCA AAC ATG ACT GAC TTC AGT GTT TTT TCT AAC CTG GTG ACC ATT    1302
Pro Pro Asn Met Thr Asp Phe Ser Val Phe Ser Asn Leu Val Thr Ile
        410                 415                 420

GGT GGA AGA GTA CTC TAT AGT GGC CTG TCC TTG CTT ATC CTC AAG CAA    1350
Gly Gly Arg Val Leu Tyr Ser Gly Leu Ser Leu Leu Ile Leu Lys Gln
    425                 430                 435

CAG GGC ATC ACC TCT CTA CAG TTC CAG TCC CTG AAG GAA ATC AGC GCA    1398
Gln Gly Ile Thr Ser Leu Gln Phe Gln Ser Leu Lys Glu Ile Ser Ala
440                 445                 450                 455

GGA AAC ATC TAT ATT ACT GAC AAC AGC AAC CTG TGT TAT TAT CAT ACC    1446
Gly Asn Ile Tyr Ile Thr Asp Asn Ser Asn Leu Cys Tyr Tyr His Thr
                460                 465                 470

ATT AAC TGG ACA ACA CTC TTC AGC ACA ATC AAC CAG AGA ATA GTA ATC    1494
Ile Asn Trp Thr Thr Leu Phe Ser Thr Ile Asn Gln Arg Ile Val Ile
            475                 480                 485

CGG GAC AAC AGA AAA GCT GAA AAT TGT ACT GCT GAA GGA ATG GTG TGC    1542
Arg Asp Asn Arg Lys Ala Glu Asn Cys Thr Ala Glu Gly Met Val Cys
        490                 495                 500

AAC CAT CTG TGT TCC AGT GAT GGC TGT TGG GGA CCT GGG CCA GAC CAA    1590
Asn His Leu Cys Ser Ser Asp Gly Cys Trp Gly Pro Gly Pro Asp Gln
    505                 510                 515
```

```
TGT CTG TCG TGT CGC CGC TTC AGT AGA GGA AGG ATC TGC ATA GAG TCT      1638
Cys Leu Ser Cys Arg Arg Phe Ser Arg Gly Arg Ile Cys Ile Glu Ser
520                 525                 530                 535

TGT AAC CTC TAT GAT GGT GAA TTT CGG GAG TTT GAG AAT GGC TCC ATC      1686
Cys Asn Leu Tyr Asp Gly Glu Phe Arg Glu Phe Glu Asn Gly Ser Ile
                540                 545                 550

TGT GTG GAG TGT GAC CCC CAG TGT GAG AAG ATG GAA GAT GGC CTC CTC      1734
Cys Val Glu Cys Asp Pro Gln Cys Glu Lys Met Glu Asp Gly Leu Leu
            555                 560                 565

ACA TGC CAT GGA CCG GGT CCT GAC AAC TGT ACA AAG TGC TCT CAT TTT      1782
Thr Cys His Gly Pro Gly Pro Asp Asn Cys Thr Lys Cys Ser His Phe
        570                 575                 580

AAA GAT GGC CCA AAC TGT GTG GAA AAA TGT CCA GAT GGC TTA CAG GGG      1830
Lys Asp Gly Pro Asn Cys Val Glu Lys Cys Pro Asp Gly Leu Gln Gly
    585                 590                 595

GCA AAC AGT TTC ATT TTC AAG TAT GCT GAT CCA GAT CGG GAG TGC CAC      1878
Ala Asn Ser Phe Ile Phe Lys Tyr Ala Asp Pro Asp Arg Glu Cys His
600                 605                 610                 615

CCA TGC CAT CCA AAC TGC ACC CAA GGG TGT AAC GGT CCC ACT AGT CAT      1926
Pro Cys His Pro Asn Cys Thr Gln Gly Cys Asn Gly Pro Thr Ser His
                620                 625                 630

GAC TGC ATT TAC TAC CCA TGG ACG GGC CAT TCC ACT TTA CCA CAA CAT      1974
Asp Cys Ile Tyr Tyr Pro Trp Thr Gly His Ser Thr Leu Pro Gln His
            635                 640                 645

GCT AGA ACT CCC CTG ATT GCA GCT GGA GTA ATT GGT GGG CTC TTC ATT      2022
Ala Arg Thr Pro Leu Ile Ala Ala Gly Val Ile Gly Gly Leu Phe Ile
        650                 655                 660

CTG GTC ATT GTG GGT CTG ACA TTT GCT GTT TAT GTT AGA AGG AAG AGC      2070
Leu Val Ile Val Gly Leu Thr Phe Ala Val Tyr Val Arg Arg Lys Ser
    665                 670                 675

ATC AAA AAG AAA AGA GCC TTG AGA AGA TTC TTG GAA ACA GAG TTG GTG      2118
Ile Lys Lys Lys Arg Ala Leu Arg Arg Phe Leu Glu Thr Glu Leu Val
680                 685                 690                 695

GAA CCA TTA ACT CCC AGT GGC ACA GCA CCC AAT CAA GCT CAA CTT CGT      2166
Glu Pro Leu Thr Pro Ser Gly Thr Ala Pro Asn Gln Ala Gln Leu Arg
                700                 705                 710

ATT TTG AAA GAA ACT GAG CTG AAG AGG GTA AAA GTC CTT GGC TCA GGT      2214
Ile Leu Lys Glu Thr Glu Leu Lys Arg Val Lys Val Leu Gly Ser Gly
            715                 720                 725

GCT TTT GGA ACG GTT TAT AAA GGT ATT TGG GTA CCT GAA GGA GAA ACT      2262
Ala Phe Gly Thr Val Tyr Lys Gly Ile Trp Val Pro Glu Gly Glu Thr
        730                 735                 740
```

```
GTG AAG ATT CCT GTG GCT ATT AAG ATT CTT AAT GAG ACA ACT GGT CCC      2310
Val Lys Ile Pro Val Ala Ile Lys Ile Leu Asn Glu Thr Thr Gly Pro
    745                 750                 755

AAG GCA AAT GTG GAG TTC ATG GAT GAA GCT CTG ATC ATG GCA AGT ATG      2358
Lys Ala Asn Val Glu Phe Met Asp Glu Ala Leu Ile Met Ala Ser Met
760                 765                 770                 775

GAT CAT CCA CAC CTA GTC CGG TTG CTG GGT GTG TGT CTG AGC CCA ACC      2406
Asp His Pro His Leu Val Arg Leu Leu Gly Val Cys Leu Ser Pro Thr
                780                 785                 790

ATC CAG CTG GTT ACT CAA CTT ATG CCC CAT GGC TGC CTG TTG GAG TAT      2454
Ile Gln Leu Val Thr Gln Leu Met Pro His Gly Cys Leu Leu Glu Tyr
            795                 800                 805

GTC CAC GAG CAC AAG GAT AAC ATT GGA TCA CAA CTG CTG CTT AAC TGG      2502
Val His Glu His Lys Asp Asn Ile Gly Ser Gln Leu Leu Leu Asn Trp
        810                 815                 820

TGT GTC CAG ATA GCT AAG GGA ATG ATG TAC CTG GAA GAA AGA CGA CTC      2550
Cys Val Gln Ile Ala Lys Gly Met Met Tyr Leu Glu Glu Arg Arg Leu
    825                 830                 835

GTT CAT CGG GAT TTG GCA GCC CGT AAT GTC TTA GTG AAA TCT CCA AAC      2598
Val His Arg Asp Leu Ala Ala Arg Asn Val Leu Val Lys Ser Pro Asn
840                 845                 850                 855

CAT GTG AAA ATC ACA GAT TTT GGG CTA GCC AGA CTC TTG GAA GGA GAT      2646
His Val Lys Ile Thr Asp Phe Gly Leu Ala Arg Leu Leu Glu Gly Asp
                860                 865                 870

GAA AAA GAG TAC AAT GCT GAT GGA GGA AAG ATG CCA ATT AAA TGG ATG      2694
Glu Lys Glu Tyr Asn Ala Asp Gly Gly Lys Met Pro Ile Lys Trp Met
            875                 880                 885

GCT CTG GAG TGT ATA CAT TAC AGG AAA TTC ACC CAT CAG AGT GAC GTT      2742
Ala Leu Glu Cys Ile His Tyr Arg Lys Phe Thr His Gln Ser Asp Val
        890                 895                 900

TGG AGC TAT GGA GTT ACT ATA TGG GAA CTG ATG ACC TTT GGA GGA AAA      2790
Trp Ser Tyr Gly Val Thr Ile Trp Glu Leu Met Thr Phe Gly Gly Lys
    905                 910                 915

CCC TAT GAT GGA ATT CCA ACG CGA GAA ATC CCT GAT TTA TTA GAG AAA      2838
Pro Tyr Asp Gly Ile Pro Thr Arg Glu Ile Pro Asp Leu Leu Glu Lys
920                 925                 930                 935

GGA GAA CGT TTG CCT CAG CCT CCC ATC TGC ACT ATT GAC GTT TAC ATG      2886
Gly Glu Arg Leu Pro Gln Pro Pro Ile Cys Thr Ile Asp Val Tyr Met
                940                 945                 950

GTC ATG GTC AAA TGT TGG ATG ATT GAT GCT GAC AGT AGA CCT AAA TTT      2934
Val Met Val Lys Cys Trp Met Ile Asp Ala Asp Ser Arg Pro Lys Phe
            955                 960                 965
```

AAG GAA CTG GCT GCT GAG TTT TCA AGG ATG GCT CGA GAC CCT CAA AGA 2982
Lys Glu Leu Ala Ala Glu Phe Ser Arg Met Ala Arg Asp Pro Gln Arg
970 975 980

TAC CTA GTT ATT CAG GGT GAT GAT CGT ATG AAG CTT CCC AGT CCA AAT 3030
Tyr Leu Val Ile Gln Gly Asp Asp Arg Met Lys Leu Pro Ser Pro Asn
985 990 995

GAC AGC AAG TTC TTT CAG AAT CTC TTG GAT GAA GAG GAT TTG GAA GAT 3078
Asp Ser Lys Phe Phe Gln Asn Leu Leu Asp Glu Glu Asp Leu Glu Asp
1000 1005 1010 1015

ATG ATG GAT GCT GAG GAG TAC TTG GTC CCT CAG GCT TTC AAC ATC CCA 3126
Met Met Asp Ala Glu Glu Tyr Leu Val Pro Gln Ala Phe Asn Ile Pro
1020 1025 1030

CCT CCC ATC TAT ACT TCC AGA GCA AGA ATT GAC TCG AAT AGG AGT GTA 3174
Pro Pro Ile Tyr Thr Ser Arg Ala Arg Ile Asp Ser Asn Arg Ser Val
1035 1040 1045

AGA AAT AAT TAT ATA CAC ATA TCA TAT TCT TTC TGAGATATAA AATCATGTAA 3227
Arg Asn Asn Tyr Ile His Ile Ser Tyr Ser Phe
1050 1055

TAGTTCATAA GCACTAACAT TTCAAAATAA TTATATAGCT CAAATCAATG TGATGCCTAG 3287

ATTAAAAATA TACCATACCC ACAAAAGATG TGCCAATCTT GCTATATGTA GTTAATTTTG 3347

GAAGACAAGC ATGGACAATA CAACATGTAC TCTGAAATAC CTTCAAGATT TCAGAAGCAA 3407

AACATTTTCC TCATCTTAAT TTATTTAAAA CAAATCTTAA CTTTAAAAAA CAATTCCAAC 3467

TAATAAAACC ATTATGTGTA TATAAATAAA TGAAAATTCC TACCAAGTAG GCTTTCTACT 3527

TTTCTTTCTT AAAAAGATAT TATGATATAT TAGTCAAGAA GTAATACAAG TATAAATCTC 3587

TTTCACTTAT TTAAGAAAAA TTAAATATTT TCTGTCAAGT TGAAGTAGAA ACACAGAAAA 3647

CCGTGCAGTC CTTTGAACCT AATCACATCG AAAAGGCTGC TGAGAAGTAG ATTTTTGTTT 3707

TTAAGAAGTA GATTTAAGTT TTGAAGGAAG TTTCTGAAAA CACTTTACAT TTTAAATGTT 3767

AAACCTACTC TATATGAATT CCATTCTTTC TTTGAAAGCT GTCAAATCCA TGCATTTATT 3827

TTTATAAATT CATTCCTCAT ACATTCAACA TATATTGAGT ACCACTGTAT GTGAAGCATT 3887

AGTATACATT TAAGACTCAA AGAATTTTGA TACAACTTCT GCTTTCAAGA AGTGAAAACC 3947

TTAATCAAAG AATCATACAG ATAGAGGGAC TGCATAGTAA GTGCTGTAAT CCAGTATTCA 4007

CTGACCAGTA CGGAGCATGA AGAAGTAGTA AATTTGTGTC TGTAATCAGT TTCTTCCATT 4067

GATAAGATAT AAACATGATG CTTAATTTTT TCTAGAAGAT AATTCTTTTC TCTTAATCTA 4127

AGAACATTAT CATAGCTAGT AGAACCGACA GCATCCGATT TCTCTTGACC ATAGCCATAA 4187

GAATATCTTC AACTTGCTGC TCATTATCTA ACAAACATAA TTTTCTTTAT TTCATATTGA 4247

TTGTAATAAG TAATATCCCC CTGGAAGTTT ACTATTCAAC ACATATATGT TAACCTCCTT 4307
AATTCCTTAA ACAAACTTCA TGAGGTTCTA TTATTATCAT CCCCTTCTTT CAAAGGAAGA 4367
AACTTGCCAC AGAGAAGTCA GGTGATATGA CTGGTGTCAC ACAGCTAGTC AGTGGAAGAG 4427
AGGAATAAGT AATCTAGATA TCTGCCTACT ACACTGTAGG TTTGCTTCAA AGTTACTGAA 4487
GYCATGTTAT TTCCATGATG TGATTAGAGT CTGGGACTTG TCTTGTTTGG GAAATTTCCC 4547
AGGTGGTTTT CTTATAAAAT GCATCTCAAA TCTGCTCTAC ACCTTTTACT CATCTACCTC 4607
CATTTAGAAG ATCTGATATG GAAAGAGACA AAGATGGAGA CCTCAATTAT TTTTTCTTTT 4667
CTGTTAAAAA TATTATAGTA CAACTGAAAC TTATCACATG CCAATGGGGA ATAGATAACT 4727
AAAAGTTTAA AATTAGATCA ATGGATAGGT AAATGAATAA TCNTTCTTTT GCTTGTGAGA 4787
GGGGAAGGAA AAGCGGTTAA GGTGGTATAA AGGAGGCTCC TCTGTACACT TGCAAAATGA 4847
TCAAATTATA TACCCTTGTA TTTATAATTT TAAGTGACAA ATTCATTACT TCTGGTTACA 4907
ACAGTGAAAT TTAAAAAAAA ATAGTTTTTC TTTCTTAGCT TGCAATGCTA TAAATCTTTT 4967
TCTTTTTATA AGAATTCTTA CATTTCAGCT TTTTGTTCAT TTTAATTTAT AATTCTCAGT 5027
GCAAGAAATT CTTAATAAAG GTTTGAGCTA GCTAGATGGA ATTATTGAGA CAAAGTCTAA 5087
ATCACCCGTG GACTTATTTG ACCTTTAGCC ATCATTTCTT ATTCCACATT ATAAAACAAT 5147
GTTACCTGTA GATTTCTTTT TACTTTTTCA GTCCTTGGAA AAGAAATGGT GATTAAATAT 5207
CATTATATCA TTTTATGTTC AGGCATTTAA AAAGCTTTAT TTGTCATCTA TATTGTCCTA 5267
ATAGTTTTCA GTCTGGCTTT ACGTAACTTT TACGGAAATT TCTAACATGT ACAAATGCCA 5327
TGTTCCTCCT TTCTTTCCTA CATGGCTGAA TTAGAAAACA AATTACTTCC ATTTTAAGTT 5387
TGGCTAAATT AGAAAACAAA TTACTACCAT TTTAAGTTTG GTGGCTAAAT AACGTGCTAA 5447
GGGAACATCT TAAAAAGTGA ATTTTGATCA AATATTTCTT AAGCATATGT GATAGACTTT 5507
GAAACCAAAA AAAAAAAAAA AAAAAAAAAA AAAAAAAAAA AAAAAAAA 5555

(2) INFORMATION FOR SEQ ID NO:4:

(i) SEQUENCE CHARACTERISTICS:
(A) LENGTH: 1058 amino acids
(B) TYPE: amino acid
(D) TOPOLOGY: linear

(ii) MOLECULE TYPE: protein

(xi) SEQUENCE DESCRIPTION: SEQ ID NO:4:

Met Lys Pro Ala Thr Gly Leu Trp Val Trp Val Ser Leu Leu Val Ala
1 5 10 15

```
Ala Gly Thr Val Gln Pro Ser Asp Ser Gln Ser Val Cys Ala Gly Thr
            20                  25                  30

Glu Asn Lys Leu Ser Ser Leu Ser Asp Leu Glu Gln Gln Tyr Arg Ala
        35                  40                  45

Leu Arg Lys Tyr Tyr Glu Asn Cys Glu Val Val Met Gly Asn Leu Glu
    50                  55                  60

Ile Thr Ser Ile Glu His Asn Arg Asp Leu Ser Phe Leu Arg Ser Val
65                  70                  75                  80

Arg Glu Val Thr Gly Tyr Val Leu Val Ala Leu Asn Gln Phe Arg Tyr
                85                  90                  95

Leu Pro Leu Glu Asn Leu Arg Ile Ile Arg Gly Thr Lys Leu Tyr Glu
            100                 105                 110

Asp Arg Tyr Ala Leu Ala Ile Phe Leu Asn Tyr Arg Lys Asp Gly Asn
        115                 120                 125

Phe Gly Leu Gln Glu Leu Gly Leu Lys Asn Leu Thr Glu Ile Leu Asn
    130                 135                 140

Gly Gly Val Tyr Val Asp Gln Asn Lys Phe Leu Cys Tyr Ala Asp Thr
145                 150                 155                 160

Ile His Trp Gln Asp Ile Val Arg Asn Pro Trp Pro Ser Asn Leu Thr
                165                 170                 175

Leu Val Ser Thr Asn Gly Ser Ser Gly Cys Gly Arg Cys His Lys Ser
            180                 185                 190

Cys Thr Gly Arg Cys Trp Gly Pro Thr Glu Asn His Cys Gln Thr Leu
        195                 200                 205

Thr Arg Thr Val Cys Ala Glu Gln Cys Asp Gly Arg Cys Tyr Gly Pro
    210                 215                 220

Tyr Val Ser Asp Cys Cys His Arg Glu Cys Ala Gly Gly Cys Ser Gly
225                 230                 235                 240

Pro Lys Asp Thr Asp Cys Phe Ala Cys Met Asn Phe Asn Asp Ser Gly
                245                 250                 255

Ala Cys Val Thr Gln Cys Pro Gln Thr Phe Val Tyr Asn Pro Thr Thr
            260                 265                 270

Phe Gln Leu Glu His Asn Phe Asn Ala Lys Tyr Thr Tyr Gly Ala Phe
        275                 280                 285

Cys Val Lys Lys Cys Pro His Asn Phe Val Val Asp Ser Ser Ser Cys
    290                 295                 300

Val Arg Ala Cys Pro Ser Ser Lys Met Glu Val Glu Glu Asn Gly Ile
305                 310                 315                 320
```

```
Lys Met Cys Lys Pro Cys Thr Asp Ile Cys Pro Lys Ala Cys Asp Gly
                325                 330                 335

Ile Gly Thr Gly Ser Leu Met Ser Ala Gln Thr Val Asp Ser Ser Asn
            340                 345                 350

Ile Asp Lys Phe Ile Asn Cys Thr Lys Ile Asn Gly Asn Leu Ile Phe
        355                 360                 365

Leu Val Thr Gly Ile His Gly Asp Pro Tyr Asn Ala Ile Glu Ala Ile
    370                 375                 380

Asp Pro Glu Lys Leu Asn Val Phe Arg Thr Val Arg Glu Ile Thr Gly
385                 390                 395                 400

Phe Leu Asn Ile Gln Ser Trp Pro Pro Asn Met Thr Asp Phe Ser Val
                405                 410                 415

Phe Ser Asn Leu Val Thr Ile Gly Gly Arg Val Leu Tyr Ser Gly Leu
            420                 425                 430

Ser Leu Leu Ile Leu Lys Gln Gln Gly Ile Thr Ser Leu Gln Phe Gln
        435                 440                 445

Ser Leu Lys Glu Ile Ser Ala Gly Asn Ile Tyr Ile Thr Asp Asn Ser
    450                 455                 460

Asn Leu Cys Tyr Tyr His Thr Ile Asn Trp Thr Thr Leu Phe Ser Thr
465                 470                 475                 480

Ile Asn Gln Arg Ile Val Ile Arg Asp Asn Arg Lys Ala Glu Asn Cys
                485                 490                 495

Thr Ala Glu Gly Met Val Cys Asn His Leu Cys Ser Ser Asp Gly Cys
            500                 505                 510

Trp Gly Pro Gly Pro Asp Gln Cys Leu Ser Cys Arg Arg Phe Ser Arg
        515                 520                 525

Gly Arg Ile Cys Ile Glu Ser Cys Asn Leu Tyr Asp Gly Glu Phe Arg
    530                 535                 540

Glu Phe Glu Asn Gly Ser Ile Cys Val Glu Cys Asp Pro Gln Cys Glu
545                 550                 555                 560

Lys Met Glu Asp Gly Leu Leu Thr Cys His Gly Pro Gly Pro Asp Asn
                565                 570                 575

Cys Thr Lys Cys Ser His Phe Lys Asp Gly Pro Asn Cys Val Glu Lys
            580                 585                 590

Cys Pro Asp Gly Leu Gln Gly Ala Asn Ser Phe Ile Phe Lys Tyr Ala
        595                 600                 605

Asp Pro Asp Arg Glu Cys His Pro Cys His Pro Asn Cys Thr Gln Gly
    610                 615                 620
```

```
Cys Asn Gly Pro Thr Ser His Asp Cys Ile Tyr Tyr Pro Trp Thr Gly
625                 630                 635                 640

His Ser Thr Leu Pro Gln His Ala Arg Thr Pro Leu Ile Ala Ala Gly
                645                 650                 655

Val Ile Gly Gly Leu Phe Ile Leu Val Ile Val Gly Leu Thr Phe Ala
            660                 665                 670

Val Tyr Val Arg Arg Lys Ser Ile Lys Lys Lys Arg Ala Leu Arg Arg
        675                 680                 685

Phe Leu Glu Thr Glu Leu Val Glu Pro Leu Thr Pro Ser Gly Thr Ala
    690                 695                 700

Pro Asn Gln Ala Gln Leu Arg Ile Leu Lys Glu Thr Glu Leu Lys Arg
705                 710                 715                 720

Val Lys Val Leu Gly Ser Gly Ala Phe Gly Thr Val Tyr Lys Gly Ile
                725                 730                 735

Trp Val Pro Glu Gly Glu Thr Val Lys Ile Pro Val Ala Ile Lys Ile
            740                 745                 750

Leu Asn Glu Thr Thr Gly Pro Lys Ala Asn Val Glu Phe Met Asp Glu
        755                 760                 765

Ala Leu Ile Met Ala Ser Met Asp His Pro His Leu Val Arg Leu Leu
    770                 775                 780

Gly Val Cys Leu Ser Pro Thr Ile Gln Leu Val Thr Gln Leu Met Pro
785                 790                 795                 800

His Gly Cys Leu Leu Glu Tyr Val His Glu His Lys Asp Asn Ile Gly
                805                 810                 815

Ser Gln Leu Leu Leu Asn Trp Cys Val Gln Ile Ala Lys Gly Met Met
            820                 825                 830

Tyr Leu Glu Glu Arg Arg Leu Val His Arg Asp Leu Ala Ala Arg Asn
        835                 840                 845

Val Leu Val Lys Ser Pro Asn His Val Lys Ile Thr Asp Phe Gly Leu
    850                 855                 860

Ala Arg Leu Leu Glu Gly Asp Glu Lys Glu Tyr Asn Ala Asp Gly Gly
865                 870                 875                 880

Lys Met Pro Ile Lys Trp Met Ala Leu Glu Cys Ile His Tyr Arg Lys
                885                 890                 895

Phe Thr His Gln Ser Asp Val Trp Ser Tyr Gly Val Thr Ile Trp Glu
            900                 905                 910

Leu Met Thr Phe Gly Gly Lys Pro Tyr Asp Gly Ile Pro Thr Arg Glu
        915                 920                 925
```

```
Ile Pro Asp Leu Leu Glu Lys Gly Glu Arg Leu Pro Gln Pro Pro Ile
    930                 935                 940

Cys Thr Ile Asp Val Tyr Met Val Met Val Lys Cys Trp Met Ile Asp
945                 950                 955                 960

Ala Asp Ser Arg Pro Lys Phe Lys Glu Leu Ala Ala Glu Phe Ser Arg
                965                 970                 975

Met Ala Arg Asp Pro Gln Arg Tyr Leu Val Ile Gln Gly Asp Asp Arg
            980                 985                 990

Met Lys Leu Pro Ser Pro Asn Asp Ser Lys Phe Phe Gln Asn Leu Leu
        995                 1000                1005

Asp Glu Glu Asp Leu Glu Asp Met Met Asp Ala Glu Glu Tyr Leu Val
    1010                1015                1020

Pro Gln Ala Phe Asn Ile Pro Pro Pro Ile Tyr Thr Ser Arg Ala Arg
1025                1030                1035                1040

Ile Asp Ser Asn Arg Ser Val Arg Asn Asn Tyr Ile His Ile Ser Tyr
                1045                1050                1055

Ser Phe
```

(2) INFORMATION FOR SEQ ID NO:5:

(i) SEQUENCE CHARACTERISTICS:
(A) LENGTH: 3321 base pairs
(B) TYPE: nucleic acid
(C) STRANDEDNESS: single
(D) TOPOLOGY: unknown

(ii) MOLECULE TYPE: DNA (genomic)

(ix) FEATURE:
(A) NAME/KEY: CDS
(B) LOCATION: 156..1782

(xi) SEQUENCE DESCRIPTION: SEQ ID NO:5:

```
CATTAGCTGC AATTGATCAA GTGACTGAGA GAAGGGCAAC ATTCCATGCA ACAGTATAGT       60

GGTATGGAAA GCCCTGGATG TTGAAATCTA GCTTCAAAAA GCCTGTCTGG AAATGTAGTT      120

AATTGGATGA AGTGAGAAGA GATAAAACCA GAGAG GAA GCT CTG ATC ATG GCA        173
                                      Glu Ala Leu Ile Met Ala
                                        1               5

AGT ATG GAT CAT CCA CAC CTA GTC CGG TTG CTG GGT GTG TGT CTG AGC       221
Ser Met Asp His Pro His Leu Val Arg Leu Leu Gly Val Cys Leu Ser
            10                  15                  20
```

```
CCA ACC ATC CAG CTG GTT ACT CAA CTT ATG CCC CAT GGC TGC CTG TTG        269
Pro Thr Ile Gln Leu Val Thr Gln Leu Met Pro His Gly Cys Leu Leu
        25                  30                  35

GAG TAT GTC CAC GAG CAC AAG GAT AAC ATT GGA TCA CAA CTG CTG CTT        317
Glu Tyr Val His Glu His Lys Asp Asn Ile Gly Ser Gln Leu Leu Leu
    40                  45                  50

AAC TGG TGT GTC CAG ATA GCT AAG GGA ATG ATG TAC CTG GAA GAA AGA        365
Asn Trp Cys Val Gln Ile Ala Lys Gly Met Met Tyr Leu Glu Glu Arg
55                  60                  65                  70

CGA CTC GTT CAT CGG GAT TTG GCA GCC CGT AAT GTC TTA GTG AAA TCT        413
Arg Leu Val His Arg Asp Leu Ala Ala Arg Asn Val Leu Val Lys Ser
                75                  80                  85

CCA AAC CAT GTG AAA ATC ACA GAT TTT GGG CTA GCC AGA CTC TTG GAA        461
Pro Asn His Val Lys Ile Thr Asp Phe Gly Leu Ala Arg Leu Leu Glu
            90                  95                  100

GGA GAT GAA AAA GAG TAC AAT GCT GAT GGA GGA AAG ATG CCA ATT AAA        509
Gly Asp Glu Lys Glu Tyr Asn Ala Asp Gly Gly Lys Met Pro Ile Lys
        105                 110                 115

TGG ATG GCT CTG GAG TGT ATA CAT TAC AGG AAA TTC ACC CAT CAG AGT        557
Trp Met Ala Leu Glu Cys Ile His Tyr Arg Lys Phe Thr His Gln Ser
    120                 125                 130

GAC GTT TGG AGC TAT GGA GTT ACT ATA TGG GAA CTG ATG ACC TTT GGA        605
Asp Val Trp Ser Tyr Gly Val Thr Ile Trp Glu Leu Met Thr Phe Gly
135                 140                 145                 150

GGA AAA CCC TAT GAT GGA ATT CCA ACG CGA GAA ATC CCT GAT TTA TTA        653
Gly Lys Pro Tyr Asp Gly Ile Pro Thr Arg Glu Ile Pro Asp Leu Leu
                155                 160                 165

GAG AAA GGA GAA CGT TTG CCT CAG CCT CCC ATC TGC ACT ATT GAC GTT        701
Glu Lys Gly Glu Arg Leu Pro Gln Pro Pro Ile Cys Thr Ile Asp Val
            170                 175                 180

TAC ATG GTC ATG GTC AAA TGT TGG ATG ATT GAT GCT GAC AGT AGA CCT        749
Tyr Met Val Met Val Lys Cys Trp Met Ile Asp Ala Asp Ser Arg Pro
        185                 190                 195

AAA TTT AAG GAA CTG GCT GCT GAG TTT TCA AGG ATG GCT CGA GAC CCT        797
Lys Phe Lys Glu Leu Ala Ala Glu Phe Ser Arg Met Ala Arg Asp Pro
    200                 205                 210

CAA AGA TAC CTA GTT ATT CAG GGT GAT GAT CGT ATG AAG CTT CCC AGT        845
Gln Arg Tyr Leu Val Ile Gln Gly Asp Asp Arg Met Lys Leu Pro Ser
215                 220                 225                 230

CCA AAT GAC AGC AAG TTC TTT CAG AAT CTC TTG GAT GAA GAG GAT TTG        893
Pro Asn Asp Ser Lys Phe Phe Gln Asn Leu Leu Asp Glu Glu Asp Leu
                235                 240                 245
```

```
GAA GAT ATG ATG GAT GCT GAG GAG TAC TTG GTC CCT CAG GCT TTC AAC     941
Glu Asp Met Met Asp Ala Glu Glu Tyr Leu Val Pro Gln Ala Phe Asn
            250                 255                 260

ATC CCA CCT CCC ATC TAT ACT TCC AGA GCA AGA ATT GAC TCG AAT AGG     989
Ile Pro Pro Pro Ile Tyr Thr Ser Arg Ala Arg Ile Asp Ser Asn Arg
        265                 270                 275

AGT GAA ATT GGA CAC AGC CCT CCT CCT GCC TAC ACC CCC ATG TCA GGA    1037
Ser Glu Ile Gly His Ser Pro Pro Pro Ala Tyr Thr Pro Met Ser Gly
    280                 285                 290

AAC CAG TTT GTA TAC CGA GAT GGA GGT TTT GCT GCT GAA CAA GGA GTG    1085
Asn Gln Phe Val Tyr Arg Asp Gly Gly Phe Ala Ala Glu Gln Gly Val
295                 300                 305                 310

TCT GTG CCC TAC AGA GCC CCA ACT AGC ACA ATT CCA GAA GCT CCT GTG    1133
Ser Val Pro Tyr Arg Ala Pro Thr Ser Thr Ile Pro Glu Ala Pro Val
                315                 320                 325

GCA CAG GGT GCT ACT GCT GAG ATT TTT GAT GAC TCC TGC TGT AAT GGC    1181
Ala Gln Gly Ala Thr Ala Glu Ile Phe Asp Asp Ser Cys Cys Asn Gly
            330                 335                 340

ACC CTA CGC AAG CCA GTG GCA CCC CAT GTC CAA GAG GAC AGT AGC ACC    1229
Thr Leu Arg Lys Pro Val Ala Pro His Val Gln Glu Asp Ser Ser Thr
        345                 350                 355

CAG AGG TAC AGT GCT GAC CCC ACC GTG TTT GCC CCA GAA CGG AGC CCA    1277
Gln Arg Tyr Ser Ala Asp Pro Thr Val Phe Ala Pro Glu Arg Ser Pro
    360                 365                 370

CGA GGA GAG CTG GAT GAG GAA GGT TAC ATG ACT CCT ATG CGA GAC AAA    1325
Arg Gly Glu Leu Asp Glu Glu Gly Tyr Met Thr Pro Met Arg Asp Lys
375                 380                 385                 390

CCC AAA CAA GAA TAC CTG AAT CCA GTG GAG GAG AAC CCT TTT GTT TCT    1373
Pro Lys Gln Glu Tyr Leu Asn Pro Val Glu Glu Asn Pro Phe Val Ser
                395                 400                 405

CGG AGA AAA AAT GGA GAC CTT CAA GCA TTG GAT AAT CCC GAA TAT CAC    1421
Arg Arg Lys Asn Gly Asp Leu Gln Ala Leu Asp Asn Pro Glu Tyr His
            410                 415                 420

AAT GCA TCC AAT GGT CCA CCC AAG GCC GAG GAT GAG TAT GTG AAT GAG    1469
Asn Ala Ser Asn Gly Pro Pro Lys Ala Glu Asp Glu Tyr Val Asn Glu
        425                 430                 435

CCA CTG TAC CTC AAC ACC TTT GCC AAC ACC TTG GGA AAA GCT GAG TAC    1517
Pro Leu Tyr Leu Asn Thr Phe Ala Asn Thr Leu Gly Lys Ala Glu Tyr
    440                 445                 450

CTG AAG AAC AAC ATA CTG TCA ATG CCA GAG AAG GCC AAG AAA GCG TTT    1565
Leu Lys Asn Asn Ile Leu Ser Met Pro Glu Lys Ala Lys Lys Ala Phe
455                 460                 465                 470
```

```
GAC AAC CCT GAC TAC TGG AAC CAC AGC CTG CCA CCT CGG AGC ACC CTT       1613
Asp Asn Pro Asp Tyr Trp Asn His Ser Leu Pro Pro Arg Ser Thr Leu
                475                 480                 485

CAG CAC CCA GAC TAC CTG CAG GAG TAC AGC ACA AAA TAT TTT TAT AAA       1661
Gln His Pro Asp Tyr Leu Gln Glu Tyr Ser Thr Lys Tyr Phe Tyr Lys
            490                 495                 500

CAG AAT GGG CGG ATC CGG CCT ATT GTG GCA GAG AAT CCT GAA TAC CTC       1709
Gln Asn Gly Arg Ile Arg Pro Ile Val Ala Glu Asn Pro Glu Tyr Leu
        505                 510                 515

TCT GAG TTC TCC CTG AAG CCA GGC ACT GTG CTG CCG CCT CCA CCT TAC       1757
Ser Glu Phe Ser Leu Lys Pro Gly Thr Val Leu Pro Pro Pro Pro Tyr
    520                 525                 530

AGA CAC CGG AAT ACT GTG GTG TAAGCTCAGT TGTGGTTTTT TAGGTGGAGA          1808

Arg His Arg Asn Thr Val Val
535                 540

GACACACCTG CTCCAATTTC CCCACCCCCC TCTCTTTCTC TGGTGGTCTT CCTTCTACCC     1868

CAAGGCCAGT AGTTTTGACA CTTCCCAGTG GAAGATACAG AGATGCAATG ATAGTTATGT     1928

GCTTACCTAA CTTGAACATT AGAGGGAAAG ACTGAAAGAG AAAGATAGGA GGAACCACAA     1988

TGTTTCTTCA TTTCTCTGCA TGGGTTGGTC AGGAGAATGA AACAGCTAGA GAAGGACCAG     2048

AAAATGTAAG GCAATGCTGC CTACTATCAA ACTAGCTGTC ACTTTTTTTC TTTTTCTTTT     2108

TCTTTCTTTG TTTCTTTCTT CCTCTTCTTT TTTTTTTTTT TTTTAAAGCA GATGGTTGAA     2168

ACACCCATGC TATCTGTTCC TATCTGCAGG AACTGATGTG TGCATATTTA GCATCCCTGG     2228

AAATCATAAT AAAGTTTCCA TTAGAACAAA AGAATAACAT TTTCTATAAC ATATGATAGT     2288

GTCTGAAATT GAGAATCCAG TTTCTTTCCC CAGCAGTTTC TGTCCTAGCA AGTAAGAATG     2348

GCCAACTCAA CTTTCATAAT TTAAAAATCT CCATTAAAGT TATAACTAGT AATTATGTTT     2408

TCAACACTTT TTGGTTTTTT TCATTTTGTT TTGCTCTGAC CGATTCCTTT ATATTTGCTC     2468

CCCTATTTTT GGCTTTAATT TCTAATTGCA AAGATGTTTA CATCAAAGCT TCTTCACAGA     2528

ATTTAAGCAA GAAATATTTT AATATAGTGA AATGGCCACT ACTTTAAGTA TACAATCTTT     2588

AAAATAAGAA AGGGAGGCTA ATATTTTTCA TGCTATCAAA TTATCTTCAC CCTCATCCTT     2648

TACATTTTTC AACATTTTTT TTTCTCCATA AATGACACTA CTTGATAGGC CGTTGGTTGT     2708

CTGAAGAGTA GAAGGGAAAC TAAGAGACAG TTCTCTGTGG TTCAGGAAAA CTACTGATAC     2768

TTTCAGGGGT GGCCCAATGA GGGAATCCAT TGAACTGGAA GAAACACACT GGATTGGGTA     2828

TGTCTACCTG GCAGATACTC AGAAATGTAG TTTGCACTTA AGCTGTAATT TTATTTGTTC     2888
```

TTTTTCTGAA CTCCATTTTG GATTTTGAAT CAAGCAATAT GGAAGCAACC AGCAAATTAA 2948

CTAATTTAAG TACATTTTTA AAAAAAGAGC TAAGATAAAG ACTGTGGAAA TGCCAAACCA 3008

AGCAAATTAG GAACCTTGCA ACGGTATCCA GGGACTATGA TGAGAGGCCA GCACATTATC 3068

TTCATATGTC ACCTTTGCTA CGCAAGGAAA TTTGTTCAGT TCGTATACTT CGTAAGAAGG 3128

AATGCGAGTA AGGATTGGCT TGAATTCCAT GGAATTTCTA GTATGAGACT ATTTATATGA 3188

AGTAGAAGGT AACTCTTTGC ACATAAATTG GTATAATAAA AAGAAAAACA CAAACATTCA 3248

AAGCTTAGGG ATAGGTCCTT GGGTCAAAAG TTGTAAATAA ATGTGAAACA TCTTCTCAAA 3308

AAAAAAAAAA AAA 3321

(2) INFORMATION FOR SEQ ID NO:6:

(i) SEQUENCE CHARACTERISTICS:
(A) LENGTH: 541 amino acids
(B) TYPE: amino acid
(D) TOPOLOGY: linear

(ii) MOLECULE TYPE: protein

(xi) SEQUENCE DESCRIPTION: SEQ ID NO:6:

Glu Ala Leu Ile Met Ala Ser Met Asp His Pro His Leu Val Arg Leu
1               5                   10                  15

Leu Gly Val Cys Leu Ser Pro Thr Ile Gln Leu Val Thr Gln Leu Met
            20                  25                  30

Pro His Gly Cys Leu Leu Glu Tyr Val His Glu His Lys Asp Asn Ile
        35                  40                  45

Gly Ser Gln Leu Leu Leu Asn Trp Cys Val Gln Ile Ala Lys Gly Met
    50                  55                  60

Met Tyr Leu Glu Glu Arg Arg Leu Val His Arg Asp Leu Ala Ala Arg
65                  70                  75                  80

Asn Val Leu Val Lys Ser Pro Asn His Val Lys Ile Thr Asp Phe Gly
                85                  90                  95

Leu Ala Arg Leu Leu Glu Gly Asp Glu Lys Glu Tyr Asn Ala Asp Gly
            100                 105                 110

Gly Lys Met Pro Ile Lys Trp Met Ala Leu Glu Cys Ile His Tyr Arg
        115                 120                 125

Lys Phe Thr His Gln Ser Asp Val Trp Ser Tyr Gly Val Thr Ile Trp
    130                 135                 140

Glu Leu Met Thr Phe Gly Gly Lys Pro Tyr Asp Gly Ile Pro Thr Arg
145                 150                 155                 160

Glu Ile Pro Asp Leu Leu Glu Lys Gly Glu Arg Leu Pro Gln Pro Pro
165 170 175

Ile Cys Thr Ile Asp Val Tyr Met Val Met Val Lys Cys Trp Met Ile
180 185 190

Asp Ala Asp Ser Arg Pro Lys Phe Lys Glu Leu Ala Ala Glu Phe Ser
195 200 205

Arg Met Ala Arg Asp Pro Gln Arg Tyr Leu Val Ile Gln Gly Asp Asp
210 215 220

Arg Met Lys Leu Pro Ser Pro Asn Asp Ser Lys Phe Phe Gln Asn Leu
225 230 235 240

Leu Asp Glu Glu Asp Leu Glu Asp Met Met Asp Ala Glu Glu Tyr Leu
245 250 255

Val Pro Gln Ala Phe Asn Ile Pro Pro Pro Ile Tyr Thr Ser Arg Ala
260 265 270

Arg Ile Asp Ser Asn Arg Ser Glu Ile Gly His Ser Pro Pro Pro Ala
275 280 285

Tyr Thr Pro Met Ser Gly Asn Gln Phe Val Tyr Arg Asp Gly Gly Phe
290 295 300

Ala Ala Glu Gln Gly Val Ser Val Pro Tyr Arg Ala Pro Thr Ser Thr
305 310 315 320

Ile Pro Glu Ala Pro Val Ala Gln Gly Ala Thr Ala Glu Ile Phe Asp
325 330 335

Asp Ser Cys Cys Asn Gly Thr Leu Arg Lys Pro Val Ala Pro His Val
340 345 350

Gln Glu Asp Ser Ser Thr Gln Arg Tyr Ser Ala Asp Pro Thr Val Phe
355 360 365

Ala Pro Glu Arg Ser Pro Arg Gly Glu Leu Asp Glu Glu Gly Tyr Met
370 375 380

Thr Pro Met Arg Asp Lys Pro Lys Gln Glu Tyr Leu Asn Pro Val Glu
385 390 395 400

Glu Asn Pro Phe Val Ser Arg Arg Lys Asn Gly Asp Leu Gln Ala Leu
405 410 415

Asp Asn Pro Glu Tyr His Asn Ala Ser Asn Gly Pro Pro Lys Ala Glu
420 425 430

Asp Glu Tyr Val Asn Glu Pro Leu Tyr Leu Asn Thr Phe Ala Asn Thr
435 440 445

Leu Gly Lys Ala Glu Tyr Leu Lys Asn Asn Ile Leu Ser Met Pro Glu
450 455 460

```
Lys Ala Lys Lys Ala Phe Asp Asn Pro Asp Tyr Trp Asn His Ser Leu
465                 470                 475                 480

Pro Pro Arg Ser Thr Leu Gln His Pro Asp Tyr Leu Gln Glu Tyr Ser
                485                 490                 495

Thr Lys Tyr Phe Tyr Lys Gln Asn Gly Arg Ile Arg Pro Ile Val Ala
            500                 505                 510

Glu Asn Pro Glu Tyr Leu Ser Glu Phe Ser Leu Lys Pro Gly Thr Val
        515                 520                 525

Leu Pro Pro Pro Pro Tyr Arg His Arg Asn Thr Val Val
    530                 535                 540
```

(2) INFORMATION FOR SEQ ID NO:7:

(i) SEQUENCE CHARACTERISTICS:
(A) LENGTH: 1210 amino acids
(B) TYPE: amino acid
(C) STRANDEDNESS: unknown
(D) TOPOLOGY: unknown

(ii) MOLECULE TYPE: protein

(xi) SEQUENCE DESCRIPTION: SEQ ID NO:7:

```
Met Arg Pro Ser Gly Thr Ala Gly Ala Ala Leu Leu Ala Leu Leu Ala
1               5                   10                  15

Ala Leu Cys Pro Ala Ser Arg Ala Leu Glu Glu Lys Lys Val Cys Gln
            20                  25                  30

Gly Thr Ser Asn Lys Leu Thr Gln Leu Gly Thr Phe Glu Asp His Phe
        35                  40                  45

Leu Ser Leu Gln Arg Met Phe Asn Asn Cys Glu Val Val Leu Gly Asn
    50                  55                  60

Leu Glu Ile Thr Tyr Val Gln Arg Asn Tyr Asp Leu Ser Phe Leu Lys
65                  70                  75                  80

Thr Ile Gln Glu Val Ala Gly Tyr Val Leu Ile Ala Leu Asn Thr Val
                85                  90                  95

Glu Arg Ile Pro Leu Glu Asn Leu Gln Ile Ile Arg Gly Asn Met Tyr
            100                 105                 110

Tyr Glu Asn Ser Tyr Ala Leu Ala Val Leu Ser Asn Tyr Asp Ala Asn
        115                 120                 125

Lys Thr Gly Leu Lys Glu Leu Pro Met Arg Asn Leu Gln Glu Ile Leu
    130                 135                 140
```

His Gly Ala Val Arg Phe Ser Asn Asn Pro Ala Leu Cys Asn Val Glu
145 150 155 160

Ser Ile Gln Trp Arg Asp Ile Val Ser Ser Asp Phe Leu Ser Asn Met
165 170 175

Ser Met Asp Phe Gln Asn His Leu Gly Ser Cys Gln Lys Cys Asp Pro
180 185 190

Ser Cys Pro Asn Gly Ser Cys Trp Gly Ala Gly Glu Glu Asn Cys Gln
195 200 205

Lys Leu Thr Lys Ile Ile Cys Ala Gln Gln Cys Ser Gly Arg Cys Arg
210 215 220

Gly Lys Ser Pro Ser Asp Cys Cys His Asn Gln Cys Ala Ala Gly Cys
225 230 235 240

Thr Gly Pro Arg Glu Ser Asp Cys Leu Val Cys Arg Lys Phe Arg Asp
245 250 255

Glu Ala Thr Cys Lys Asp Thr Cys Pro Pro Leu Met Leu Tyr Asn Pro
260 265 270

Thr Thr Tyr Gln Met Asp Val Asn Pro Glu Gly Lys Tyr Ser Phe Gly
275 280 285

Ala Thr Cys Val Lys Lys Cys Pro Arg Asn Tyr Val Val Thr Asp His
290 295 300

Gly Ser Cys Val Arg Ala Cys Gly Ala Asp Ser Tyr Glu Met Glu Glu
305 310 315 320

Asp Gly Val Arg Lys Cys Lys Lys Cys Glu Gly Pro Cys Arg Lys Val
325 330 335

Cys Asn Gly Ile Gly Ile Gly Glu Phe Lys Asp Ser Leu Ser Ile Asn
340 345 350

Ala Thr Asn Ile Lys His Phe Lys Asn Cys Thr Ser Ile Ser Gly Asp
355 360 365

Leu His Ile Leu Pro Val Ala Phe Arg Gly Asp Ser Phe Thr His Thr
370 375 380

Pro Pro Leu Asp Pro Gln Glu Leu Asp Ile Leu Lys Thr Val Lys Glu
385 390 395 400

Ile Thr Gly Phe Leu Leu Ile Gln Ala Trp Pro Glu Asn Arg Thr Asp
405 410 415

Leu His Ala Phe Glu Asn Leu Glu Ile Ile Arg Gly Arg Thr Lys Gln
420 425 430

His Gly Gln Phe Ser Leu Ala Val Val Ser Leu Asn Ile Thr Ser Leu
435 440 445

Gly Leu Arg Ser Leu Lys Glu Ile Ser Asp Gly Asp Val Ile Ile Ser
450 455 460

Gly Asn Lys Asn Leu Cys Tyr Ala Asn Thr Ile Asn Trp Lys Lys Leu
465 470 475 480

Phe Gly Thr Ser Gly Gln Lys Thr Lys Ile Ile Ser Asn Arg Gly Glu
485 490 495

Asn Ser Cys Lys Ala Thr Gly Gln Val Cys His Ala Leu Cys Ser Pro
500 505 510

Glu Gly Cys Trp Gly Pro Glu Pro Arg Asp Cys Val Ser Cys Arg Asn
515 520 525

Val Ser Arg Gly Arg Glu Cys Val Asp Lys Cys Lys Leu Leu Glu Gly
530 535 540

Glu Pro Arg Glu Phe Val Glu Asn Ser Glu Cys Ile Gln Cys His Pro
545 550 555 560

Glu Cys Leu Pro Gln Ala Met Asn Ile Thr Cys Thr Gly Arg Gly Pro
565 570 575

Asp Asn Cys Ile Gln Cys Ala His Tyr Ile Asp Gly Pro His Cys Val
580 585 590

Lys Thr Cys Pro Ala Gly Val Met Gly Glu Asn Asn Thr Leu Val Trp
595 600 605

Lys Tyr Ala Asp Ala Gly His Val Cys His Leu Cys His Pro Asn Cys
610 615 620

Thr Tyr Gly Cys Thr Gly Pro Gly Leu Glu Gly Cys Pro Thr Asn Gly
625 630 635 640

Pro Lys Ile Pro Ser Ile Ala Thr Gly Met Val Gly Ala Leu Leu Leu
645 650 655

Leu Leu Val Val Ala Leu Gly Ile Gly Leu Phe Met Arg Arg Arg His
660 665 670

Ile Val Arg Lys Arg Thr Leu Arg Arg Leu Leu Gln Glu Arg Glu Leu
675 680 685

Val Glu Pro Leu Thr Pro Ser Gly Glu Ala Pro Asn Gln Ala Leu Leu
690 695 700

Arg Ile Leu Lys Glu Thr Glu Phe Lys Lys Ile Lys Val Leu Gly Ser
705 710 715 720

Gly Ala Phe Gly Thr Val Tyr Lys Gly Leu Trp Ile Pro Glu Gly Glu
725 730 735

Lys Val Lys Ile Pro Val Ala Ile Lys Glu Leu Arg Glu Ala Thr Ser
740 745 750

```
Pro Lys Ala Asn Lys Glu Ile Leu Asp Glu Ala Tyr Val Met Ala Ser
        755                 760                 765

Val Asp Asn Pro His Val Cys Arg Leu Leu Gly Ile Cys Leu Thr Ser
    770                 775                 780

Thr Val Gln Leu Ile Thr Gln Leu Met Pro Phe Gly Cys Leu Leu Asp
785                 790                 795                 800

Tyr Val Arg Glu His Lys Asp Asn Ile Gly Ser Gln Tyr Leu Leu Asn
                805                 810                 815

Trp Cys Val Gln Ile Ala Lys Gly Met Met Tyr Leu Glu Asp Arg Arg
            820                 825                 830

Leu Val His Arg Asp Leu Ala Ala Arg Asn Val Leu Val Lys Thr Pro
        835                 840                 845

Gln His Val Lys Ile Thr Asp Phe Gly Leu Ala Lys Leu Leu Gly Ala
    850                 855                 860

Glu Glu Lys Glu Tyr His Ala Glu Gly Gly Lys Val Pro Ile Lys Trp
865                 870                 875                 880

Met Ala Leu Glu Ser Ile Leu His Arg Ile Tyr Thr His Gln Ser Asp
                885                 890                 895

Val Trp Ser Tyr Gly Val Thr Val Trp Glu Leu Met Thr Phe Gly Ser
            900                 905                 910

Lys Pro Tyr Asp Gly Ile Pro Ala Ser Glu Ile Ser Ser Ile Leu Glu
        915                 920                 925

Lys Gly Glu Arg Leu Pro Gln Pro Pro Ile Cys Thr Ile Asp Val Tyr
    930                 935                 940

Met Ile Met Val Lys Cys Trp Met Ile Asp Ala Asp Ser Arg Pro Lys
945                 950                 955                 960

Phe Arg Glu Leu Ile Ile Glu Phe Ser Lys Met Ala Arg Asp Pro Gln
                965                 970                 975

Arg Tyr Leu Val Ile Gln Gly Asp Glu Arg Met His Leu Pro Ser Pro
            980                 985                 990

Thr Asp Ser Asn Phe Tyr Arg Ala Leu Met Asp Glu Glu Asp Met Asp
        995                 1000                1005

Asp Val Val Asp Ala Asp Glu Tyr Leu Ile Pro Gln Gln Gly Phe Phe
    1010                1015                1020

Ser Ser Pro Ser Thr Ser Arg Thr Pro Leu Leu Ser Ser Leu Ser Ala
1025                1030                1035                1040

Thr Ser Asn Asn Ser Thr Val Ala Cys Ile Asp Arg Asn Gly Leu Gln
                1045                1050                1055
```

```
Ser Cys Pro Ile Lys Glu Asp Ser Phe Leu Gln Arg Tyr Ser Ser Asp
            1060                1065                1070

Pro Thr Gly Ala Leu Thr Glu Asp Ser Ile Asp Asp Thr Phe Leu Pro
        1075                1080                1085

Val Pro Glu Tyr Ile Asn Gln Ser Val Pro Lys Arg Pro Ala Gly Ser
    1090                1095                1100

Val Gln Asn Pro Val Tyr His Asn Gln Pro Leu Asn Pro Ala Pro Ser
1105                1110                1115                1120

Arg Asp Pro His Tyr Gln Asp Pro His Ser Thr Ala Val Gly Asn Pro
                1125                1130                1135

Glu Tyr Leu Asn Thr Val Gln Pro Thr Cys Val Asn Ser Thr Phe Asp
            1140                1145                1150

Ser Pro Ala His Trp Ala Gln Lys Gly Ser His Gln Ile Ser Leu Asp
        1155                1160                1165

Asn Pro Asp Tyr Gln Gln Asp Phe Phe Pro Lys Glu Ala Lys Pro Asn
    1170                1175                1180

Gly Ile Phe Lys Gly Ser Thr Ala Glu Asn Ala Glu Tyr Leu Arg Val
1185                1190                1195                1200

Ala Pro Gln Ser Ser Glu Phe Ile Gly Ala
                1205                1210
```

(2) INFORMATION FOR SEQ ID NO:8:

(i) SEQUENCE CHARACTERISTICS:
(A) LENGTH: 1255 amino acids
(B) TYPE: amino acid
(C) STRANDEDNESS: unknown
(D) TOPOLOGY: unknown

(ii) MOLECULE TYPE: protein

(xi) SEQUENCE DESCRIPTION: SEQ ID NO:8:

```
Met Glu Leu Ala Ala Leu Cys Arg Trp Gly Leu Leu Leu Ala Leu Leu
1               5                   10                  15

Pro Pro Gly Ala Ala Ser Thr Gln Val Cys Thr Gly Thr Asp Met Lys
            20                  25                  30

Leu Arg Leu Pro Ala Ser Pro Glu Thr His Leu Asp Met Leu Arg His
        35                  40                  45

Leu Tyr Gln Gly Cys Gln Val Val Gln Gly Asn Leu Glu Leu Thr Tyr
    50                  55                  60
```

```
Leu Pro Thr Asn Ala Ser Leu Ser Phe Leu Gln Asp Ile Gln Glu Val
65                  70                  75                  80

Gln Gly Tyr Val Leu Ile Ala His Asn Gln Val Arg Gln Val Pro Leu
                85                  90                  95

Gln Arg Leu Arg Ile Val Arg Gly Thr Gln Leu Phe Glu Asp Asn Tyr
            100                 105                 110

Ala Leu Ala Val Leu Asp Asn Gly Asp Pro Leu Asn Asn Thr Thr Pro
        115                 120                 125

Val Thr Gly Ala Ser Pro Gly Gly Leu Arg Glu Leu Gln Leu Arg Ser
    130                 135                 140

Leu Thr Glu Ile Leu Lys Gly Gly Val Leu Ile Gln Arg Asn Pro Gln
145                 150                 155                 160

Leu Cys Tyr Gln Asp Thr Ile Leu Trp Lys Asp Ile Phe His Lys Asn
                165                 170                 175

Asn Gln Leu Ala Leu Thr Leu Ile Asp Thr Asn Arg Ser Arg Ala Cys
            180                 185                 190

His Pro Cys Ser Pro Met Cys Lys Gly Ser Arg Cys Trp Gly Glu Ser
        195                 200                 205

Ser Glu Asp Cys Gln Ser Leu Thr Arg Thr Val Cys Ala Gly Gly Cys
    210                 215                 220

Ala Arg Cys Lys Gly Pro Leu Pro Thr Asp Cys Cys His Glu Gln Cys
225                 230                 235                 240

Ala Ala Gly Cys Thr Gly Pro Lys His Ser Asp Cys Leu Ala Cys Leu
                245                 250                 255

His Phe Asn His Ser Gly Ile Cys Glu Leu His Cys Pro Ala Leu Val
            260                 265                 270

Thr Tyr Asn Thr Asp Thr Phe Glu Ser Met Pro Asn Pro Glu Gly Arg
        275                 280                 285

Tyr Thr Phe Gly Ala Ser Cys Val Thr Ala Cys Pro Tyr Asn Tyr Leu
    290                 295                 300

Ser Thr Asp Val Gly Ser Cys Thr Leu Val Cys Pro Leu His Asn Gln
305                 310                 315                 320

Glu Val Thr Ala Glu Asp Gly Thr Gln Arg Cys Glu Lys Cys Ser Lys
                325                 330                 335

Pro Cys Ala Arg Val Cys Tyr Gly Leu Gly Met Glu His Leu Arg Glu
            340                 345                 350

Val Arg Ala Val Thr Ser Ala Asn Ile Gln Glu Phe Ala Gly Cys Lys
        355                 360                 365
```

```
Lys Ile Phe Gly Ser Leu Ala Phe Leu Pro Glu Ser Phe Asp Gly Asp
    370                 375                 380

Pro Ala Ser Asn Thr Ala Pro Leu Gln Pro Glu Gln Leu Gln Val Phe
385                 390                 395                 400

Glu Thr Leu Glu Glu Ile Thr Gly Tyr Leu Tyr Ile Ser Ala Trp Pro
                405                 410                 415

Asp Ser Leu Pro Asp Leu Ser Val Phe Gln Asn Leu Gln Val Ile Arg
            420                 425                 430

Gly Arg Ile Leu His Asn Gly Ala Tyr Ser Leu Thr Leu Gln Gly Leu
        435                 440                 445

Gly Ile Ser Trp Leu Gly Leu Arg Ser Leu Arg Glu Leu Gly Ser Gly
    450                 455                 460

Leu Ala Leu Ile His His Asn Thr His Leu Cys Phe Val His Thr Val
465                 470                 475                 480

Pro Trp Asp Gln Leu Phe Arg Asn Pro His Gln Ala Leu Leu His Thr
                485                 490                 495

Ala Asn Arg Pro Glu Asp Glu Cys Val Gly Glu Gly Leu Ala Cys His
            500                 505                 510

Gln Leu Cys Ala Arg Arg Ala Leu Leu Gly Ser Gly Pro Thr Gln Cys
        515                 520                 525

Val Asn Cys Ser Gln Phe Leu Arg Gly Gln Glu Cys Val Glu Glu Cys
    530                 535                 540

Arg Val Leu Gln Gly Leu Pro Arg Glu Tyr Val Asn Ala Arg His Cys
545                 550                 555                 560

Leu Pro Cys His Pro Glu Cys Gln Pro Gln Asn Gly Ser Val Thr Cys
                565                 570                 575

Phe Gly Pro Glu Ala Asp Gln Cys Val Ala Cys Ala His Tyr Lys Asp
            580                 585                 590

Pro Pro Phe Cys Val Ala Arg Cys Pro Ser Gly Val Lys Pro Asp Leu
        595                 600                 605

Ser Tyr Met Pro Ile Trp Lys Phe Pro Asp Glu Glu Gly Ala Cys Gln
    610                 615                 620

Pro Cys Pro Ile Asn Cys Thr His Ser Cys Val Asp Leu Asp Asp Lys
625                 630                 635                 640

Gly Cys Pro Ala Glu Gln Arg Ala Ser Pro Leu Thr Ser Ile Val Ser
                645                 650                 655

Ala Val Val Gly Ile Leu Leu Val Val Val Leu Gly Val Val Phe Gly
            660                 665                 670
```

```
Ile Leu Ile Lys Arg Arg Gln Gln Lys Ile Arg Lys Tyr Thr Met Arg
        675                 680                 685

Arg Leu Leu Gln Glu Thr Glu Leu Val Glu Pro Leu Thr Pro Ser Gly
    690                 695                 700

Ala Met Pro Asn Gln Ala Gln Met Arg Ile Leu Lys Glu Thr Glu Leu
705                 710                 715                 720

Arg Lys Val Lys Val Leu Gly Ser Gly Ala Phe Gly Thr Val Tyr Lys
                725                 730                 735

Gly Ile Trp Ile Pro Asp Gly Glu Asn Val Lys Ile Pro Val Ala Ile
            740                 745                 750

Lys Val Leu Arg Glu Asn Thr Ser Pro Lys Ala Asn Lys Glu Ile Leu
        755                 760                 765

Asp Glu Ala Tyr Val Met Ala Gly Val Gly Ser Pro Tyr Val Ser Arg
    770                 775                 780

Leu Leu Gly Ile Cys Leu Thr Ser Thr Val Gln Leu Val Thr Gln Leu
785                 790                 795                 800

Met Pro Tyr Gly Cys Leu Leu Asp His Val Arg Glu Asn Arg Gly Arg
                805                 810                 815

Leu Gly Ser Gln Asp Leu Leu Asn Trp Cys Met Gln Ile Ala Lys Gly
            820                 825                 830

Met Ser Tyr Leu Glu Asp Val Arg Leu Val His Arg Asp Leu Ala Ala
        835                 840                 845

Arg Asn Val Leu Val Lys Ser Pro Asn His Val Lys Ile Thr Asp Phe
    850                 855                 860

Gly Leu Ala Arg Leu Leu Asp Ile Asp Glu Thr Glu Tyr His Ala Asp
865                 870                 875                 880

Gly Gly Lys Val Pro Ile Lys Trp Met Ala Leu Glu Ser Ile Leu Arg
                885                 890                 895

Arg Arg Phe Thr His Gln Ser Asp Val Trp Ser Tyr Gly Val Thr Val
            900                 905                 910

Trp Glu Leu Met Thr Phe Gly Ala Lys Pro Tyr Asp Gly Ile Pro Ala
        915                 920                 925

Arg Glu Ile Pro Asp Leu Leu Glu Lys Gly Glu Arg Leu Pro Gln Pro
    930                 935                 940

Pro Ile Cys Thr Ile Asp Val Tyr Met Ile Met Val Lys Cys Trp Met
945                 950                 955                 960

Ile Asp Ser Glu Cys Arg Pro Arg Phe Arg Glu Leu Val Ser Glu Phe
                965                 970                 975
```

```
Ser Arg Met Ala Arg Asp Pro Gln Arg Phe Val Val Ile Gln Asn Glu
            980                 985                 990

Asp Leu Gly Pro Ala Ser Pro Leu Asp Ser Thr Phe Tyr Arg Ser Leu
        995                 1000                1005

Leu Glu Asp Asp Asp Met Gly Asp Leu Val Asp Ala Glu Glu Tyr Leu
    1010                1015                1020

Val Pro Gln Gln Gly Phe Phe Cys Pro Asp Pro Ala Pro Gly Ala Gly
1025                1030                1035                1040

Gly Met Val His His Arg His Arg Ser Ser Ser Thr Arg Ser Gly Gly
                1045                1050                1055

Gly Asp Leu Thr Leu Gly Leu Glu Pro Ser Glu Glu Glu Ala Pro Arg
            1060                1065                1070

Ser Pro Leu Ala Pro Ser Glu Gly Ala Gly Ser Asp Val Phe Asp Gly
        1075                1080                1085

Asp Leu Gly Met Gly Ala Ala Lys Gly Leu Gln Ser Leu Pro Thr His
    1090                1095                1100

Asp Pro Ser Pro Leu Gln Arg Tyr Ser Glu Asp Pro Thr Val Pro Leu
1105                1110                1115                1120

Pro Ser Glu Thr Asp Gly Tyr Val Ala Pro Leu Thr Cys Ser Pro Gln
                1125                1130                1135

Pro Glu Tyr Val Asn Gln Pro Asp Val Arg Pro Gln Pro Pro Ser Pro
            1140                1145                1150

Arg Glu Gly Pro Leu Pro Ala Ala Arg Pro Ala Gly Ala Thr Leu Glu
        1155                1160                1165

Arg Ala Lys Thr Leu Ser Pro Gly Lys Asn Gly Val Val Lys Asp Val
    1170                1175                1180

Phe Ala Phe Gly Gly Ala Val Glu Asn Pro Glu Tyr Leu Thr Pro Gln
1185                1190                1195                1200

Gly Gly Ala Ala Pro Gln Pro His Pro Pro Pro Ala Phe Ser Pro Ala
                1205                1210                1215

Phe Asp Asn Leu Tyr Tyr Trp Asp Gln Asp Pro Pro Glu Arg Gly Ala
            1220                1225                1230

Pro Pro Ser Thr Phe Lys Gly Thr Pro Thr Val Ala Glu Asn Pro Glu
        1235                1240                1245

Tyr Gly Leu Asp Val Pro Val
    1250                1255
```

(2) INFORMATION FOR SEQ ID NO:9:

(i) SEQUENCE CHARACTERISTICS:

(A) LENGTH: 1342 amino acids
(B) TYPE: amino acid
(C) STRANDEDNESS: unknown
(D) TOPOLOGY: unknown

(ii) MOLECULE TYPE: protein

(xi) SEQUENCE DESCRIPTION: SEQ ID NO:9:

```
Met Arg Ala Asn Asp Ala Leu Gln Val Leu Gly Leu Leu Phe Ser Leu
1               5                   10                  15

Ala Arg Gly Ser Glu Val Gly Asn Ser Gln Ala Val Cys Pro Gly Thr
            20                  25                  30

Leu Asn Gly Leu Ser Val Thr Gly Asp Ala Glu Asn Gln Tyr Gln Thr
        35                  40                  45

Leu Tyr Lys Leu Tyr Glu Arg Cys Glu Val Val Met Gly Asn Leu Glu
    50                  55                  60

Ile Val Leu Thr Gly His Asn Ala Asp Leu Ser Phe Leu Gln Trp Ile
65                  70                  75                  80

Arg Glu Val Thr Gly Tyr Val Leu Val Ala Met Asn Glu Phe Ser Thr
                85                  90                  95

Leu Pro Leu Pro Asn Leu Arg Val Val Arg Gly Thr Gln Val Tyr Asp
            100                 105                 110

Gly Lys Phe Ala Ile Phe Val Met Leu Asn Tyr Asn Thr Asn Ser Ser
        115                 120                 125

His Ala Leu Arg Gln Leu Arg Leu Thr Gln Leu Thr Glu Ile Leu Ser
    130                 135                 140

Gly Gly Val Tyr Ile Glu Lys Asn Asp Lys Leu Cys His Met Asp Thr
145                 150                 155                 160

Ile Asp Trp Arg Asp Ile Val Arg Asp Arg Asp Ala Glu Ile Val Val
                165                 170                 175

Lys Asp Asn Gly Arg Ser Cys Pro Pro Cys His Glu Val Cys Lys Gly
            180                 185                 190

Arg Cys Trp Gly Pro Gly Ser Glu Asp Cys Gln Thr Leu Thr Lys Thr
        195                 200                 205

Ile Cys Ala Pro Gln Cys Asn Gly His Cys Phe Gly Pro Asn Pro Asn
    210                 215                 220

Gln Cys Cys His Asp Glu Cys Ala Gly Gly Cys Ser Gly Pro Gln Asp
225                 230                 235                 240
```

```
Thr Asp Cys Phe Ala Cys Arg His Phe Asn Asp Ser Gly Ala Cys Val
                245                 250                 255

Pro Arg Cys Pro Gln Pro Leu Val Tyr Asn Lys Leu Thr Phe Gln Leu
            260                 265                 270

Glu Pro Asn Pro His Thr Lys Tyr Gln Tyr Gly Gly Val Cys Val Ala
        275                 280                 285

Ser Cys Pro His Asn Phe Val Val Asp Gln Thr Ser Cys Val Arg Ala
    290                 295                 300

Cys Pro Pro Asp Lys Met Glu Val Asp Lys Asn Gly Leu Lys Met Cys
305                 310                 315                 320

Glu Pro Cys Gly Gly Leu Cys Pro Lys Ala Cys Glu Gly Thr Gly Ser
                325                 330                 335

Gly Ser Arg Phe Gln Thr Val Asp Ser Ser Asn Ile Asp Gly Phe Val
            340                 345                 350

Asn Cys Thr Lys Ile Leu Gly Asn Leu Asp Phe Leu Ile Thr Gly Leu
        355                 360                 365

Asn Gly Asp Pro Trp His Lys Ile Pro Ala Leu Asp Pro Glu Lys Leu
    370                 375                 380

Asn Val Phe Arg Thr Val Arg Glu Ile Thr Gly Tyr Leu Asn Ile Gln
385                 390                 395                 400

Ser Trp Pro Pro His Met His Asn Phe Ser Val Phe Ser Asn Leu Thr
                405                 410                 415

Thr Ile Gly Gly Arg Ser Leu Tyr Asn Arg Gly Phe Ser Leu Leu Ile
            420                 425                 430

Met Lys Asn Leu Asn Val Thr Ser Leu Gly Phe Arg Ser Leu Lys Glu
        435                 440                 445

Ile Ser Ala Gly Arg Ile Tyr Ile Ser Ala Asn Arg Gln Leu Cys Tyr
    450                 455                 460

His His Ser Leu Asn Trp Thr Lys Val Leu Arg Gly Pro Thr Glu Glu
465                 470                 475                 480

Arg Leu Asp Ile Lys His Asn Arg Pro Arg Arg Asp Cys Val Ala Glu
                485                 490                 495

Gly Lys Val Cys Asp Pro Leu Cys Ser Ser Gly Gly Cys Trp Gly Pro
            500                 505                 510

Gly Pro Gly Gln Cys Leu Ser Cys Arg Asn Tyr Ser Arg Gly Gly Val
        515                 520                 525

Cys Val Thr His Cys Asn Phe Leu Asn Gly Glu Pro Arg Glu Phe Ala
    530                 535                 540
```

```
His Glu Ala Glu Cys Phe Ser Cys His Pro Glu Cys Gln Pro Met Gly
545                 550                 555                 560

Gly Thr Ala Thr Cys Asn Gly Ser Gly Ser Asp Thr Cys Ala Gln Cys
                565                 570                 575

Ala His Phe Arg Asp Gly Pro His Cys Val Ser Ser Cys Pro His Gly
            580                 585                 590

Val Leu Gly Ala Lys Gly Pro Ile Tyr Lys Tyr Pro Asp Val Gln Asn
        595                 600                 605

Glu Cys Arg Pro Cys His Glu Asn Cys Thr Gln Gly Cys Lys Gly Pro
    610                 615                 620

Glu Leu Gln Asp Cys Leu Gly Gln Thr Leu Val Leu Ile Gly Lys Thr
625                 630                 635                 640

His Leu Thr Met Ala Leu Thr Val Ile Ala Gly Leu Val Val Ile Phe
                645                 650                 655

Met Met Leu Gly Gly Thr Phe Leu Tyr Trp Arg Gly Arg Arg Ile Gln
            660                 665                 670

Asn Lys Arg Ala Met Arg Arg Tyr Leu Glu Arg Gly Glu Ser Ile Glu
        675                 680                 685

Pro Leu Asp Pro Ser Glu Lys Ala Asn Lys Val Leu Ala Arg Ile Phe
    690                 695                 700

Lys Glu Thr Glu Leu Arg Lys Leu Lys Val Leu Gly Ser Gly Val Phe
705                 710                 715                 720

Gly Thr Val His Lys Gly Val Trp Ile Pro Glu Gly Glu Ser Ile Lys
                725                 730                 735

Ile Pro Val Cys Ile Lys Val Ile Glu Asp Lys Ser Gly Arg Gln Ser
            740                 745                 750

Phe Gln Ala Val Thr Asp His Met Leu Ala Ile Gly Ser Leu Asp His
        755                 760                 765

Ala His Ile Val Arg Leu Leu Gly Leu Cys Pro Gly Ser Ser Leu Gln
    770                 775                 780

Leu Val Thr Gln Tyr Leu Pro Leu Gly Ser Leu Leu Asp His Val Arg
785                 790                 795                 800

Gln His Arg Gly Ala Leu Gly Pro Gln Leu Leu Leu Asn Trp Gly Val
                805                 810                 815

Gln Ile Ala Lys Gly Met Tyr Tyr Leu Glu Glu His Gly Met Val His
            820                 825                 830

Arg Asn Leu Ala Ala Arg Asn Val Leu Leu Lys Ser Pro Ser Gln Val
        835                 840                 845
```

Gln Val Ala Asp Phe Gly Val Ala Asp Leu Leu Pro Pro Asp Asp Lys
850 855 860

Gln Leu Leu Tyr Ser Glu Ala Lys Thr Pro Ile Lys Trp Met Ala Leu
865 870 875 880

Glu Ser Ile His Phe Gly Lys Tyr Thr His Gln Ser Asp Val Trp Ser
885 890 895

Tyr Gly Val Thr Val Trp Glu Leu Met Thr Phe Gly Ala Glu Pro Tyr
900 905 910

Ala Gly Leu Arg Leu Ala Glu Val Pro Asp Leu Leu Glu Lys Gly Glu
915 920 925

Arg Leu Ala Gln Pro Gln Ile Cys Thr Ile Asp Val Tyr Met Val Met
930 935 940

Val Lys Cys Trp Met Ile Asp Glu Asn Ile Arg Pro Thr Phe Lys Glu
945 950 955 960

Leu Ala Asn Glu Phe Thr Arg Met Ala Arg Asp Pro Pro Arg Tyr Leu
965 970 975

Val Ile Lys Arg Glu Ser Gly Pro Gly Ile Ala Pro Gly Pro Glu Pro
980 985 990

His Gly Leu Thr Asn Lys Lys Leu Glu Glu Val Glu Leu Glu Pro Glu
995 1000 1005

Leu Asp Leu Asp Leu Asp Leu Glu Ala Glu Glu Asp Asn Leu Ala Thr
1010 1015 1020

Thr Thr Leu Gly Ser Ala Leu Ser Leu Pro Val Gly Thr Leu Asn Arg
1025 1030 1035 1040

Pro Arg Gly Ser Gln Ser Leu Leu Ser Pro Ser Ser Gly Tyr Met Pro
1045 1050 1055

Met Asn Gln Gly Asn Leu Gly Gly Ser Cys Gln Glu Ser Ala Val Ser
1060 1065 1070

Gly Ser Ser Glu Arg Cys Pro Arg Pro Val Ser Leu His Pro Met Pro
1075 1080 1085

Arg Gly Cys Leu Ala Ser Glu Ser Ser Glu Gly His Val Thr Gly Ser
1090 1095 1100

Glu Ala Glu Leu Gln Glu Lys Val Ser Met Cys Arg Ser Arg Ser Arg
1105 1110 1115 1120

Ser Arg Ser Pro Arg Pro Arg Gly Asp Ser Ala Tyr His Ser Gln Arg
1125 1130 1135

His Ser Leu Leu Thr Pro Val Thr Pro Leu Ser Pro Pro Gly Leu Glu
1140 1145 1150

Glu Glu Asp Val Asn Gly Tyr Val Met Pro Asp Thr His Leu Lys Gly
1155 1160 1165

Thr Pro Ser Ser Arg Glu Gly Thr Leu Ser Ser Val Gly Leu Ser Ser
1170 1175 1180

Val Leu Gly Thr Glu Glu Glu Asp Glu Asp Glu Glu Tyr Glu Tyr Met
1185 1190 1195 1200

Asn Arg Arg Arg Arg His Ser Pro Pro His Pro Pro Arg Pro Ser Ser
1205 1210 1215

Leu Glu Glu Leu Gly Tyr Glu Tyr Met Asp Val Gly Ser Asp Leu Ser
1220 1225 1230

Ala Ser Leu Gly Ser Thr Gln Ser Cys Pro Leu His Pro Val Pro Ile
1235 1240 1245

Met Pro Thr Ala Gly Thr Thr Pro Asp Glu Asp Tyr Glu Tyr Met Asn
1250 1255 1260

Arg Gln Arg Asp Gly Gly Gly Pro Gly Gly Asp Tyr Ala Ala Met Gly
1265 1270 1275 1280

Ala Cys Pro Ala Ser Glu Gln Gly Tyr Glu Glu Met Arg Ala Phe Gln
1285 1290 1295

Gly Pro Gly His Gln Ala Pro His Val His Tyr Ala Arg Leu Lys Thr
1300 1305 1310

Leu Arg Ser Leu Glu Ala Thr Asp Ser Ala Phe Asp Asn Pro Asp Tyr
1315 1320 1325

Trp His Ser Arg Leu Phe Pro Lys Ala Asn Ala Gln Arg Thr
1330 1335 1340

(2) INFORMATION FOR SEQ ID NO:10:

(i) SEQUENCE CHARACTERISTICS:
(A) LENGTH: 911 amino acids
(B) TYPE: amino acid
(C) STRANDEDNESS: unknown
(D) TOPOLOGY: unknown

(ii) MOLECULE TYPE: protein

(xi) SEQUENCE DESCRIPTION: SEQ ID NO:10:

Met Lys Pro Ala Thr Gly Leu Trp Val Trp Val Ser Leu Leu Val Ala
1 5 10 15

Ala Gly Thr Val Gln Pro Ser Asp Ser Gln Ser Val Cys Ala Gly Thr
20 25 30

```
Glu Asn Lys Leu Ser Ser Leu Ser Asp Leu Glu Gln Gln Tyr Arg Ala
        35                  40                  45

Leu Arg Lys Tyr Tyr Glu Asn Cys Glu Val Val Met Gly Asn Leu Glu
    50                  55                  60

Ile Thr Ser Ile Glu His Asn Arg Asp Leu Ser Phe Leu Arg Ser Val
65                  70                  75                  80

Arg Glu Val Thr Gly Tyr Val Leu Val Ala Leu Asn Gln Phe Arg Tyr
                85                  90                  95

Leu Pro Leu Glu Asn Leu Arg Ile Ile Arg Gly Thr Lys Leu Tyr Glu
            100                 105                 110

Asp Arg Tyr Ala Leu Ala Ile Phe Leu Asn Tyr Arg Lys Asp Gly Asn
        115                 120                 125

Phe Gly Leu Gln Glu Leu Gly Leu Lys Asn Leu Thr Glu Ile Leu Asn
    130                 135                 140

Gly Gly Val Tyr Val Asp Gln Asn Lys Phe Leu Cys Tyr Ala Asp Thr
145                 150                 155                 160

Ile His Trp Gln Asp Ile Val Arg Asn Pro Trp Pro Ser Asn Leu Thr
                165                 170                 175

Leu Val Ser Thr Asn Gly Ser Ser Gly Cys Gly Arg Cys His Lys Ser
            180                 185                 190

Cys Thr Gly Arg Cys Trp Gly Pro Thr Glu Asn His Cys Gln Thr Leu
        195                 200                 205

Thr Arg Thr Val Cys Ala Glu Gln Cys Asp Gly Arg Cys Tyr Gly Pro
    210                 215                 220

Tyr Val Ser Asp Cys Cys His Arg Glu Cys Ala Gly Gly Cys Ser Gly
225                 230                 235                 240

Pro Lys Asp Thr Asp Cys Phe Ala Cys Met Asn Phe Asn Asp Ser Gly
                245                 250                 255

Ala Cys Val Thr Gln Cys Pro Gln Thr Phe Val Tyr Asn Pro Thr Thr
            260                 265                 270

Phe Gln Leu Glu His Asn Phe Asn Ala Lys Tyr Thr Tyr Gly Ala Phe
        275                 280                 285

Cys Val Lys Lys Cys Pro His Asn Phe Val Val Asp Ser Ser Ser Cys
    290                 295                 300

Val Arg Ala Cys Pro Ser Ser Lys Met Glu Val Glu Glu Asn Gly Ile
305                 310                 315                 320

Lys Met Cys Lys Pro Cys Thr Asp Ile Cys Pro Lys Ala Cys Asp Gly
                325                 330                 335
```

```
Ile Gly Thr Gly Ser Leu Met Ser Ala Gln Thr Val Asp Ser Ser Asn
            340                 345                 350

Ile Asp Lys Phe Ile Asn Cys Thr Lys Ile Asn Gly Asn Leu Ile Phe
        355                 360                 365

Leu Val Thr Gly Ile His Gly Asp Pro Tyr Asn Ala Ile Glu Ala Ile
    370                 375                 380

Asp Pro Glu Lys Leu Asn Val Phe Arg Thr Val Arg Glu Ile Thr Gly
385                 390                 395                 400

Phe Leu Asn Ile Gln Ser Trp Pro Pro Asn Met Thr Asp Phe Ser Val
                405                 410                 415

Phe Ser Asn Leu Val Thr Ile Gly Gly Arg Val Leu Tyr Ser Gly Leu
            420                 425                 430

Ser Leu Leu Ile Leu Lys Gln Gln Gly Ile Thr Ser Leu Gln Phe Gln
        435                 440                 445

Ser Leu Lys Glu Ile Ser Ala Gly Asn Ile Tyr Ile Thr Asp Asn Ser
    450                 455                 460

Asn Leu Cys Tyr Tyr His Thr Ile Asn Trp Thr Thr Leu Phe Ser Thr
465                 470                 475                 480

Ile Asn Gln Arg Ile Val Ile Arg Asp Asn Arg Lys Ala Glu Asn Cys
                485                 490                 495

Thr Ala Glu Gly Met Val Cys Asn His Leu Cys Ser Ser Asp Gly Cys
            500                 505                 510

Trp Gly Pro Gly Pro Asp Gln Cys Leu Ser Cys Arg Arg Phe Ser Arg
        515                 520                 525

Gly Arg Ile Cys Ile Glu Ser Cys Asn Leu Tyr Asp Gly Glu Phe Arg
    530                 535                 540

Glu Phe Glu Asn Gly Ser Ile Cys Val Glu Cys Asp Pro Gln Cys Glu
545                 550                 555                 560

Lys Met Glu Asp Gly Leu Leu Thr Cys His Gly Pro Gly Pro Asp Asn
                565                 570                 575

Cys Thr Lys Cys Ser His Phe Lys Asp Gly Pro Asn Cys Val Glu Lys
            580                 585                 590

Cys Pro Asp Gly Leu Gln Gly Ala Asn Ser Phe Ile Phe Lys Tyr Ala
        595                 600                 605

Asp Pro Asp Arg Glu Cys His Pro Cys His Pro Asn Cys Thr Gln Gly
    610                 615                 620

Cys Asn Gly Pro Thr Ser His Asp Cys Ile Tyr Tyr Pro Trp Thr Gly
625                 630                 635                 640
```

```
His Ser Thr Leu Pro Gln Asp Pro Val Lys Val Lys Ala Leu Glu Gly
                645                 650                 655

Phe Pro Arg Leu Val Gly Pro Asp Phe Phe Gly Cys Ala Glu Pro Ala
            660                 665                 670

Asn Thr Phe Leu Asp Pro Glu Glu Pro Lys Ser Cys Asp Lys Thr His
        675                 680                 685

Thr Cys Pro Pro Cys Pro Ala Pro Glu Leu Leu Gly Gly Pro Ser Val
    690                 695                 700

Phe Leu Phe Pro Pro Lys Pro Lys Asp Thr Leu Met Ile Ser Arg Thr
705                 710                 715                 720

Pro Glu Val Thr Cys Val Val Val Asp Val Ser His Glu Asp Pro Glu
                725                 730                 735

Val Lys Phe Asn Trp Tyr Val Asp Gly Val Glu Val His Val Ala Lys
            740                 745                 750

Thr Lys Pro Arg Glu Glu Gln Tyr Asn Ser Thr Tyr Arg Val Val Ser
        755                 760                 765

Val Leu Thr Val Leu His Gln Asp Trp Leu Asn Gly Lys Glu Tyr Lys
    770                 775                 780

Cys Lys Val Ser Asn Lys Ala Leu Pro Ala Pro Ile Glu Lys Thr Ile
785                 790                 795                 800

Ser Lys Ala Lys Gly Gln Pro Arg Glu Pro Gln Val Tyr Thr Leu Pro
                805                 810                 815

Pro Ser Arg Asp Glu Leu Thr Lys Asn Gln Val Ser Leu Thr Cys Leu
            820                 825                 830

Val Lys Gly Phe Tyr Pro Ser Asp Ile Ala Val Glu Trp Glu Ser Asn
        835                 840                 845

Gly Gln Pro Glu Asn Asn Tyr Lys Thr Thr Pro Pro Val Leu Asp Ser
    850                 855                 860

Asp Gly Ser Phe Phe Leu Tyr Ser Lys Leu Thr Val Asp Lys Ser Arg
865                 870                 875                 880

Trp Gln Gln Gly Asn Val Phe Ser Cys Ser Val Met His Glu Ala Leu
                885                 890                 895

His Asn His Tyr Thr Gln Lys Ser Leu Ser Leu Ser Pro Gly Lys
            900                 905                 910
```

(2) INFORMATION FOR SEQ ID NO:11:

(i) SEQUENCE CHARACTERISTICS:
(A) LENGTH: 6 amino acids
(B) TYPE: amino acid
(C) STRANDEDNESS: unknown

(D) TOPOLOGY: unknown

(ii) MOLECULE TYPE: peptide

(xi) SEQUENCE DESCRIPTION: SEQ ID NO:11:

Gly Xaa Gly Xaa Xaa Gly
1 5

(2) INFORMATION FOR SEQ ID NO:12:

(i) SEQUENCE CHARACTERISTICS:
(A) LENGTH: 6 amino acids
(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:12:

Asp Leu Ala Ala Arg Asn
1 5

(2) INFORMATION FOR SEQ ID NO:13:

(i) SEQUENCE CHARACTERISTICS:
(A) LENGTH: 6 amino acids
(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:13:

Pro Ile Lys Trp Met Ala
1 5

(2) INFORMATION FOR SEQ ID NO:14:

(i) SEQUENCE CHARACTERISTICS:
(A) LENGTH: 20 base pairs
(B) TYPE: nucleic acid
(C) STRANDEDNESS: single
(D) TOPOLOGY: unknown

(ii) MOLECULE TYPE: DNA (genomic)

(xi) SEQUENCE DESCRIPTION: SEQ ID NO:14:

ACNGTNTGGG ARYTNAYHAC 20

(2) INFORMATION FOR SEQ ID NO:15:

(i) SEQUENCE CHARACTERISTICS:
(A) LENGTH: 23 base pairs
(B) TYPE: nucleic acid
(C) STRANDEDNESS: single
(D) TOPOLOGY: unknown

(ii) MOLECULE TYPE: DNA (genomic)

(xi) SEQUENCE DESCRIPTION: SEQ ID NO:15:

CAYGTNAARA THACNGAYTT YGG 23

(2) INFORMATION FOR SEQ ID NO:16:

(i) SEQUENCE CHARACTERISTICS:
(A) LENGTH: 25 base pairs
(B) TYPE: nucleic acid
(C) STRANDEDNESS: single
(D) TOPOLOGY: unknown

(ii) MOLECULE TYPE: DNA (genomic)

(xi) SEQUENCE DESCRIPTION: SEQ ID NO:16:

GACGAATTCC NATHAARTGG ATGGC 25

(2) INFORMATION FOR SEQ ID NO:17:

(i) SEQUENCE CHARACTERISTICS:
(A) LENGTH: 24 base pairs
(B) TYPE: nucleic acid
(C) STRANDEDNESS: single
(D) TOPOLOGY: unknown

(ii) MOLECULE TYPE: DNA (genomic)

(xi) SEQUENCE DESCRIPTION: SEQ ID NO:17:

ACAYTTNARD ATDATCATRT ANAC 24

(2) INFORMATION FOR SEQ ID NO:18:

(i) SEQUENCE CHARACTERISTICS:
(A) LENGTH: 17 base pairs
(B) TYPE: nucleic acid

(C) STRANDEDNESS: single
(D) TOPOLOGY: unknown

(ii) MOLECULE TYPE: DNA (genomic)

(xi) SEQUENCE DESCRIPTION: SEQ ID NO:18:

AANGTCATNA RYTCCCA 17

(2) INFORMATION FOR SEQ ID NO:19:

(i) SEQUENCE CHARACTERISTICS:
(A) LENGTH: 23 base pairs
(B) TYPE: nucleic acid
(C) STRANDEDNESS: single
(D) TOPOLOGY: unknown

(ii) MOLECULE TYPE: DNA (genomic)

(xi) SEQUENCE DESCRIPTION: SEQ ID NO:19:

TCCAGNGCGA TCCAYTTDAT NGG 23

(2) INFORMATION FOR SEQ ID NO:20:

(i) SEQUENCE CHARACTERISTICS:
(A) LENGTH: 18 base pairs
(B) TYPE: nucleic acid
(C) STRANDEDNESS: single
(D) TOPOLOGY: unknown

(ii) MOLECULE TYPE: DNA (genomic)

(xi) SEQUENCE DESCRIPTION: SEQ ID NO:20:

GGRTCDATCA TCCARCCT 18

(2) INFORMATION FOR SEQ ID NO:21:

(i) SEQUENCE CHARACTERISTICS:
(A) LENGTH: 20 base pairs
(B) TYPE: nucleic acid
(C) STRANDEDNESS: single
(D) TOPOLOGY: unknown

(ii) MOLECULE TYPE: DNA (genomic)

(xi) SEQUENCE DESCRIPTION: SEQ ID NO:21:

CTGCTGTCAG CATCGATCAT 20

(2) INFORMATION FOR SEQ ID NO:22:

(i) SEQUENCE CHARACTERISTICS:
(A) LENGTH: 7 amino acids
(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:22:

Thr Val Trp Glu Leu Met Thr
1 5

(2) INFORMATION FOR SEQ ID NO:23:

(i) SEQUENCE CHARACTERISTICS:
(A) LENGTH: 8 amino acids
(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:23:

His Val Lys Ile Thr Asp Phe Gly
1 5

(2) INFORMATION FOR SEQ ID NO:24:

(i) SEQUENCE CHARACTERISTICS:
(A) LENGTH: 7 amino acids
(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:24:

Val Tyr Met Ile Ile Leu Lys
1 5

(2) INFORMATION FOR SEQ ID NO:25:

(i) SEQUENCE CHARACTERISTICS:
(A) LENGTH: 6 amino acids

(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:25:

Trp Glu Leu Met Thr Phe
1 5

(2) INFORMATION FOR SEQ ID NO:26:

(i) SEQUENCE CHARACTERISTICS:
(A) LENGTH: 8 amino acids
(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:26:

Pro Ile Lys Trp Met Ala Leu Glu
1 5

(2) INFORMATION FOR SEQ ID NO:27:

(i) SEQUENCE CHARACTERISTICS:
(A) LENGTH: 6 amino acids
(B) TYPE: amino acid
(C) STRANDEDNESS: unknown
(D) TOPOLOGY: unknown

(ii) MOLECULE TYPE: peptide

(xi) SEQUENCE DESCRIPTION: SEQ ID NO:27:

Cys Trp Met Ile Asp Pro
1 5

(2) INFORMATION FOR SEQ ID NO:28:

(i) SEQUENCE CHARACTERISTICS:
(A) LENGTH: 35 base pairs
(B) TYPE: nucleic acid
(C) STRANDEDNESS: single
(D) TOPOLOGY: unknown

(ii) MOLECULE TYPE: DNA (genomic)

(xi) SEQUENCE DESCRIPTION: SEQ ID NO:28:

GACTCGAGTC GACATCGATT TTTTTTTTTT TTTTT 35

(2) INFORMATION FOR SEQ ID NO:29:

(i) SEQUENCE CHARACTERISTICS:
(A) LENGTH: 24 base pairs
(B) TYPE: nucleic acid
(C) STRANDEDNESS: single
(D) TOPOLOGY: unknown

(ii) MOLECULE TYPE: DNA (genomic)

(xi) SEQUENCE DESCRIPTION: SEQ ID NO:29:

GAAGAAAGAC GACTCGTTCA TCGG 24

(2) INFORMATION FOR SEQ ID NO:30:

(i) SEQUENCE CHARACTERISTICS:
(A) LENGTH: 25 base pairs
(B) TYPE: nucleic acid
(C) STRANDEDNESS: single
(D) TOPOLOGY: unknown

(ii) MOLECULE TYPE: DNA (genomic)

(xi) SEQUENCE DESCRIPTION: SEQ ID NO:30:

GACCATGACC ATGTAAACGT CAATA 25